(19) Europäisches Patentamt
European Patent Office
Office européen des brevets

(11) EP 3 967 763 A1

(12) **EUROPEAN PATENT APPLICATION**
published in accordance with Art. 153(4) EPC

(43) Date of publication:
**16.03.2022 Bulletin 2022/11**

(21) Application number: **20806642.3**

(22) Date of filing: **08.05.2020**

(51) International Patent Classification (IPC):
***C12P 7/42*** *(2006.01)* ***C12N 5/10*** *(2006.01)*
***C12N 1/15*** *(2006.01)* ***C12N 1/19*** *(2006.01)*
***C12N 1/21*** *(2006.01)* ***C12N 9/02*** *(2006.01)*

(52) Cooperative Patent Classification (CPC):
**C12N 5/10; C12P 7/42**

(86) International application number:
**PCT/JP2020/018665**

(87) International publication number:
**WO 2020/230719 (19.11.2020 Gazette 2020/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.05.2019 JP 2019089771**

(71) Applicant: **Toray Industries, Inc.**
**Tokyo, 103-8666 (JP)**

(72) Inventors:
- **ISOBE, Kyohei**
**Kamakura-shi, Kanagawa 248-8555 (JP)**
- **KAWAMURA, Kenji**
**Kamakura-shi, Kanagawa 248-8555 (JP)**
- **YAMADA, Katsushige**
**Kamakura-shi, Kanagawa 248-8555 (JP)**

(74) Representative: **Kador & Partner PartG mbB**
**Corneliusstraße 15**
**80469 München (DE)**

(54) **GENETICALLY MODIFIED MICROORGANISM FOR PRODUCING 3-HYDROXYHEXANEDIOIC ACID, (E)-HEX-2-ENEDIOIC ACID AND/OR HEXANEDIOIC ACID, AND PRODUCTION METHOD FOR SAID CHEMICALS**

(57) Disclosed are a genetically modified microorganism with an ability to produce 3-hydroxyadipic acid, α-hydromuconic acid, and/or adipic acid in high yield, and a method of producing 3-hydroxyadipic acid, α-hydromuconic acid, and/or adipic acid by using the genetically modified microorganism. The genetically modified microorganism has an ability to produce 3-hydroxyadipic acid, α-hydromuconic acid, and/or adipic acid and is deficient in the function of pyruvate kinase, in which the activities of phosphoenolpyruvate carboxykinase and of an enzyme that catalyzes the reaction of reducing 3-oxoadipyl-CoA to 3-hydroxyadipyl-CoA are enhanced.



Printed by Jouve, 75001 PARIS (FR)

## Description

Technical Field

The present invention relates to a genetically modified microorganism able to produce 3-hydroxyadipic acid, α-hydromuconic acid, and/or adipic acid in high yield and to a method of producing 3-hydroxyadipic acid, α-hydromuconic acid, and/or adipic acid by using the genetically modified microorganism.

Background Art

3-Hydroxyadipic acid (IUPAC name: 3-hydroxyhexanedioic acid) and α-hydromuconic acid (IUPAC name: (E)-hex-2-enedioic acid) are dicarboxylic acids containing six carbon atoms. These dicarboxylic acids can be polymerized with a polyhydric alcohol or a polyfunctional amine, to be used as raw materials for the production of polyesters or polyamides, respectively. Additionally, these dicarboxylic acids can be used alone after ammonia addition at a terminal position in these chemicals to form lactams as raw materials for the production of polyamides.

The following documents related to the production of 3-hydroxyadipic acid or α-hydromuconic acid using a microorganism are known.

Patent Document 1 describes a method of producing 1,3-butadiene by using a microorganism in which a relevant metabolic pathway is modified, wherein 3-hydroxyadipic acid (3-hydroxyadipate) is described to be a metabolic intermediate in the metabolic pathway for biosynthesis of 1,3-butadiene from acetyl-CoA and succinyl-CoA.

Patent Document 2 describes a method of producing muconic acid by using a microorganism in which a relevant metabolic pathway is modified, wherein α-hydromuconic acid (2,3-dehydroadipate) is described to be a metabolic intermediate in the metabolic pathway for biosynthesis of *trans,trans*-muconic acid from acetyl-CoA and succinyl-CoA.

Patent Documents 3 and 4 describe a method of producing adipic acid and hexamethylene diamine (HMDA) by using a non-natural microorganism, wherein the biosynthetic pathways for these substances are described to share a common reaction to synthesize 3-oxoadipyl-CoA from acetyl-CoA and succinyl-CoA but diverge after the synthesis of 3-oxoadipyl-CoA. Furthermore, Patent Document 3 describes the pyruvate kinase gene as a candidate gene that is additionally deleted to improve the HMDA formation coupled with proliferation for the HMDA production, but a potential relationship between pyruvate kinase deficiency and increased adipic acid production is not mentioned in this document.

Additionally, all the biosynthetic pathways mentioned in Patent Documents 1 to 4 are described to share a common enzymatic reaction that reduces 3-oxoadipyl-CoA to 3-hydroxyadipyl-CoA.

Patent Documents 5 and 6 describe methods of producing 3-hydroxyadipic acid and α-hydromuconic acid by using a microorganism of the genus *Serratia,* respectively. The patent documents disclose that the efficiency of producing 3-hydroxyadipic acid and α-hydromuconic acid can be increased particularly by enhancing the activity of an acyl transferase that catalyzes a reaction to produce 3-oxoadipyl-CoA from acetyl-CoA and succinyl-CoA, but these documents have no description related to pyruvate kinase.

Moreover, a method of modifying a microorganism based on an *in silico* analysis is disclosed in Patent Document 7, in which the production of succinic acid is increased by deleting genes encoding pyruvate kinase and a phosphotransferase system enzyme in *Escherichia coli* (*E. coli*), *pykF, pykA,* and *ptsG,* and culturing the resulting *E. coli* bacteria under anaerobic conditions.

Prior Art Documents

Patent Documents

Patent Document 1: JP 2013-535203 A
Patent Document 2: US 2011/0124911 A1
Patent Document 3: JP 2015-146810 A
Patent Document 4: JP 2011-515111 A
Patent Document 5: WO 2017/209102
Patent Document 6: WO 2017/209103
Patent Document 7: JP 2008-527991 A

## Summary of the Invention

### Problems to be Solved by the Invention

Patent Documents 1 and 2 describe metabolic pathways by which the microorganisms can produce 3-hydroxyadipic acid and α-hydromuconic acid, but have no description about interruption of the metabolic pathways to allow the microorganisms to secrete 3-hydroxyadipic acid or α-hydromuconic acid into culture medium. Moreover, the prior studies described in Patent Documents 1 to 4 have not examined whether or not 3-hydroxyadipic acid or α-hydromuconic acid can be actually produced by using a microorganism in which a relevant metabolic pathway is modified by introducing a nucleic acid encoding an enzyme that catalyzes a reaction to reduce 3-oxoadipyl-CoA to 3-hydroxyadipyl-CoA. Patent Documents 3 to 6 disclose enhancement of gene expression of enzymes involved in increased production of 3-hydroxyadipic acid, α-hydromuconic acid, or adipic acid but have no description about enhancement of any enzymatic activity in the metabolic pathways upstream of acetyl-CoA and succinyl-CoA, wherein all the enzyme genes whose expression is increased are limited only to reactions downstream of acetyl-CoA and succinyl-CoA in the biosynthetic pathways.

Accordingly, an object of the present invention is to provide a genetically modified microorganism for producing 3-hydroxyadipic acid, α-hydromuconic acid, and/or adipic acid in high yield and a method of producing a substance by using the modified microorganism, wherein the modified microorganism is based on a genetically modified microorganism in which a nucleic acid encoding an enzyme that exhibits excellent activity in 3-oxoadipyl-CoA reduction reaction is introduced or the expression of the enzyme is enhanced to increase the activity of the enzyme, and wherein the modified microorganism is further modified to have an altered upstream metabolic pathway.

### Means for Solving the Problem

The inventors intensively studied in order to achieve the above-described object and consequently found that a genetically modified microorganism having an ability to produce 3-hydroxyadipic acid, α-hydromuconic acid, and/or adipic acid, having impaired pyruvate kinase function, and having enhanced activities of phosphoenolpyruvate carboxykinase and of an enzyme that catalyzes the reaction of reducing 3-oxoadipyl-CoA to 3-hydroxyadipyl-CoA has an excellent ability to produce 3-hydroxyadipic acid, α-hydromuconic acid, and/or adipic acid, to complete the present invention.

That is, the present invention provides the following:

(1) A genetically modified microorganism with an ability to produce 3-hydroxyadipic acid, α-hydromuconic acid, and/or adipic acid, in which the function of pyruvate kinase is impaired and the activities of phosphoenolpyruvate carboxykinase and of an enzyme that catalyzes a reaction to reduce 3-oxoadipyl-CoA to 3-hydroxyadipyl-CoA are enhanced.
(2) The genetically modified microorganism according to (1), wherein the function of a phosphotransferase system enzyme is further impaired.
(3) The genetically modified microorganism according to (1) or (2), wherein the enzyme that catalyzes a reaction to reduce 3-oxoadipyl-CoA to 3-hydroxyadipyl-CoA is any one of the following polypeptides (a) to (c):

(a) a polypeptide composed of an amino acid sequence represented by any one of SEQ ID NOs: 1 to 7;
(b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;
(c) a polypeptide composed of an amino acid sequence with a sequence identity of not less than 70% to the sequence represented by any one of SEQ ID NOs: 1 to 7 and having an enzymatic activity that catalyzes a reaction to reduce 3-oxoadipyl-CoA to 3-hydroxyadipyl-CoA.

(4) A method of producing 3-hydroxyadipic acid, α-hydromuconic acid, and/or adipic acid, comprising the step of culturing the genetically modified microorganism according to any one of (1) to (3).

### Effects of the Invention

A genetically modified microorganism with an ability to produce 3-hydroxyadipic acid, α-hydromuconic acid, and/or adipic acid and with impaired pyruvate kinase function and with enhanced activities of phosphoenolpyruvate carboxykinase and of an enzyme that catalyzes the reaction of reducing 3-oxoadipyl-CoA to 3-hydroxyadipyl-CoA can produce 3-hydroxyadipic acid, α-hydromuconic acid, and/or adipic acid in high yield compared to a parental strain of the microorganism in which the genes encoding those enzymes are unaltered.

Detailed Description of the Invention

In the context of this invention, the inventors have found that 3-hydroxyadipic acid, α-hydromuconic acid, and/or adipic acid can be produced in high yield in a microorganism that originally has an ability to produce 3-hydroxyadipic acid, α-hydromuconic acid, and/or adipic acid by impairing the function of pyruvate kinase and enhancing the activities of phosphoenolpyruvate carboxykinase and of an enzyme that catalyzes the reaction of reducing 3-oxoadipyl-CoA to 3-hydroxyadipyl-CoA in the microorganism.

An enzyme that catalyzes the reaction of reducing 3-oxoadipyl-CoA to 3-hydroxyadipyl-CoA is hereinafter also referred to as "3-oxoadipyl-CoA reductase" in this specification. Additionally, phosphoenolpyruvic acid may be abbreviated as PEP, 3-hydroxyadipic acid may be abbreviated as 3HA, α-hydromuconic acid may be abbreviated as HMA, and adipic acid may be abbreviated as ADA, respectively, in this specification.

In the present invention, examples of enhancing the activities of phosphoenolpyruvate carboxykinase and of an enzyme that catalyzes the reaction of reducing 3-oxoadipyl-CoA to 3-hydroxyadipyl-CoA include a method in which nucleic acids encoding these polypeptides are introduced from the outside to the inside of a host microorganism; a method in which the copy numbers of nucleic acids encoding the polypeptides are increased; and a method in which a promoter region or a ribosome-binding sequence upstream of the region coding for each of the polypeptides is modified. These methods may be carried out individually or in combination. The method of introduction of a nucleic acid is not limited to a particular method, and examples of the method that can be used include a method in which a nucleic acid of interest is integrated into an expression vector capable of autonomous replication in a microorganism and then integrated into a host microorganism, and a method in which a nucleic acid of interest is integrated into the genome of a microorganism.

One or more of the above nucleic acids may be introduced. Moreover, the introduction of a nucleic acid and the enhancement of polypeptide expression may be combined.

When a nucleic acid encoding a polypeptide expressed in the present invention is integrated into an expression vector or the genome of a host microorganism, the nucleic acid to be integrated into the expression vector or the genome is preferably composed of a promoter, a ribosome-binding sequence, a nucleic acid encoding the polypeptide to be expressed, and a transcription termination sequence, and may additionally contain a gene that controls the activity of the promoter.

The promoter used in the present invention is not limited to a particular promoter, as long as the promoter drives expression of the enzyme in the host microorganism; examples of the promoter include *gap* promoter, *trp* promoter, *lac* promoter, *tac* promoter, and T7 promoter.

In cases where an expression vector is used in the present invention to introduce the nucleic acid or to enhance the expression of the polypeptide, the expression vector is not limited to a particular vector, as long as the vector is capable of autonomous replication in the microorganism; examples of the vector include pBBR1MCS vector, pBR322 vector, pMW vector, pET vector, pRSF vector, pCDF vector, pACYC vector, and derivatives of the above vectors.

In cases where a nucleic acid for genome integration is used in the present invention to introduce the nucleic acid or to enhance the expression of the polypeptide, the nucleic acid for genome integration is introduced by site-specific homologous recombination. The method for site-specific homologous recombination is not limited to a particular method, and examples of the method include a method in which λ Red recombinase and FLP recombinase are used (Proc Natl Acad Sci U.S.A. 2000 Jun 6; 97 (12): 6640-6645.), and a method in which λ Red recombinase and the *sacB* gene are used (Biosci Biotechnol Biochem. 2007 Dec;71 (12):2905-11.).

The method of introducing the expression vector or the nucleic acid for genome integration is not limited to a particular method, as long as the method is for introduction of a nucleic acid into a microorganism; examples of the method include the calcium ion method (Journal of Molecular Biology, 53, 159 (1970)), and electroporation (NM Calvin, PC Hanawalt. J. Bacteriol, 170 (1988), pp. 2796-2801).

The scheme 1 below shows an exemplary reaction pathway required for the production of 3-hydroxyadipic acid, α-hydromuconic acid, and/or adipic acid. In this scheme, the reaction A represents a reaction that generates 3-oxoadipyl-CoA and coenzyme A from acetyl-CoA and succinyl-CoA. The reaction B represents a reaction that reduces 3-oxoadipyl-CoA to 3-hydroxyadipyl-CoA. The reaction C represents a reaction that generates 2,3-dehydroadipyl-CoA from 3-hydroxyadipyl-CoA. The reaction D represents a reaction that generates adipyl-CoA from 2,3-dehydroadipyl-CoA. The reaction E represents a reaction that generates 3-hydroxyadipic acid from 3-hydroxyadipyl-CoA. The reaction F represents a reaction that generates α-hydromuconic acid from 2,3-dehydroadipyl-CoA. The reaction G represents a reaction that generates adipic acid from adipyl-CoA.

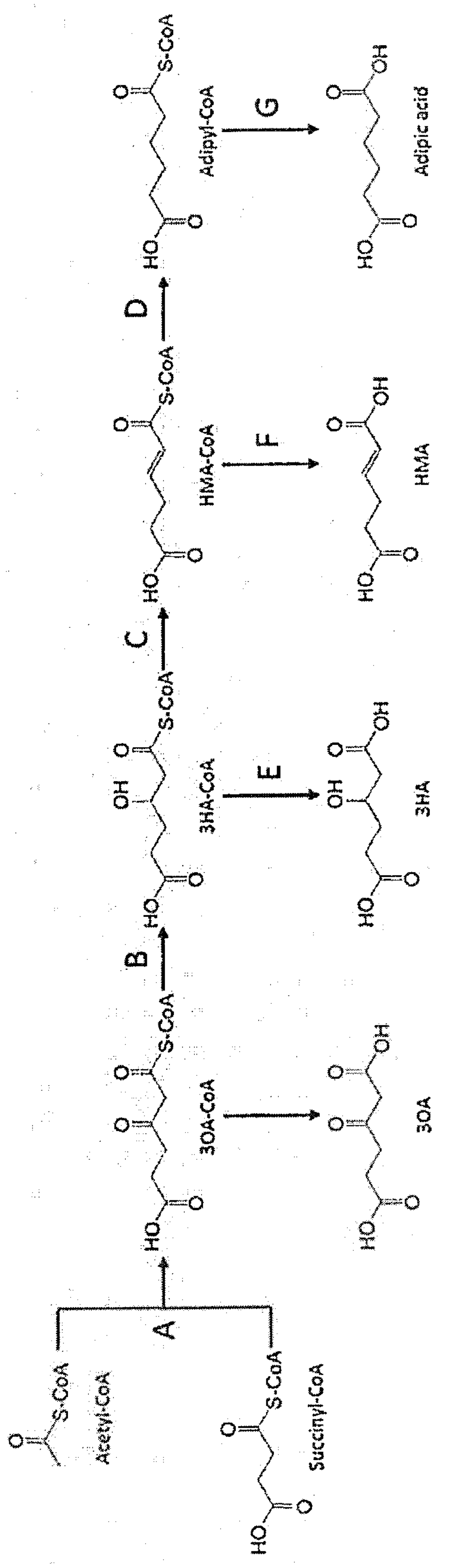


Scheme 1

In cases where a microorganism has an ability to produce 3-hydroxyadipic acid, α-hydromuconic acid, and/or adipic acid, such a microorganism is known to have an enzyme that catalyzes at least the reaction A in a biosynthetic pathway shown in the above scheme 1. Preferably, reactions to generate 3-hydroxyadipic acid, α-hydromuconic acid,

or adipic acid from 3-oxoadipyl-CoA are involved in the biosynthetic pathway shown in the scheme 1. That is, in cases where a genetically modified microorganism according to the present invention has an ability to produce 3-hydroxyadipic acid, a host microorganism used for the generation of the genetically modified microorganism preferably has an ability to generate 3-oxoadipyl-CoA and coenzyme A from acetyl-CoA and succinyl-CoA (the reaction A), an ability to reduce 3-oxoadipyl-CoA to 3-hydroxyadipyl-CoA (the reaction B), and an ability to generate 3-hydroxyadipic acid from 3-hydroxyadipyl-CoA (the reaction E). Moreover, in cases where a genetically modified microorganism according to the present invention has an ability to produce α-hydromuconic acid, a host microorganism used for the generation of the genetically modified microorganism preferably has an ability to generate 3-oxoadipyl-CoA and coenzyme A from acetyl-CoA and succinyl-CoA (the reaction A), an ability to reduce 3-oxoadipyl-CoA to 3-hydroxyadipyl-CoA (the reaction B), an ability to generate 2,3-dehydroadipyl-CoA from 3-hydroxyadipyl-CoA (the reaction C), and an ability to generate α-hydromuconic acid from 2,3-dehydroadipyl-CoA (the reaction F). Furthermore, in cases where a genetically modified microorganism according to the present invention has an ability to produce adipic acid, a host microorganism used for the generation of the genetically modified microorganism preferably has an ability to generate 3-oxoadipyl-CoA and coenzyme A from acetyl-CoA and succinyl-CoA (the reaction A), an ability to reduce 3-oxoadipyl-CoA to 3-hydroxyadipyl-CoA (the reaction B), an ability to generate 2,3-dehydroadipyl-CoA from 3-hydroxyadipyl-CoA (the reaction C), an ability to generate adipyl-CoA from 2,3-dehydroadipyl-CoA (the reaction D), and an ability to generate adipic acid from adipyl-CoA (the reaction G).

A genetically modified microorganism that can produce 3-hydroxyadipic acid, α-hydromuconic acid, and/or adipic acid in high yield can be obtained by impairing the function of pyruvate kinase and enhancing the activities of PEP carboxykinase and of an enzyme that catalyzes the reaction of reducing 3-oxoadipyl-CoA to 3-hydroxyadipyl-CoA in a host microorganism, which is a microorganism that originally carries biosynthetic pathways for the above substances.

Microorganisms that originally have an ability to produce 3-hydroxyadipic acid include microorganisms belonging to the following species:

species of the genus *Escherichia,* such as *Escherichia fergusonii* and *Escherichia coli;*
species of the genus *Pseudomonas,* such as *Pseudomonas chlororaphis, Pseudomonas putida, Pseudomonas azotoformans,* and *Pseudomonas chlororaphis* subsp. *aureofaciens;*
species of the genus *Hafnia,* such as *Hafnia alvei;*
species of the genus *Corynebacterium,* such as *Corynebacterium aceloacidophilum, Corynebacterium acetoglutamicum, Corynebacterium ammoniagenes,* and *Corynebacterium glutamicum;*
species of the genus *Bacillus,* such as *Bacillus badius, Bacillus magalerium,* and *Bacillus roseus;*
species of the genus *Streptomyces,* such as *Streptomyces vinaceus, Streptomyces karnatakensis,* and *Streptomyces olivaceus;*
species of the genus *Cupriavidus,* such as *Cupriavidus metallidurans, Cupriavidus necator,* and *Cupriavidus oxalaticus*;
species of the genus *Acinetobacter,* such as *Acinetobacter baylyi* and *Acinetobacter radioresistens;*
species of the genus *Alcaligenes,* such as *Alcaligenes faecalis*;
species of the genus *Nocardioides,* such as *Nocardioides albus*;
species of the genus *Brevibacterium,* such as *Brevibacterium iodinum*;
species of the genus *Delftia,* such as *Delftia acidovorans*;
species of the genus *Shimwellia,* such as *Shimwellia blattae*;
species of the genus *Aerobacter,* such as *Aerobacter cloacae*;
species of the genus *Rhizobium,* such as *Rhizobium radiobacter*;
species of the genus *Serratia,* such as *Serratia grimesii, Serratia ficaria, Serratia fonticola, Serratia odorifera, Serratia plymuthica, Serratia entomophila,* and *Serratia nematodiphila.*

In cases where a genetically modified microorganism according to the present invention originally has no ability to produce 3-hydroxyadipic acid, an appropriate combination of nucleic acids that encode enzymes catalyzing the reactions A, B, and E can be introduced into the microorganism to impart those production abilities.

Microorganisms that are speculated to originally have an ability to produce α-hydromuconic acid include microorganisms belonging to the following species:

species of the genus *Escherichia,* such as *Escherichia fergusonii* and *Escherichia coli;*
species of the genus *Pseudomonas,* such as *Pseudomonas fluorescens, Pseudomonas putida, Pseudomonas azotoformans,* and *Pseudomonas chlororaphis* subsp. *aureofaciens;*
species of the genus *Hafnia,* such as *Hafnia alvei;*
species of the genus *Bacillus,* such as *Bacillus badius;*
species of the genus *Cupriavidus,* such as *Cupriavidus metallidurans, Cupriavidus numazuensis,* and *Cupriavidus*

*oxalaticus;*
species of the genus *Acinetobacter,* such as *Acinetobacter baylyi* and *Acinetobacter radioresistens;*
species of the genus *Alcaligenes,* such as *Alcaligenes faecalis;*
species of the genus *Delftia,* such as *Delftia acidovorans;*
species of the genus *Shimwellia,* such as *Shimwellia blattae;*
species of the genus *Serratia,* such as *Serratia grimesii, Serratia ficaria, Serratia fonticola, Serratia odorifera, Serratia plymuthica, Serratia entomophila,* and *Serratia nematodiphila.*

In cases where a genetically modified microorganism according to the present invention originally has no ability to produce α-hydromuconic acid, an appropriate combination of nucleic acids that encode enzymes catalyzing the reactions A, B, C, and F can be introduced into the microorganism to impart those production abilities.

Microorganisms that are speculated to originally have the ability to produce adipic acid include microorganisms belonging to the genus *Thermobifida,* such as *Thermobifida fusca* In cases where a genetically modified microorganism according to the present invention originally has no ability to produce adipic acid, an appropriate combination of nucleic acids that encode enzymes catalyzing the reactions A, B, C, D, and G can be introduced into the microorganism to impart those production abilities.

In the present invention, examples of the microorganism that can be used as a host to obtain the genetically modified microorganism preferably include the microorganisms listed above, especially preferably microorganisms belonging to the genera *Escherichia, Serratia, Hafnia, Pseudomonas, Corynebacterium, Bacillus, Streptomyces, Cupriavidus, Acinetobacter, Alcaligenes, Brevibacterium, Delftia, Shimwellia, Aerobacter, Rhizobium, Thermobifida, Clostridium, Schizosaccharomyces, Kluyveromyces, Pichia,* and *Candida.* Among these, microorganisms belonging to the genera *Escherichia, Serratia, Hafnia,* and *Pseudomonas* are especially preferred.

Specific examples of the enzyme that catalyzes the reaction of reducing 3-oxoadipyl-CoA to 3-hydroxyadipyl-CoA include the polypeptides described in (a) to (c) below:

(a) a polypeptide composed of an amino acid sequence represented by any one of SEQ ID NOs: 1 to 7;
(b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;
(c) a polypeptide composed of an amino acid sequence with a sequence identity of not less than 70% to the sequence represented by any one of SEQ ID NOs: 1 to 7 and having activity in reduction of 3-oxoadipyl-CoA to 3-hydroxyadipyl-CoA.

In addition, either an enzyme classified as 3-hydroxyacyl-CoA dehydrogenase with EC number 1.1.1.35 or an enzyme classified as 3-hydroxybutyryl-CoA dehydrogenase with EC number 1.1.1.157 can also be used as an enzyme with 3-oxoadipyl-CoA reductase activity. Specifically, PaaH from *Pseudomonas putida* strain KT2440 (NCBI-Protein ID: NP_745425.1), PaaH from *Escherichia coli* strain K-12 substrain MG1655 (NCBI-Protein ID: NP_415913.1), DeaH from *Acinetobacter baylyi* strain ADP1 (NCBI-Protein ID: CAG68533.1), PaaH from *Serratia plymuthica* strain NBRC102599 (NCBI-Protein ID: WP_063197120), and a polypeptide from *Serratia nematodiphila* strain DSM21420 (NCBI-Protein ID: WP_033633399.1) are also included as examples of the enzyme that catalyzes the reaction of reducing 3-oxoadipyl-CoA to 3-hydroxyadipyl-CoA. Among these, the polypeptides described in (a) to (c) above are preferred.

For the polypeptide used in the present invention and composed of the same amino acid sequence as that represented by any one of SEQ ID NOs: 1 to 7, except that one or several amino acids are substituted, deleted, inserted, and/or added, and having 3-oxoadipyl-CoA reductase activity, the range represented by the phrase "one or several" is preferably 10 or less, more preferably 5 or less, especially preferably 4 or less, and most preferably one or two. In the case of amino acid substitution, the activity of the original polypeptide is more likely to be maintained when an amino acid(s) is/are replaced by an amino acid(s) with similar properties (so-called conservative substitution). That is, the physiological properties of the original polypeptide are often maintained when an amino acid(s) is/are replaced by an amino acid(s) with similar properties. Therefore, in the case of substitution, a given amino acid is preferably replaced by another amino acid with similar properties. That is, the natural 20 amino acids that make up natural proteins can be divided into groups of amino acids with similar properties, such as neutral amino acids with a less polar side chain (Gly, Ile, Val, Leu, Ala, Met, Pro), neutral amino acids with a hydrophilic side chain (Asn, Gln, Thr, Ser, Tyr, Cys), acidic amino acids (Asp, Glu), and basic amino acids (Arg, Lys, His), and aromatic amino acids (Phe, Tyr, Trp). It is often the case that substitution between amino acids in the same group does not change the properties of the original polypeptide.

For the polypeptide used in the present invention and having an amino acid sequence with a sequence identity of not less than 70% to the sequence represented by any one of SEQ ID NOs: 1 to 7 and having 3-oxoadipyl-CoA reductase activity, the sequence identity is preferably not less than 80%, more preferably not less than 85%, further preferably not less than 90%, still further preferably not less than 95%, yet further preferably not less than 97%, and

even further preferably not less than 99%.

In the present invention, the term "sequence identity" means a ratio (percentage) of the number of identical amino acid or nucleotide residues relative to the total number of amino acid or nucleotide residues over the overlapping portion of an amino acid sequence alignment (including an amino acid corresponding to the translation start site) or a nucleotide sequence alignment (including the start codon), which is obtained by aligning two amino acid or nucleotide sequences with or without introduction of gaps for an optimal match, and is calculated by the following formula (1). In the formula (1), the length of a shorter sequence being compared is not less than 400 amino acids; in cases where the length of the shorter sequence is less than 400 amino acids, the sequence identity is not defined. The sequence identity can be easily determined using BLAST (Basic Local Alignment Search Tool), an algorithm widely used in this field. For example, BLAST is publicly available on a website, such as that of NCBI (National Center for Biotechnology Information) or KEGG (Kyoto Encyclopedia of Genes and Genomes), on which the sequence identity can be easily determined using default parameters. Additionally, the sequence identity can also be determined using a similar function implemented in a software program such as Genetyx.

Sequence identity (%) = the number of matches (without counting the number of gaps) / the length of a shorter sequence (excluding the terminal gaps) x 100

Formula (1)

By using a function of Genetyx (% Identity Matrix) to calculate sequence identities based on the formula (1) among the amino acid sequences represented by SEQ ID NOs: 1 to 7, the lowest sequence identity of 71.51% is found between the sequences represented by SEQ ID NOs: 2 and 4, and the sequence identities among the amino acid sequences represented by SEQ ID NOs: 1 to 7 are found to be at least not less than 70%. The results of calculation of sequence identity using Genetyx are presented in Table 1. In Table 1 below, the numbers in the leftmost column represent SEQ ID NOs.

[Table 1]

| [GENETYX :%Identity Matrix] ∗Gaps are NOT taken into account. [%] | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | ∗ | | | | | | |
| 2 Serratia nematodiphila DSM21420 | 98.23 | * | | | | | |
| 3 Serratia plymuthica NBRC102599 | 72.10 | 71.51 | * | | | | |
| 4 Serratia proteamaculans 568 | 72.29 | 71.51 | 86.24 | ∗ | | | |
| 5 Serratia ureilytica Lr5/4 | 90.76 | 90.76 | 72.88 | 73.28 | * | | |
| 6 Serratia sp. BW106 | 72.29 | 71.90 | 87.03 | 92.33 | 73.67 | ∗ | |
| 7 Serratia liquefacians FK01 | 72.29 | 71,70 | 84.67 | 86.83 | 73.47 | 87.81 | * |

| [Match Count/Length] | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | ∗ | | | | | | |
| 2 Serratia nematodiphila DSM21420 | 500/509 | * | | | | | |

(continued)

| [GENETYX : %Identity Matrix] *Gaps are NOT taken into account. | | | | | | | |
|---|---|---|---|---|---|---|---|
| 3 Serratia plymuthica NBRC102599 | 367/509 | 364/509 | * | | | | |
| 4 Serratia proteamaculans 568 | 368/509 | 364/509 | 439/509 | * | | | |
| 5 Serratia urailytica Lr5/4 | 462/509 | 462/509 | 371/509 | 373/509 | * | | |
| 6 Serratia sp. BW106 | 368/509 | 366/509 | 443/509 | 470/509 | 375/509 | * | |
| 7 Serratia liquefaciens FK01 | 368/509 | 365/509 | 431/509 | 442/509 | 374/509 | 447/509 | * |

When each of the amino acid sequences represented by SEQ ID NO: 1 to 7 as queries was compared using BLASTP to all the amino acid sequences registered in the NCBI amino acid database (non-redundant protein sequences) to determine sequence identities, all sequences with a sequence identity of not less than 70% were found to be from bacteria of the genus *Serratia.*

All the polypeptides represented by SEQ ID NOs: 1 to 7 as described above in (a) contain a common sequence 1 composed of 24 amino acid residues and represented by SEQ ID NO: 173 within a region from the 15th to the 38th amino acid residues from the N terminus (hereinafter, an amino acid residue at the n-th position from the N terminus may conveniently be represented by n "a.a."; for example, the region from the 15th to the 38th amino acid residues from the N terminus may be thus simply represented by "15 to 38 a.a."). In the common sequence 1, Xaa represents an arbitrary amino acid residue, and the 13 a.a. is preferably a phenylalanine or leucine, and the 15 a.a. is preferably a leucine or glutamine, and the 16 a.a. is preferably a lysine or asparagine, and the 17 a.a. is a glycine or serine, more preferably a glycine, and the 19 a.a. is preferably a proline or arginine, and the 21 a.a. is preferably a leucine, methionine, or valine. The common sequence 1 corresponds to the region including the $NAD^+$-binding residue and the surrounding amino acid residues. In the $NAD^+$-binding residues, the 24th amino acid residue in the common sequence 1 should be an aspartic acid, as described in Biochimie., 2012 Feb, 94 (2): 471-8., but in the common sequence 1, the residue is an asparagine, which is characteristic. It is thought that the presence of the common sequence 1 causes the polypeptides represented by SEQ ID NOs: 1 to 7 to show excellent enzymatic activity as 3-oxoadipyl-CoA reductases.

The polypeptides as described above in (b) and (c) also preferably contain the common sequence 1 composed of 24 amino acid residues and represented by SEQ ID NO: 173 within a region from 1 to 200 a.a. The common sequence is more preferably located within a region from 1 to 150 a.a., and further preferably within a region from 1 to 100 a.a. Specific examples of the polypeptides include those with the amino acid sequences represented by SEQ ID NOs: 8 to 86. The amino acid sequences represented by SEQ ID NOs: 8 to 86 contain the common sequence 1 composed of 24 amino acid residues and represented by SEQ ID NO: 173 within a region from 15 to 38 a.a. The amino acid sequences represented by SEQ ID NOs: 8 to 86 have a sequence identity of not less than 90% to the amino acid sequence represented by any one of SEQ ID NOs: 1 to 7. The results of calculation of sequence identity using Genetyx are presented in Tables 2-1 to 2-3 and Tables 3-1 to 3-3.

[Table 2-1]

| [GENETYX : %Identity Matrix] *Gaps are NOT taken into account. | | | | | | | |
|---|---|---|---|---|---|---|---|
| [%] | 1 Serratia | 2 Serratia | 3 *Serratia,* | 4 Serratia | 5 *Serratia* | 6 *Serratia* | 7 Serratia |
| 1 Serratia marcescens ATCC13880 | * | | | | | | |
| 2 *Serratia* nematodiphila DSM21420 | 98.23 | * | | | | | |

(continued)

| [GENETYX : %Identity Matrix] ∗Gaps are NOT taken into account. | | | | | | | |
|---|---|---|---|---|---|---|---|
| 3 Serratia plymuthica NBRC102599 | 72.10 | 71. 51 | ∗ | | | | |
| 4 Serratia proteamaculans 568 | 72.29 | 71. 51 | 86.24 | * | | | |
| 5 Serratia ureilytica Lr5/4 | 90.76 | 90.76 | 72.88 | 73.28 | ∗ | | |
| 6 Serratia sp. BW106 | 72.29 | 71.90 | 87.03 | 92.33 | 73.67 | ∗ | |
| 7 Serratia liquefaciens FK01 | 72.29 | 71.70 | 84.67 | 86.83 | 73.47 | 87.81 | * |
| 8 Serratia sp. S119 | 94.89 | 94.30 | 72.88 | 72.49 | 91.55 | 73,08 | 72.88 |
| 9 Serratia sp. YD25 | 92.33 | 92.33 | 72.49 | 72.49 | 93.51 | 72.69 | 72.88 |
| 10 Serratia sp. FS14 | 98.62 | 99.60 | 71.70 | 71.70 | 91.15 | 72.10 | 72.10 |
| 11 Serratia sp. HMSC15F11 | 94.89 | 94.30 | 73.28 | 73.28 | 91.35 | 73.47 | 73.47 |
| 12 Serratia sp. JKS000199 | 90.76 | 90.76 | 72.69 | 73.08 | 99.41 | 73.47 | 73.28 |
| 13 Serratia sp. TEL | 90.56 | 90.56 | 72.88 | 73.28 | 99.80 | 73.67 | 73.47 |
| 14 Serratia sp. ISTD04 | 90,56 | 90.56 | 72.49 | 73.08 | 99.41 | 73.47 | 73.28 |
| 15 Serratia sp. SCBI | 90,76 | 90.76 | 72.88 | 73.28 | 99.60 | 73.47 | 73.47 |
| 16 Serratia sp. S4 | 72.10 | 71.31 | 86.44 | 98.62 | 73.08 | 91.94 | 86.64 |
| 17 Serratia sp. C-1 | 72.49 | 71.90 | 98.03 | 86.05 | 73.28 | 86.64 | 84.08 |
| 18 Serratia marcescens 532 | 99.80 | 98.03 | 72.29 | 72,10 | 90.56 | 72.10 | 72.10 |
| 19 Serratia marcescens 2880STDY5683033 | 99.60 | 97.83 | 72.10 | 72.29 | 90.37 | 72.10 | 72.29 |
| 20 Serratia marcescens WW4 | 98.42 | 99.41 | 71.90 | 71.90 | 90.96 | 72.29 | 71.90 |
| 21 Serratia marcescens K27 | 98, 23 | 99, 21 | 71.31 | 71.31 | 90.96 | 71.70 | 71.70 |
| 22 Serratia marcescens 280 | 98.42 | 99.41 | 71.70 | 71.70 | 90.96 | 72.10 | 72.10 |
| 23 Serratia marcescens 19F | 98.42 | 99.41 | 71.51 | 71.70 | 90.96 | 72.10 | 72.10 |
| 24 Serratia marcescens 1185 | 98.23 | 99.60 | 71.31 | 71.31 | 90.37 | 71.70 | 71.51 |

[Table 2-2]

| | | | | | | | |
|---|---|---|---|---|---|---|---|
| 25 Serratia marcescens S217 | 98.23 | 99.21 | 71. 31 | 71.51 | 90.96 | 71.90 | 71.90 |
| 26 Serratia marcescens KHCo-24B | 98.03 | 99.80 | 71.31 | 71.31 | 90.56 | 71.70 | 71.90 |
| 27 Serratia marcescens Z6 | 98.03 | 98.01 | 71.70 | 71.90 | 90.56 | 72.29 | 71. 90 |
| 28 Serratia marcescens 546 | 97.83 | 99.21 | 71.51 | 71.70 | 90.37 | 72.10 | 71.70 |
| 29 Serratia nematodiphila MB:307 | 98.03 | 99.80 | 71.31 | 71.51 | 90.56 | 71.90 | 71.70 |
| 30 Serratia marcescens VGH107 | 98.03, | 99.01 | 71.31 | 71.51 | 90.56 | 71.90 | 71.90 |
| 31 Serratia marcescens MCB | 95.48 | 95.28 | 72.29 | 72.69 | 91.15 | 72.88 | 72.69 |
| 32 Serratia marcescens AH0650 | 95.67 | 95.48 | 72.29 | 72.69 | 90.76 | 73.28 | 72.69 |
| 33 Serratia marcescens UMH12 | 95.48 | 95.28 | 72.10 | 72.49 | 90.56 | 73.08 | 72.49 |
| 34 Serratia sp. OMLW3 | 95.48 | 95.28 | 72.29 | 72.49 | 90.76 | 73.28 | 72.69 |

(continued)

| | | | | | | | |
|---|---|---|---|---|---|---|---|
| 35 Serratia marcescens UMH11 | 95.28 | 95.08 | 72.10 | 72.69 | 90.56 | 73.47 | 72.49 |
| 36 Serratia marcescens UMH1 | 95.08 | 94.89 | 72.29 | 72. 49 | 90.17 | 73. 08 | 72.29 |
| 37 Serratia marcescens 2880STDY5883020 | 95.48 | 94.89 | 73.08 | 72.69 | 92.14 | 73.28 | 73.08 |
| 38 Serratia marcescens 99 | 95. 48 | 94.69 | 73.28 | 72.88 | 91.55 | 73.67 | 73. 28 |
| 39 Serratia marcescens 374 | 94.89 | 94.69 | 72.29 | 72.29 | 90.17 | 73.08 | 72.29 |
| 40 Serratia marcescens 2880S:TDY5683036 | 95.28 | 94.49 | 73.08 | 72.69 | 91.35 | 73.47 | 73.08 |
| 41 Serratia marcescens 2880STDY5683034 | 95.28 | 94.69 | 73. 08 | 72.69 | 91.94 | 73.28 | 73.08 |
| 42 Serratia marcescens 2880STDY5682892 | 95.28 | 94.69 | 73.28 | 72.88 | 91.94 | 73.47 | 73.28 |
| 43 Serratia marcescens SM39 | 95.08 | 94.49 | 73.28 | 72.69 | 92.14 | 73.28 | 73.28 |
| 44 Serratia marcescens 189 | 95.08 | 94.49 | 73.28 | 72.88 | 92.14 | 73.47 | 73.28 |
| 45 Serratia marcescens SMB2099 | 95.08 | 94.49 | 73.47 | 72.69 | 91.74 | 73.67 | 73.47 |
| 46 Serratia marcescens 2880S:TDY5682862 | 94.89 | 94.30 | 73.47 | 72.88 | 91.55 | 73.47 | 73.47 |
| 47 Serratia marcescens SE4145 | 94.89 | 94.30 | 73.08 | 72.49 | 91.94 | 73.08 | 73.08 |
| 48 Serratia marcescens 2880S:T0Y5682876 | 95.08 | 94.49 | 73.28 | 72.88 | 91.74 | 73.47 | 73.28 |
| 49 Serratia marcescens 709 | 95.08 | 94.49 | 73.08 | 72.69 | 91.74 | 73.28 | 73.08 |
| 50 Serratia marcescens MGH136 | 94. 89 | 94.30 | 72.88 | 72.49 | 91.94 | 73.08 | 72.88 |
| 51 Serratia marcescens 2880STDY5682884 | 94.69 | 94.10 | 72.88 | 72.49 | 91.74 | 73.08 | 73.08 |
| 52 Serratia marcescens D-3 | 95.08 | 94,49 | 73.08 | 72.69 | 91.74 | 73.28 | 73.08 |
| 53 Serratia marcescens 2880S:TDY5682957 | 94.89 | 94.30 | 72.88 | 72.69 | 91.55 | 73.28 | 72.88 |
| 54 Serratia marcescens YDC563 | 94.69 | 94.10 | 72.88 | 72.69 | 91.35 | 73.28 | 72.88 |
| 55 Serratia marcescens 2880STDY5683035 | 94.89 | 94.30 | 73.08 | 72.69 | 91.55 | 73.28 | 73.08 |

[Table 2-3]

| | | | | | | | |
|---|---|---|---|---|---|---|---|
| 56 Serratia marcescens 2880STDY5682930 | 94.69 | 94.10 | 72.88 | 72.49 | 91.35 | 73.08 | 72.88 |
| 57 Serratia marcescens 790 | 94.49 | 94.30 | 73.28 | 72.88 | 91.35 | 73.47 | 73.28 |
| 58 Serratia marcescens UMH5 | 93.51 | 92.92 | 72.69 | 72.88 | 90.37 | 72.69 | 72.49 |
| 59 Serratia marcescens 2880STOY5682988 | 93.32 | 92.73 | 72.69 | 72.88 | 90.17 | 72.69 | 72.49 |
| 60 Serratia marcescens 945154301 | 94.89 | 94.30 | 73.28 | 73.28 | 91.35 | 73.67 | 73.47 |
| 61 Serratia marcescens at10508 | 94.69 | 94.10 | 73.47 | 73.47 | 91.15 | 73.67 | 73.67 |
| 62 Serratia marcescens ML2637 | 94.49 | 93.90 | 73.28 | 73.47 | 90.96 | 73.67 | 73.67 |
| 63 Serratia marcescens SM1978 | 94.30 | 93.71 | 73.28 | 73.28 | 90.76 | 73.67 | 73.67 |
| 64 Serratia marcescens PWN146 | 94.10 | 93.51 | 72.88 | 72.88 | 90.96 | 72.88 | 73.28 |
| 65 Serratia marcescens H1q | 92.53 | 92.53 | 72.49 | 72.49 | 93.51 | 72.69 | 73. 08 |
| 66 Serratia marcescens UMH6 | 91.15 | 91.15 | 72.69 | 73.08 | 99.60 | 73.47 | 73.28 |
| 67 Serratia nematodiphila WCU338 | 91.15 | 91.15 | 72.69 | 73.08 | 99.41 | 73.47 | 73.28 |
| 68 Serratia sp. 0LEL1 | 90.96 | 90.96 | 72.88 | 73.28 | 99.80 | 73.67 | 73.47 |
| 69 Serratia marcescens 7209 | 90.96 | 90.96 | 72.49 | 72.88 | 99.41 | 73.28 | 73.08 |
| 70 Serratia marcescens sicaria (Ss1) | 90.96 | 90.96 | 72.69 | 73.08 | 99.41 | 73.28 | 73.28 |
| 71 Serratia sp. 0LFL2 | 90.76 | 90.76 | 72.69 | 73.08 | 99.60 | 73.47 | 73.28 |
| 72 Serratia marcescens BIDMC 81 | 90.76 | 90.76 | 72.88 | 73.28 | 99.60 | 73.67 | 73. 47 |
| 73 Serratia marcescens BIDMC 50 | 90.76 | 90.76 | 72.69 | 73.08 | 99.21 | 73.47 | 73.28 |
| 74 Serratia marcescens UMH7 | 90.56 | 90.56 | 72.88 | 73.28 | 99.80 | 73.67 | 73. 47 |
| 75 Serratia marcescens RSC-14 | 90.56 | 90.56 | 72. 88 | 73.47 | 99.21 | 73.87 | 73.67 |
| 76 Serratia marcescens SM03 | 92.33 | 92. 33 | 72. 29 | 72.29 | 93.51 | 72.49 | 72.88 |
| 77 Serratia marcescens 90-166 | 90.17 | 89.78 | 72.49 | 73.47 | 96.66 | 73.67 | 73.08 |
| 78 Serratia marcescens UMH2 | 90.76 | 90.76 | 72.88 | 73.28 | 99.21 | 73.67 | 73. 47 |
| 79 Serratia plymuthica AS9 | 72.49 | 71.90 | 96.66 | 85.06 | 73.47 | 86.05 | 83.69 |
| 80 Serratia plymuthica tumat 205 | 72.69 | 72. 10 | 98.03 | 86.24 | 73.47 | 86.64 | 84.28 |
| 81 Serratia plymuthica A30 | 72.29 | 71.70 | 98.82 | 85.65 | 72.88 | 86.44 | 84.08 |
| 82 Serratia plymuthica 4Rx13 | 72.29 | 71.70 | 97.83 | 85.85 | 73.08 | 86.44 | 84.28 |

(continued)

| | | | | | | | |
|---|---|---|---|---|---|---|---|
| 83 Serratia plymuthica V4 | 72.29 | 71. 70 | 98.42 | 85.85 | 73.08 | 86.44 | 84.28 |
| 84 Serratia plymuthica 3Rp8 | 72.29 | 71.70 | 98.62 | 86.05 | 73.08 | 86.64 | 84.08 |
| 85 Serratia proteamaculans MFPA44A14 | 72.29 | 71.90 | 87.03 | 92.53 | 73.28 | 98.82 | 87.22 |
| 86 Serratia plymuthica A153 | 72.10 | 71.51 | 99.21 | 86.05 | 72.88 | 86.64 | 84.47 |

[Table 3-1]

[Match Count/Length]

| | 1 Serratia | 2 Serratia | 3 Serratia | 4 Serratia | 5 Serratia | 6 Serratia | 7 Serratia |
|---|---|---|---|---|---|---|---|
| 1 Serratia marcescens ATCC13880 | * | | | | | | |
| 2 Serratia nematodiphila DSM21420 | 500/509 | * | | | | | |
| 3 Serratia plymuthica NBRC102599 | 367/509 | 364/509 | * | | | | |
| 4 Serratia proteamaculans 568 | 368/509 | 364/509 | 439/509 | * | | | |
| 5 Serratia ureilytica Lr5/4 | 462/509 | 462/509 | 371/509 | 373/509 | * | | |
| 6 Serratia sp. BW106 | 368/509 | 365/509 | 443/509 | 470/509 | 375/509 | * | |
| 7 Serratia liquefaciens FK01 | 368/509 | 365/509 | 431/509 | 442/509 | 374/509 | 447/509 | * |
| 8 Serratia sp. S119 | 483/509 | 480/509 | 371/509 | 369/509 | 466/509 | 372/509 | 371/509 |
| 9 Serratia sp. YD25 | 470/509 | 470/509 | 369/509 | 369/509 | 476/509 | 370/509 | 371/509 |
| 10 Serratia sp. FS14 | 502/509 | 507/509 | 365/509 | 365/509 | 464/509 | 367/509 | 367/509 |
| 11 Serratia sp. HMSC15F11 | 483/509 | 480/509 | 373/509 | 373/509 | 465/509 | 374/509 | 374/509 |
| 12 Serratia sp. JKS000199 | 462/509 | 462/509 | 370/509 | 372/509 | 506/509 | 374/509 | 373/509 |
| 13 Serratia sp, TEL | 461/509 | 461/509 | 371/509 | 373/509 | 508/509 | 375/509 | 374/509 |
| 14 Serratia sp. ISTD04 | 461/509 | 461/509 | 369/509 | 372/509 | 506/509 | 374/509 | 373/509 |
| 15 Serratia sp. SCBI | 462/509 | 462/509 | 371/509 | 373/509 | 507/509 | 374/509 | 374/509 |
| 16 Serratia sp. S4 | 367/509 | 363/509 | 440/509 | 502/509 | 372/509 | 468/509 | 441/509 |
| 17 Serratia sp. C-1 | 369/509 | 366/509 | 499/509 | 438/509 | 373/509 | 441/509 | 428/509 |
| 18 Serratia marcescens 532 | 508/509 | 499/509 | 368/509 | 367/509 | 461/509 | 367/509 | 367/509 |
| 19 Serratia marcescens 2880STDY5683033 | 507/509 | 498/509 | 367/509 | 368/509 | 460/509 | 367/509 | 368/509 |
| 20 Serratia marcescens WW4 | 501/509 | 506/509 | 366/509 | 366/509 | 463/509 | 368/509 | 366/509 |
| 21 Serratia marcescens K27 | 500/509 | 505/509 | 363/509 | 363/509 | 463/509 | 366/509 | 365/509 |
| 22 Serratia marcescens 280 | 501/509 | 500/509 | 365/509 | 365/509 | 463/509 | 367/509 | 367/509 |
| 23 Serratia marcescens 19F | 501/509 | 500/509 | 364/509 | 365/509 | 463/509 | 367/509 | 367/509 |
| 24 Serratia marcescens 1185 | 500/509 | 507/509 | 363/509 | 363/509 | 460/509 | 365/509 | 364/509 |

[Table 3-2]

| | | | | | | | |
|---|---|---|---|---|---|---|---|
| 25 Serratia marcescens S217 | 500/509 | 505/509 | 363/509 | 364/509 | 463/509 | 366/509 | 366/509 |
| 26 Serratia marcescens KHCo-24B | 499/509 | 508/509 | 363/509 | 363/509 | 461/509 | 365/509 | 366/509 |
| 27 Serratia marcescens Z6 | 499/509 | 504/509 | 365/509 | 366/509 | 461/509 | 368/509 | 366/509 |
| 28 Serratia marcescens 546 | 498/509 | 505/509 | 364/509 | 365/509 | 460/509 | 367/509 | 365/509 |
| 29 Serratia nematodiphila MB307 | 499/509 | 508/509 | 363/509 | 364/509 | 461/509 | 366/509 | 365/509 |
| 30 Serratia marcescens VGH107 | 499/509 | 504/509 | 363/509 | 364/509 | 461/509 | 366/509 | 366/509 |
| 31 Serratia marcescens MCB | 486/509 | 485/509 | 368/509 | 370/509 | 464/509 | 371/509 | 370/509 |
| 32 Serratia marcescens AH0650 | 487/509 | 486/509 | 368/509 | 370/509 | 462/509 | 373/509 | 370/509 |
| 33 Serratia marcescens UMH12 | 486/509 | 485/509 | 367/509 | 369/509 | 461/509 | 372/509 | 369/509 |
| 34 Serratia sp. OMLW3 | 486/509 | 485/509 | 368/509 | 369/509 | 462/509 | 373/509 | 370/509 |
| 35 Serratia marcescens UMH11 | 485/509 | 484/509 | 367/509 | 370/509 | 461/509 | 374/509 | 369/509 |
| 36 Serratia marcescens UMH1 | 484/509 | 483/509 | 368/509 | 369/509 | 459/509 | 372/509 | 368/509 |
| 37 Serratia marcescens 2880STDY5683020 | 486/509 | 483/509 | 372/509 | 370/509 | 469/509 | 373/509 | 372/509 |
| 38 Serratia marcescens 99 | 486/509 | 482/509 | 373/509 | 371/509 | 466/509 | 375/509 | 373/509 |
| 39 Serratia marcescens 374 | 483/509 | 482/509 | 368/509 | 368/509 | 459/509 | 372/509 | 368/509 |
| 40 Serratia marcescens 2880STDY5683036 | 485/509 | 481/509 | 372/509 | 370/509 | 465/509 | 374/509 | 372/509 |
| 41 Serratia marcescens 2880STDY5683034 | 485/509 | 482/509 | 372/509 | 370/509 | 468/509 | 373/509 | 372/509 |
| 42 Serratia marcescens 2880STTDY5682892 | 485/509 | 482/509 | 373/509 | 371/509 | 468/509 | 374/509 | 373/509 |
| 43 Serratia marcescens SM39 | 484/509 | 481/509 | 373/509 | 370/509 | 469/509 | 373/509 | 373/509 |
| 44 Serratia marcescens 189 | 484/509 | 481/509 | 373/509 | 371/509 | 469/509 | 374/509 | 373/509 |
| 45 Serratia marcescens SMB2099 | 484/509 | 481/509 | 374/509 | 370/509 | 467/509 | 375/509 | 374/509 |
| 46 Serratia marcescens 2880STDY5682862 | 483/509 | 480/509 | 374/509 | 371/509 | 466/509 | 374/509 | 374/509 |
| 47 Serratia marcescens SE4145 | 483/509 | 480/509 | 372/509 | 369/509 | 468/509 | 372/509 | 372/509 |
| 48 Serratia marcescens 2880STDY5682876 | 484/509 | 481/509 | 373/509 | 371/509 | 467/509 | 374/509 | 373/509 |
| 49 Serratia marcescens 709 | 484/509 | 481/509 | 372/509 | 370/509 | 467/509 | 373/509 | 372/509 |
| 50 Serratia marcescens MGH136 | 483/509 | 480/509 | 371/509 | 369/509 | 468/509 | 372/509 | 371/509 |
| 51 Serratia marcescens 2880STDY5682884 | 482/509 | 479/509 | 371/509 | 369/509 | 467/509 | 372/509 | 372/509 |
| 52 Serratia marcescens D-3 | 484/509 | 481/509 | 372/509 | 370/509 | 467/509 | 373/509 | 372/509 |
| 53 Serratia marcescens 2880STDY5682957 | 483/509 | 480/509 | 371/509 | 370/509 | 466/509 | 373/509 | 371/509 |
| 54 Serratia marcescens YDC563 | 482/509 | 479/509 | 371/509 | 370/509 | 465/509 | 373/509 | 371/509 |
| 55 Serratia marcescens 2880STDY5683035 | 483/509 | 480/509 | 372/509 | 370/S09 | 466/509 | 373/509 | 372/509 |

[Table 3-3]

| | | | | | | | |
|---|---|---|---|---|---|---|---|
| 56 Serratia marcescens 2880STDY5682930 | 482/509 | 479/509 | 371/509 | 369/509 | 465/509 | 372/509 | 371/509 |
| 57 Serratia marcescens 790 | 481/509 | 480/509 | 373/509 | 371/509 | 465/509 | 374/509 | 373/509 |
| 58 Serratia marcescens UMH5 | 476/509 | 473/509 | 370/509 | 371/509 | 460/509 | 370/509 | 369/509 |
| 59 Serratia marcescens 2880STDY5682988 | 475/509 | 472/509 | 370/509 | 371/509 | 459/509 | 370/509 | 369/509 |
| 60 Serratia marcescens 945154301 | 483/509 | 480/509 | 373/509 | 373/509 | 465/509 | 375/509 | 374/509 |
| 61 Serratia marcescens at10508 | 482/509 | 479/509 | 374/509 | 374/509 | 464/509 | 375/509 | 375/509 |
| 62 Serratia marcescens ML2637 | 481/509 | 478/509 | 373/509 | 374/509 | 463/509 | 375/509 | 375/509 |
| 63 Serratia marcescens SM1978 | 480/509 | 477/509 | 373/509 | 373/509 | 462/509 | 375/509 | 375/509 |
| 64 Serratia marcescens PWN146 | 479/509 | 476/509 | 371/509 | 371/509 | 463/509 | 371/509 | 373/509 |
| 65 Serratia marcescens H1q | 471/509 | 471/509 | 369/509 | 369/509 | 476/509 | 370/509 | 372/509 |
| 66 Serratia marcescens UMH6 | 464/509 | 464/509 | 370/509 | 372/509 | 507/509 | 374/509 | 373/509 |
| 67 Serratia nematodiphila WCU338 | 464/509 | 464/509 | 370/509 | 372/509 | 506/509 | 374/509 | 373/509 |
| 68 Serratia sp. 0LEL1 | 463/509 | 463/509 | 371/509 | 373/509 | 508/509 | 375/509 | 374/509 |
| 69 Serratia marcescens 7209 | 463/509 | 463/509 | 369/509 | 371/509 | 506/509 | 373/509 | 372/509 |
| 70 Serratia marcescens sicaria (Ss1) | 463/509 | 463/509 | 370/509 | 372/509 | 506/509 | 373/509 | 373/509 |
| 71 Serratia sp. 0LFL2 | 462/509 | 462/509 | 370/509 | 372/509 | 507/509 | 374/509 | 373/509 |
| 72 Serratia marcescens BIDMC 81 | 462/509 | 462/509 | 371/509 | 373/509 | 507/509 | 375/509 | 374/509 |
| 73 Serratia marcescens BIDMC 50 | 462/509 | 462/509 | 370/509 | 372/509 | 505/509 | 374/509 | 373/509 |
| 74 Serratia marcescens UMH7 | 461/509 | 461/509 | 371/509 | 373/509 | 508/509 | 375/509 | 374/509 |
| 75 Serratia marcescens RSC-14 | 461/509 | 461/509 | 371/509 | 374/509 | 505/509 | 376/509 | 375/509 |
| 76 Serratia marcescens SM03 | 470/509 | 470/509 | 368/509 | 368/509 | 476/509 | 369/509 | 371/509 |
| 77 Serratia marcescens 90-166 | 459/509 | 457/509 | 369/509 | 374/509 | 492/509 | 375/509 | 372/509 |
| 78 Serratia marcescens UMH2 | 462/509 | 462/509 | 371/509 | 373/509 | 505/509 | 375/509 | 374/509 |
| 79 Serratia plymuthica AS9 | 369/509 | 366/509 | 492/509 | 433/509 | 374/509 | 438/509 | 426/509 |
| 80 Serratia plymuthica tumat 205 | 370/509 | 367/509 | 499/509 | 439/509 | 374/509 | 441/509 | 429/509 |
| 81 Serratia plymuthica A30 | 368/509 | 365/509 | 503/509 | 436/509 | 371/509 | 440/509 | 428/509 |
| 82 Serratia plymuthica 4Rx13 | 368/509 | 365/509 | 498/509 | 437/509 | 372/509 | 440/509 | 429/509 |
| 83 Serratia plymuthica V4 | 368/509 | 365/509 | 501/509 | 437/509 | 372/509 | 440/509 | 429/509 |
| 84 Serratia plymuthica 3Rp8 | 368/509 | 365/509 | 502/509 | 438/509 | 372/509 | 441/509 | 428/509 |
| 85 Serratia proteamaculans MFPA44A14 | 368/509 | 366/509 | 443/509 | 471/509 | 373/509 | 503/509 | 444/509 |
| 86 Serratia plymuthica A153 | 367/509 | 364/509 | 505/509 | 438/509 | 371/509 | 441/509 | 430/509 |

The nucleic acids encoding the polypeptides described in (a) to (c) according to the present invention may contain an additional sequence that encodes a peptide or protein added to the original polypeptides at the N terminus and/or the C terminus. Examples of such a peptide or protein can include secretory signal sequences, translocation proteins, binding proteins, peptide tags for purification, and fluorescent proteins. Among those peptides or proteins, a peptide or protein with a desired function can be selected depending on the purpose and can be added to the polypeptides of the present invention by those skilled in the art. It should be noted that the amino acid sequence of such a peptide or

protein is excluded from the calculation of sequence identity.

The nucleic acids encoding the polypeptides represented by SEQ ID NOs: 1 to 86 are not specifically limited, as long as the nucleic acids have nucleotide sequences that can be translated to the amino acid sequences represented by SEQ ID NOs: 1 to 86, and the nucleotide sequences can be determined considering the set of codons (standard genetic code) corresponding to each amino acid. In this respect, the nucleotide sequences may be redesigned using codons that are frequently used by a host microorganism used in the present invention.

Specific examples of the nucleotide sequences of the nucleic acids that encode the polypeptides with the amino acid sequences represented by SEQ ID NOs: 1 to 86 include the nucleotide sequences represented by SEQ ID NOs: 87 to 172.

In the present invention, whether or not a polypeptide encoded by a certain nucleic acid has 3-oxoadipyl-CoA reductase activity is determined as follows: transformants A and B below are produced and grown in a culture test; if 3-hydroxyadipic acid or $\alpha$-hydromuconic acid is confirmed in the resulting culture medium, it is judged that the nucleic acid encodes a polypeptide having 3-oxoadipyl-CoA reductase activity. The determination method will be described using the above scheme 1 which shows a biosynthesis pathway.

The transformant A has enzymes that catalyze the reactions A, E, and F. The transformant B has enzymes that catalyze the reactions A, C, E, and F.

The transformant A is first produced. Plasmids that allow for expression of the enzymes that catalyze the reactions A, E, and F are produced. The reactions E and F can be catalyzed by an identical enzyme. The plasmids are introduced into *Escherichia coli* strain BL21 (DE3), which is a microorganism strain lacking abilities to produce all of 3-hydroxyadipic acid, $\alpha$-hydromuconic acid, and adipic acid. Into the obtained transformant, an expression plasmid carrying a nucleic acid that encodes a polypeptide to be analyzed for the presence of the enzymatic activity of interest and is integrated downstream of an appropriate promoter is introduced to obtain the transformant A. The transformant A is cultured, and the post-culture fluid is examined for the presence of 3-hydroxyadipic acid. Once the presence of 3-hydroxyadipic acid in the culture fluid is successfully confirmed, the transformant B is then produced. The transformant B is obtained by producing a plasmid for the expression of an enzyme that catalyzes the reaction C and introducing the resulting plasmid into the transformant A. The transformant B is cultured, and the post-culture fluid is examined for the presence of $\alpha$-hydromuconic acid. When the presence of $\alpha$-hydromuconic acid in the post-culture fluid is confirmed, it indicates that 3-hydroxyadipic acid produced in the transformant A and $\alpha$-hydromuconic acid produced in the transformant B are generated via production of 3-hydroxyadipyl-CoA, and that the polypeptide of interest has 3-oxoadipyl-CoA reductase activity.

As the gene encoding the enzyme that catalyzes the reaction A, *pcaF* from *Pseudomonas putida* strain KT2440 (NCBI Gene ID: 1041755; SEQ ID NO: 174) is used.

As the genes encoding the enzyme that catalyzes the reactions E and F, a continuous sequence including the full lengths of *pcaI* and *pcaJ* from *Pseudomonas putida* strain KT2440 (NCBI Gene IDs: 1046613 and 1046612; SEQ ID NOs: 175 and 176) is used. The polypeptides encoded by *pcaI* and *pcaJ* forms a complex and then catalyze the reactions E and F.

As the nucleic acid encoding the enzyme that catalyzes the reaction C, the *paaF* gene from *Pseudomonas putida* strain KT2440 (NCBI Gene ID: 1046932, SEQ ID NO: 177) is used.

The method of culturing the transformant A and the transformant B is as follows. Antibiotics for stable maintenance of the plasmids and inducer substances for induction of expression of the polypeptides encoded by the incorporated nucleic acids may be added as appropriate to the culture. A loopful of either the transformant A or B is inoculated into 5 mL of the culture medium I (10 g/L Bacto Tryptone (manufactured by Difco Laboratories), 5 g/L Bacto Yeast Extract (manufactured by Difco Laboratories), 5 g/L sodium chloride) adjusted at pH 7 and is cultured at 30°C with shaking at 120 min-1 for 18 hours to prepare a preculture fluid. Subsequently, 0.25 mL of the preculture fluid is added to 5 mL of the culture medium II (10 g/L succinic acid, 10 g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract) adjusted to pH 6.5 and is cultured at 30°C with shaking at 120 min-1 for 24 hours. The obtained culture fluid is examined for the presence of 3-hydroxyadipic acid or $\alpha$-hydromuconic acid.

The presence of 3-hydroxyadipic acid or $\alpha$-hydromuconic acid in the culture fluid can be confirmed by centrifuging the culture fluid and analyzing the supernatant with LC-MS/MS. The analysis conditions are as described below:

- HPLC: 1290 Infinity (manufactured by Agilent Technologies, Inc.)

Column: Synergi hydro-RP (manufactured by Phenomenex Inc.), length: 100 mm, internal diameter: 3 mm, particle size: 2.5 $\mu$m

Mobile phase: 0.1% aqueous formic acid solution / methanol = 70/30

Flow rate: 0.3 mL/min

Column temperature: 40°C
LC detector: DAD (210 nm)
• MS/MS: Triple-Quad LC/MS (manufactured by Agilent Technologies, Inc.)
Ionization method: ESI in negative mode.

The 3-oxoadipyl-CoA reductase activity value can be calculated by quantifying 3-hydroxyadipyl-CoA generated from 3-oxoadipyl-CoA used as a substrate by using purified 3-oxoadipyl-CoA reductase, wherein the 3-oxoadipyl-CoA is prepared from 3-oxoadipic acid by an enzymatic reaction. The specific method is as follows.

3-Oxoadipic acid can be prepared by a known method (for example, a method described in Reference Example 1 of WO 2017/099209).

Preparation of 3-oxoadipyl-CoA solution: A PCR using the genomic DNA of *Pseudomonas putida* strain KT2440 as a template is performed in accordance with routine procedures, to amplify a nucleic acid encoding a CoA transferase (*pcaI* and *pcaJ*; NCBI-GeneIDs: 1046613 and 1046612) in the full-length form. The nucleotide sequences of primers used in this PCR are, for example, those represented by SEQ ID NOs: 194 and 195. The amplified fragment is inserted into the *Kpn*I site of pRSF-1b (manufactured by Novagen), an expression vector for *E. coli,* in frame with the histidine-tag sequence. The plasmid is introduced into *E. coli* BL21 (DE3), and the enzyme is expressed from the plasmid under isopropyl-β-thiogalactopyranoside (IPTG) induction and is then purified using the histidine tag from the culture fluid in accordance with routine procedures to obtain a CoA transferase solution. The solution is used to prepare an enzymatic reaction solution for 3-oxoadipyl-CoA preparation with the following composition, and the enzymatic reaction solution is kept at 25°C for 3 minutes to allow the reaction to proceed and is then filtered through a UF membrane (Amicon Ultra-0.5mL 10K; manufactured by Merck Millipore) to remove the enzyme, and the obtained filtrate is designated as 3-oxoadipyl-CoA solution.

(Enzymatic Reaction Solution)

100 mM Tris-HCl (pH 8.2)
10 mM $MgCl_2$
0.5 mM succinyl-CoA
5 mM 3-oxoadipic acid sodium salt
2 μM CoA transferase.

Identification of 3-oxoadipyl-CoA reductase activity: A PCR using the genomic DNA of a microorganism strain of interest as a template is performed in accordance with routine procedures, to amplify a nucleic acid encoding 3-oxoadipyl-CoA reductase in the full-length form. The nucleotide sequences of primers used in this PCR are, for example, those represented by SEQ ID NOs: 196 and 197. The amplified fragment is inserted into the *Bam*HI site of pACYCDuet-1 (manufactured by Novagen), an expression vector for *E. coli,* in frame with the histidine-tag sequence. The plasmid is introduced into *E. coli* BL21 (DE3), and the enzyme is expressed from the plasmid under isopropyl-β-thiogalactopyranoside (IPTG) induction and is then purified using the histidine tag from the culture fluid in accordance with routine procedures to obtain a 3-oxoadipyl-CoA reductase solution. The 3-oxoadipyl-CoA reductase activity can be determined by using the enzyme solution to prepare an enzymatic reaction solution with the following composition and quantifying 3-hydroxyadipyl-CoA generated using the enzymatic reaction solution at 25°C.

(Enzymatic Reaction Solution)

100 mM Tris-HCl (pH 8.2)
10 mM $MgCl_2$
150 μL/mL 3-oxoadipyl-CoA solution
0.5 mM NADH
1 mM dithiothreitol
10 μM 3-oxoadipyl-CoA reductase.

Specific examples of the enzymes that catalyze the reactions A and C to G are presented below. As an enzyme that catalyzes the reaction A to generate 3-oxoadipyl-CoA, for example, an acyl transferase (β-ketothiolase) can be used. The acyl transferase is not limited to a particular number in the EC classification but is preferably an acyl transferase

classified into EC 2.3.1.-, specifically including an enzyme classified as 3-oxoadipyl-CoA thiolase and classified into EC number 2.3.1.174, an enzyme classified as acetyl-CoA C-acetyltransferase and classified into EC number 2.3.1.9, and an enzyme classified as acetyl-CoA C-acyl transferase and classified into EC number 2.3.1.16. Among these, PaaJ from *Escherichia coli* strain MG1655 (NCBI-Protein ID: NP_415915), PcaF from *Pseudomonas putida* strain KT2440 (NCBI-Protein ID: NP 743536), and the like can be suitably used.

Whether or not the above acyl transferases can generate 3-oxoadipyl-CoA from succinyl-CoA and acetyl-CoA as substrates can be determined by measuring a decrease in NADH coupled with reduction of 3-oxoadipyl-CoA in a combination of a reaction catalyzed by purified acyl transferase to generate 3-oxoadipyl-CoA and a reaction catalyzed by purified 3-oxoadipyl-CoA reductase to reduce 3-oxoadipyl-CoA as a substrate. The specific measurement method is, for example, as follows.

Identification of acyl transferase activity: A PCR using the genomic DNA of a subject microorganism strain as a template is performed in accordance with routine procedures, to amplify a nucleic acid encoding an acyl transferase in the full-length form. The amplified fragment is inserted into the *Sac*I site of pACYCDuet-1 (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain an acyl transferase solution. The acyl transferase activity can be determined by using the enzyme solution to prepare an enzymatic reaction solution with the following composition and measuring a decrease in absorbance at 340 nm coupled with oxidation of NADH at 30°C.

100 mM Tris-HCl (pH 8.0)
10 mM $MgCl_2$
0.1 mM succinyl-CoA
0.2 mM acctyl-CoA
0.2 mM NADH
1 mM dithiothreitol
10 μg/mL 3-oxoadipyl-CoA reductase
5 μg/mL acyl transferase.

Whether or not an enzyme originally expressed in a host microorganism used in the present invention has acyl transferase activity can be determined by performing the above-described measurement using CFE instead of purified acyl transferase. The specific measurement method targeted to *E. coli* is, for example, as follows.

Preparation of CFE: A loopful of *E. coli* strain MG1655 to be subjected to the measurement of the activity is inoculated into 5 mL of a culture medium (culture medium composition: 10 g/L tryptone, 5 g/L yeast extract, 5 g/L sodium chloride) adjusted to pH 7, and incubated at 30°C with shaking for 18 hours. The obtained culture fluid is added to 5 mL of a culture medium (culture medium composition: 10 g/L tryptone, 5 g/L yeast extract, 5 g/L sodium chloride, 2.5 mM ferulic acid, 2.5 mM *p*-coumaric acid, 2.5 mM benzoic acid, 2.5 mM *cis*,*cis*-muconic acid, 2.5 mM protocatechuic acid, 2.5 mM catechol, 2.5 mM 3OA, 2.5 mM 3-hydroxyadipic acid, 2.5 mM α-hydromuconic acid, 2.5 mM adipic acid, 2.5 mM phenylethylamine) adjusted to pH 7, and incubated at 30°C with shaking for 3 hours.

The obtained culture fluid is supplemented with 10 mL of 0.9% sodium chloride and then centrifuged to remove the supernatant from bacterial cells, and this operation is repeated three times in total to wash the bacterial cells. The washed bacterial cells are suspended in 1 mL of a Tris-HCl buffer composed of 100 mM Tris-HCl (pH 8.0) and 1 mM dithiothreitol, and glass beads (with a diameter of 0.1 mm) are added to the resulting suspension to disrupt the bacterial cells at 4°C with an ultrasonic disruptor. The resulting bacterial homogenate is centrifuged to obtain the supernatant, and 0.5 mL of the supernatant is filtered through a UF membrane (Amicon Ultra-0.5mL 10K; manufactured by Merck Millipore) to remove the resulting filtrate, followed by application of 0.4 mL of the Tris-HCl buffer to the UF membrane, and this operation is repeated three times in total to remove low-molecular-weight impurities, and the resulting supernatant is then resuspended in the Tris-HCl buffer to a final volume of 0.1 mL, which is designated as CFE. Instead of purified enzyme, 0.05 mL of the CFE is added to a total of 0.1 mL of the enzymatic reaction solution to determine the enzymatic activity.

As an enzyme that catalyzes the reaction C to generate 2,3-dehydroadipyl-CoA, for example, an enoyl-CoA hydratase can be used. The enoyl-CoA hydratase is not limited by a particular number in the EC classification, and is preferably an enoyl-CoA hydratase classified into EC 4.2.1.-, specifically including an enzyme classified as enoyl-CoA hydratase or 2,3-dehydroadipyl-CoA hydratase and classified into EC 4.2.1.17. Among them, PaaF from *Escherichia coli* strain MG1655 (NCBI-ProteinID: NP_415911), PaaF from *Pseudomonas putida* strain KT2440 (NCBI-ProteinID: NP_745427), and the like can be suitably used.

Since the reaction catalyzed by enoyl-CoA hydratase is generally reversible, whether or not an enoyl-CoA

hydratase has an activity to catalyze a reaction that generates 2,3-dehydroadipyl-CoA from 3-hydroxyadipyl-CoA used as a substrate can be determined by detecting 3-hydroxyadipyl-CoA generated using purified enoyl-CoA hydratase with 2,3-dehydroadipyl-CoA used as a substrate thereof, which is prepared from α-hydromuconic acid through an enzymatic reaction. The specific measurement method is, for example, as follows.

Preparation of α-hydromuconic acid: Preparation of α-hydromuconic acid is performed according to the method described in Reference Example 1 of WO 2016/199858 A1.

Preparation of 2,3-dehydroadipyl-CoA solution: A PCR using the genomic DNA of *Pseudomonas putida* strain KT2440 as a template is performed in accordance with routine procedures, to amplify a nucleic acid encoding a CoA transferase (including *pcaI* and *pcaJ*; NCBI-GeneIDs: 1046613 and 1046612) in the full-length form. The amplified fragment is inserted into the *Kpn*I site of pRSF-1b (manufactured by Novagen), an expression vector for *E. coli*, in-frame with the histidine-tag sequence. The plasmid is introduced into *E. coli* BL21 (DE3), and expression of the enzyme is induced with isopropyl-p-thiogalactopyranoside (IPTG) in accordance with routine procedures and the enzyme is purified using the histidine tag from the culture fluid to obtain a CoA transferase solution. The solution is used to prepare an enzymatic reaction solution for 2,3-dehydroadipyl-CoA preparation with the following composition, which is allowed to react at 30°C for 10 minutes and then filtered through a UF membrane (Amicon Ultra-0.5mL 10K; manufactured by Merck Millipore) to remove the enzyme, and the obtained filtrate is designated as 2,3-dehydroadipyl-CoA solution.

Enzymatic reaction solution for 2,3-dehydroadipyl-CoA preparation

100 mM Tris-HCl (pH 8.0)
10 mM $MgCl_2$
0.4 mM succinyl-CoA
2 mM α-hydromuconic acid sodium salt
20 μg/mL CoA transferase.

Identification of enoyl-CoA hydratase activity: A PCR using the genomic DNA of a subject microorganism strain as a template is performed in accordance with routine procedures, to amplify a nucleic acid encoding an enoyl-CoA hydratase in the full-length form. The amplified fragment is inserted into the *Nde*I site of pET-16b (manufactured by Novagen), an expression vector for *E. coli,* in-frame with the histidine-tag sequence. The plasmid is introduced into E. *coli* BL21 (DE3), and expression of the enzyme is induced with isopropyl-β-thiogalactopyranoside (IPTG) in accordance with routine procedures and the enzyme is purified using the histidine tag from the culture fluid to obtain an enoyl-CoA hydratase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The enoyl-CoA hydratase activity can be confirmed by detecting 3-hydroxyadipyl-CoA in the resulting filtrate on high-performance liquid chromatograph-tandem mass spectrometer (LC-MS/MS) (Agilent Technologies, Inc.).

100 mM Tris-HCl (pH 8.0)
10 mM $MgCl_2$
300 μL/mL 2,3-dehydroadipyl-CoA solution
1 mM dithiothreitol
20 μg/mL enoyl-CoA hydratase.

Whether or not an enzyme originally expressed in a host microorganism used in the present invention has enoyl-CoA hydratase activity can be determined by adding 0.05 mL of the CFE, instead of purified enoyl-CoA hydratase, to a total of 0.1 mL of the enzymatic reaction solution and performing the above-described measurement. The specific CFE preparation method targeted to *E. coli* is as described for that used in determination of acyl transferase activity.

As an enzyme that catalyzes the reaction D to generate adipyl-CoA, for example, an enoyl-CoA reductase can be used. The enoyl-CoA reductase is not limited by a particular number in the EC classification, and is preferably an enoyl-CoA reductase classified into EC 1.3.-.-, specifically including an enzyme classified as *trans*-2-enoyl-CoA reductase and classified into EC 1.3.1.44, and an enzyme classified as acyl-CoA dehydrogenase and classified into EC 1.3.8.7. These specific examples are disclosed in, for example JP 2011-515111 A, J Appl Microbiol. 2015 Oct;119 (4): 1057-63., and the like; among them, TER from *Euglena gracilis* strain Z (UniProtKB: Q5EU90), Tfu_1647 from *Thermobifida fusca* strain YX (NCBI-ProteinID: AAZ55682), DcaA from *Acinetobacter baylyi* strain ADP1 (NCBI-ProteinID: AAL09094.1), and the like can be suitably used.

The fact that an enoyl-CoA reductase has an activity to generate adipyl-CoA from 2,3-dehydroadipyl-CoA used as a substrate can be confirmed by measuring a decrease in NADH coupled with reduction of 2,3-dehydroadipyl-CoA in a reaction using purified enoyl-CoA reductase with 2,3-dehydroadipyl-CoA used as a substrate thereof, which is prepared from α-hydromuconic acid through another enzymatic reaction.

Preparation of α-hydromuconic acid and of 2,3-dehydroadipyl-CoA solution can be performed in the same manner as described above.

Identification of enoyl-CoA reductase activity: A PCR using the genomic DNA of a subject microorganism strain as a template is performed in accordance with routine procedures, to amplify a nucleic acid encoding an enoyl-CoA reductase in the full-length form. The amplified fragment is inserted into the *Nde*I site of pET-16b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain an enoyl-CoA reductase solution. The enoyl-CoA reductase activity can be determined by using the enzyme solution to prepare an enzymatic reaction solution with the following composition and measuring a decrease in absorbance at 340 nm coupled with oxidation of NADH at 30°C.

100 mM Tris-HCl (pH 8.0)
10 mM $MgCl_2$
300 μL/mL 2,3-dehydroadipyl-CoA solution
0.2 mM NADH
1 mM dithiothreitol
20 μg/mL enoyl-CoA reductase.

Whether or not an enzyme originally expressed in a host microorganism used in the present invention has enoyl-CoA reductase activity can be determined by adding 0.05 mL of the CFE, instead of purified enoyl-CoA reductase, to a total of 0.1 mL of the enzymatic reaction solution and performing the above-described measurement. The specific CFE preparation method targeted to *E. coli* is as described for that used in determination of acyl transferase activity.

As an enzyme that catalyzes the reaction E to generate 3-hydroxyadipic acid, the reaction F to generate α-hydromuconic acid, and the reaction G to generate adipic acid, for example, a CoA transferase or an acyl-CoA hydrolase, preferably a CoA transferase, can be used.

The CoA transferase is not limited by a particular number in the EC classification, and is preferably a CoA transferase classified into EC 2.8.3.-, specifically including an enzyme classified as CoA transferase or acyl-CoA transferase and classified into EC 2.8.3.6, and the like.

In the present invention, the term "CoA transferase" refers to an enzyme with activity (CoA transferase activity) to catalyze a reaction that generates carboxylic acid and succinyl-CoA from acyl-CoA and succinic acid used as substrates.

As an enzyme that catalyzes the reaction E to generate 3-hydroxyadipic acid and the reaction F to generate α-hydromuconic acid, PcaI and PcaJ from *Pseudomonas putida* strain KT2440 (NCBI-ProteinIDs: NP_746081 and NP_746082), and the like can be suitably used, among others.

As an enzyme that catalyzes the reaction G to generate adipic acid, DcaI and DcaJ from *Acinetobacter baylyi* strain ADP1 (NCBI-ProteinIDs: CAG68538 and CAG68539), and the like can be suitably used.

Since the above enzymatic reactions are reversible, the CoA transferase activity against 3-hydroxyadipyl-CoA, 2,3-dehydroadipyl-CoA, or adipyl-CoA used as a substrate can be determined by detecting 3-hydroxyadipyl-CoA, 2,3-dehydroadipyl-CoA, or adipyl-CoA generated respectively using purified CoA transferase with 3-hydroxyadipic acid and succinyl-CoA, α-hydromuconic acid and succinyl-CoA, or adipic acid and succinyl-CoA used as substrates thereof. The specific measurement method is, for example, as follows.

Preparation of 3-hydroxyadipic acid: Preparation of 3-hydroxyadipic acid is performed according to the method described in Reference Example 1 of WO 2016/199856 A1.

Identification of CoA transferase activity using 3-hydroxyadipic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can

be confirmed by detecting 3-hydroxyadipyl-CoA in the resulting filtrate on high-performance liquid chromatograph-tandem mass spectrometer (LC-MS/MS) (Agilent Technologies, Inc.).

100 mM Tris-HCl (pH 8.0)
10 mM $MgCl_2$
0.4 mM succinyl-CoA
2 mM 3-hydroxyadipic acid sodium salt
20 μg/mL CoA transferase.

Preparation of α-hydromuconic acid: Preparation of α-hydromuconic acid is performed according to the method described in Reference Example 1 of WO 2016/199858 A1.

Identification of CoA transferase activity using α-hydromuconic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can be confirmed by detecting 2,3-dehydroadipyl-CoA in the resulting filtrate on high-performance liquid chromatograph-tandem mass spectrometer (LC-MS/MS) (Agilent Technologies, Inc.).

100 mM Tris-HCl (pH 8.0)
10 mM $MgCl_2$
0.4 mM succinyl-CoA
2 mM α-hydromuconic acid sodium salt
20 μg/mL CoA transferase.

Identification of CoA transferase activity using adipic acid as a substrate: A PCR using the genomic DNA of a subject microorganism strain as a template is performed in accordance with routine procedures, to amplify a nucleic acid encoding a CoA transferase in the full-length form. The amplified fragment is inserted into the *Kpn*I site of pRSF-1b (manufactured by Novagen), an expression vector for *E. coli,* in-frame with the histidine-tag sequence. The plasmid is introduced into *E. coli* BL21 (DE3), and expression of the enzyme is induced with isopropyl-β-thiogalactopyranoside (IPTG) in accordance with routine procedures and the enzyme is purified using the histidine tag from the culture fluid to obtain a CoA transferase solution. The solution is used to prepare an enzymatic reaction solution with the following composition, which is allowed to react at 30°C for 10 minutes and then filtered through a UF membrane (Amicon Ultra-0.5mL 10K; manufactured by Merck Millipore) to remove the enzyme. The CoA transferase activity can be confirmed by detecting adipyl-CoA in the resulting filtrate on high-performance liquid chromatograph-tandem mass spectrometer (LC-MS/MS) (Agilent Technologies, Inc.).

100 mM Tris-HCl (pH 8.0)
10 mM $MgCl_2$
0.4 mM succinyl-CoA
2 mM adipic acid sodium salt
20 μg/mL CoA-transferase.

Whether or not an enzyme originally expressed in a host microorganism used in the present invention has CoA transferase activity can be determined by adding 0.05 mL of the CFE, instead of purified CoA transferase, to a total of 0.1 mL of the enzymatic reaction solution and performing the above-described measurement. The specific CFE preparation method targeted to *E. coli* is as described for that used in determination of acyl transferase activity.

In the present invention, where a nucleic acid encoding any one selected from the group consisting of a PEP carboxykinase, an acyl transferase, a 3-oxoadipyl-CoA reductase, an enoyl-CoA hydratase, an enoyl-CoA reductase, and a CoA transferase is introduced into a host microorganism, the nucleic acid may be artificially synthesized based on the amino acid sequence information of the enzyme in a database or be isolated from the natural environment. In

cases where the nucleic acid is artificially synthesized, the usage frequency of codons corresponding to each amino acid in the nucleic acid sequence may be changed depending on the host microorganism into which the nucleic acid is introduced.

In cases where a nucleic acid encoding any one of the enzymes is isolated from the natural environment, the sources of the genes are not limited to particular organisms, and examples of the organisms include those of the genus *Acinetobacter*, such as *Acinetobacter baylyi* and *Acinetobacter radioresistens*; the genus *Aerobacter*, such as *Aerobacter cloacae*; the genus *Alcaligenes*, such as *Alcaligenes faecalis*; the genus *Bacillus*, such as *Bacillus badius*, *Bacillus magaterium*, and *Bacillus roseus*; the genus *Brevibacterium*, such as *Brevibacterium iodinum*; the genus *Corynebacterium*, such as *Corynebacterium acetoacidophilum*, *Corynebacterium acetoglutamicum*, *Corynebacterium ammoniagenes*, and *Corynebacterium glutamicum*; the genus *Cupriavidus*, such as *Cupriavidus metallidurans*, *Cupriavidus necator*, *Cupriavidus numazuensis*, and *Cupriavidus oxalaticus*; the genus *Delftia*, such as *Delftia acidovorans*; the genus *Escherichia*, such as *Escherichia coli* and *Escherichia fergusonii*; the genus *Hafnia*, such as *Hafnia alvei*; the genus *Microbacterium*, such as *Microbacterium ammoniaphilum*; the genus *Nocardioides*, such as *Nocardioides albus*; the genus *Planomicrobium*, such as *Planomicrobium okeanokoites*; the genus *Pseudomonas*, such as *Pseudomonas azotoformans*, *Pseudomonas chlororaphis, Pseudomonas fluorescens*, *Pseudomonas fragi*, *Pseudomonas putida*, and *Pseudomonas reptilivora*; the genus *Rhizobium*, such as *Rhizobium radiobacter*; the genus *Rhodosporidium*, such as *Rhodosporidium toruloides*; the genus *Saccharomyces*, such as *Saccharomyces cerevisiae*; the genus *Serratia,* such as *Serratia entomophila*, *Serratia ficaria*, *Serratia fonticola*, *Serratia grimesii*, *Serratia nematodiphila*, *Serratia odorifera*, and *Serratia plymuthica*; the genus *Shimwellia*, such as *Shimwellia blattae*; the genus *Streptomyces*, such as *Streptomyces vinaceus*, *Streptomyces karnatakensis*, *Streptomyces olivaceus*, and *Streptomyces vinaceus*; the genus *Yarrowia*, such as *Yarrowia lipolytica*; the genus *Yersinia*, such as *Yersinia ruckeri*; the genus *Euglena*, such as *Euglena gracilis*; and the genus *Thermobifida*, such as *Thermobifida fusca*; and preferably include those of the genera *Acinetobacter*, *Corynebacterium*, *Escherichia*, *Pseudomonas*, *Serratia*, *Euglena*, and *Thermobifida.*

In the present invention, impairing the function of pyruvate kinase or a phosphotransferase system enzyme means impairing the enzymatic activity of the enzyme. The method of impairment of the function is not limited to a particular method, but the function can be impaired, for example, by disrupting a gene that encodes the enzyme, such as via partial or complete deletion of the gene by mutagenesis with a chemical mutagen, ultraviolet irradiation, or the like, or by site-directed mutagenesis or the like, or via introduction of a frame-shift mutation or a stop codon into the nucleotide sequence of the gene. Alternatively, recombinant DNA technologies can be used to disrupt the gene by partial or complete deletion of the nucleotide sequence or by partial or complete substitution of the nucleotide sequence with another nucleotide sequence. Among these, the methods for partial or complete deletion of the nucleotide sequence are preferred.

Pyruvate kinase is classified as EC 2.7.1.40 and is an enzyme that catalyzes a reaction to dephosphorylate phosphoenolpyruvic acid to pyruvic acid and ATP. Specific examples of pyruvate kinase include pykF (NCBI-Protein ID: NP_416191, SEQ ID NO: 178) and pykA (NCBI-Protein ID: NP416368, SEQ ID NO: 179) from *Escherichia coli* strain K-12 substrain MG1655, and pykF (SEQ ID NO: 180) and pykA (SEQ ID NO: 181) from *Serratia grimesii* strain NBRC13537. In cases where a microorganism used in the present invention has two or more genes that each encode a pyruvate kinase, as illustrated in the metabolic pathway shown in the scheme 2 below, it is desirable to impair the function of all the pyruvate kinases. Whether or not a polypeptide encoded by a certain gene of a microorganism used in the present invention is a pyruvate kinase may be determined by BLAST (Basic Local Alignment Search Tool) searching on a website, such as that of NCBI (National Center for Biotechnology Information) or KEGG (Kyoto Encyclopedia of Genes and Genomes).

Phosphoenolpyruvate carboxykinase is classified as EC 4.1.1.49 and is an enzyme that catalyzes a reaction to generate oxaloacetic acid and ATP from phosphoenolpyruvic acid, carbon dioxide, and ADP. Specific examples of phosphoenolpyruvate carboxykinase include pck from *Escherichia coli* strain K-12 substrain MG1655 (NCBI-Protein ID: NP_417862, SEQ ID NO: 182) and pckA_1 (SEQ ID NO: 183) and pckA_2 (SEQ ID NO: 184) from *Serratia grimesii* strain NBRC13537.

Physiologically, PEP carboxykinase is responsible for a major reaction to produce glucose from fatty acids in the gluconeogenesis pathway. Though a reaction catalyzed by PEP carboxykinase is a reversible reaction, the reaction in the gluconeogenesis pathway proceeds in a direction which promotes conversion of oxaloacetic acid to PEP and carbon dioxide.

Whether or not a polypeptide encoded by a certain enzyme gene used in the present invention is a PEP carboxykinase may be determined by BLAST (Basic Local Alignment Search Tool) searching on a website, such as that of NCBI (National Center for Biotechnology Information) or KEGG (Kyoto Encyclopedia of Genes and Genomes).

JP 2015-146810 A describes that by means of metabolic network modeling, disruption of the PEP carboxykinase gene is found to be effective in the *in silico* generation of a microorganism strain capable of producing adipic acid from acetyl-CoA and succinyl-CoA in high yield. Additionally, JP 2015-504688 A describes that the PEP carboxykinase activity is enhanced for the purpose of increasing the pool of PEP for the production of muconic acid, which is produced

biosynthetically from PEP. That is, from the description that the reaction catalyzed by PEP carboxykinase proceeds in a direction which promotes generation of PEP from oxaloacetic acid, it is appreciated by those skilled in the art that enhancement of the PEP carboxykinase activity by increased expression of the gene encoding the enzyme would result in decreased yields of 3-hydroxyadipic acid, α-hydromuconic acid, and/or adipic acid. However, in the present invention, it has been found that the production of 3-hydroxyadipic acid, α-hydromuconic acid, and/or adipic acid is increased in a genetically modified microorganism with an ability to produce 3-hydroxyadipic acid, α-hydromuconic acid, and/or adipic acid and with a pyruvate kinase defect and with increased expression of the phosphoenolpyruvate carboxykinase gene and of a gene encoding an enzyme that catalyzes the reaction of reducing 3-oxoadipyl-CoA to 3-hydroxyadipyl-CoA when the genetically modified microorganism is cultured, which is contrary to the above expectation.

In the genetically modified microorganism of the present invention, it is desirable that the function of a phosphotransferase system enzyme be further impaired. The phosphotransferase system enzyme refers to the phosphoenolpyruvate-dependent phosphotransferase system (PTS) (in this specification, also referred to as a PTS enzyme). PTS is a major mechanism for the uptake of carbohydrates such as hexose, hexitol, and disaccharide into a cell, as illustrated in the metabolic pathway shown in the scheme 2 below. PTS involves uptake of carbohydrates into a cell and simultaneous conversion of the carbohydrates to a phosphate ester, while converting a phosphate donor, PEP, to pyruvic acid.

Acetyl-CoA, an intermediate produced in the biosynthesis of 3-hydroxyadipic acid, α-hydromuconic acid, and/or adipic acid, can be synthesized from PEP via production of pyruvic acid by the functions of PTS enzymes.

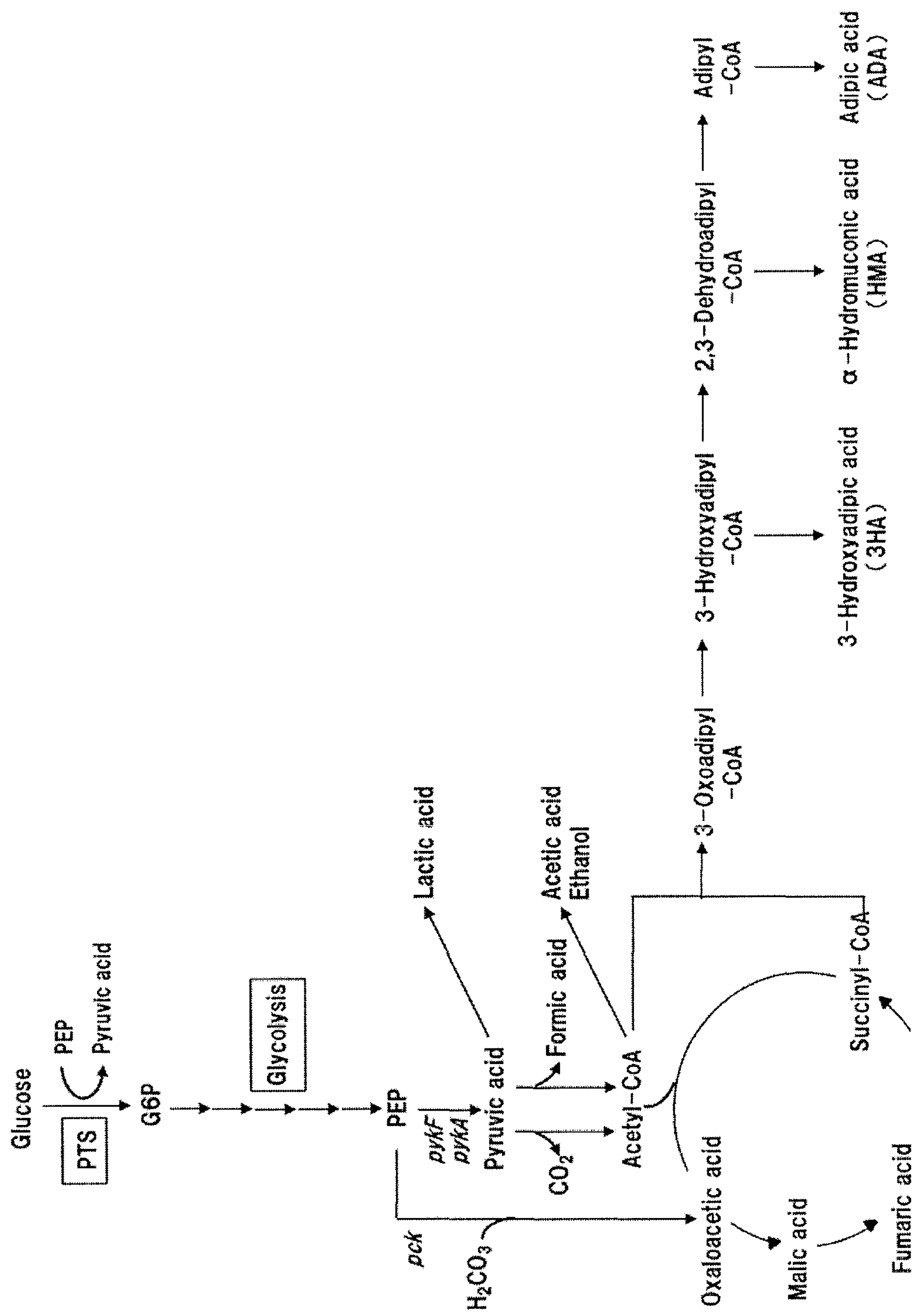


Scheme 2

PTS enzymes are composed of two common enzymes that exert their functions on any type of carbohydrate, phosphoenolpyruvate sugar phosphotransferase enzyme I and phospho carrier protein HPr, and membrane-bound sugar specific permeases (enzymes II) that are specific for particular carbohydrates. The enzymes II are further composed of sugar-specific components IIA, IIB, and IIC. The enzymes II exist as independent proteins or as fused domains in a single protein, and this depends on the organism which those enzymes are originated from.

In microorganisms, phosphoenolpyruvate sugar phosphotransferase enzyme I is encoded by the *ptsI* gene; phospho carrier protein HPr is encoded by the *ptsH* gene; glucose-specific enzyme IIA is encoded by the *crr* gene; and glucose-specific enzymes IIB and IIC are encoded by the *ptsG* gene.

The enzyme encoded by the *ptsG* gene is classified as EC 2.7.1.199 and is called protein-Npi-phosphohistidine-D-glucose phosphotransferase, and examples of the enzyme include PtsG from *Escherichia coli* strain K-12 substrain MG1655 (NCBI-Protein ID: NP_415619) and PtsG from *Serratia grimesii* strain NBRC13537 (SEQ ID NO: 185). Whether

or not a polypeptide encoded by a certain gene of a microorganism used in the present invention is a PTS enzyme may be determined by BLAST searching on a website, such as that of NCBI or KEGG.

In the present invention, one or more of the above PTS enzyme genes may be disrupted. Although any of the above PTS enzyme genes may be disrupted, it is desirable to impair an enzyme gene that is involved in glucose uptake, particularly the *ptsG* gene. Specific examples of the *ptsG* gene include *ptsG* from *Escherichia coli* strain K-12 substrain MG1655 (NCBI-Gene ID: 945651) and *ptsG* from *Serratia grimesii* strain NBRC13537 (SEQ ID NO: 243).

As described above, *E. coli* is a microorganism that has an ability to produce 3-hydroxyadipic acid and α-hydromuconic acid, and JP 2008-527991 A describes production of a mutant *E. coli* strain with defects in the *pykF* and *pykA* genes, which each encode a pyruvate kinase, and in the *ptsG* gene, which encodes a phosphotransferase system enzyme, wherein the yield of succinic acid is increased and the yields of acetic acid and ethanol are decreased, by culturing the mutant strain under anaerobic conditions. In this respect, acetic acid and ethanol are compounds generated from the metabolism of acetyl-CoA, as illustrated in the metabolic pathway shown in the above scheme 2. That is, in JP 2008-527991 A, it is presumed that the defects of the *ptsG*, *pykF*, and *pykA* genes in *E. coli* resulted in a reduced supply of acetyl-CoA and in turn a lower yield of acetic acid and ethanol.

On the other hand, the 3-hydroxyadipic acid, α-hydromuconic acid, and/or adipic acid produced by the method of the present invention are compounds generated through a plurality of reactions in the metabolism of 3-oxoadipyl-CoA, which is produced from acetyl-CoA and succinyl-CoA by the reaction A, as described above. Accordingly, from the description in JP 2008-527991 A, it is expected that disruption of genes encoding pyruvate kinase and a phosphotransferase system enzyme also results in a decreased yields of 3-hydroxyadipic acid, α-hydromuconic acid, and/or adipic acid due to the reduced supply of acetyl-CoA. However, in the present invention, disruption of genes encoding pyruvate kinase and a phosphotransferase system enzyme increases the yields of 3-hydroxyadipic acid, α-hydromuconic acid, and/or adipic acid and also the yield of acetic acid in a genetically modified microorganism with enhanced activities of phosphoenolpyruvate carboxykinase and of an enzyme that catalyzes the reaction of reducing 3-oxoadipyl-CoA to 3-hydroxyadipyl-CoA, which is contrary to the above expectation.

The genetically modified microorganism of the present invention is cultured in a culture medium, preferably a liquid culture medium, containing a carbon source available to ordinary microorganisms as a raw material for fermentation. The culture medium used contains, in addition to the carbon source available to the genetically modified microorganism, appropriate amounts of a nitrogen source and inorganic salts, and organic trace nutrients such as amino acids and vitamins as necessary. Either a natural or synthetic culture medium can be used as long as the medium contains the above-described nutrients.

The material for fermentation is a material that can be metabolized by the genetically modified microorganism. The term "metabolize" refers to conversion of a chemical substance, which a microorganism has taken up from the extracellular environment or intracellularly generated from a different chemical substance, to another chemical substance through an enzymatic reaction. Sugars can be suitably used as the carbon source. Specific examples of the sugars include monosaccharides, such as glucose, sucrose, fructose, galactose, mannose, xylose, and arabinose; disaccharides and polysaccharides formed by linking these monosaccharides; and saccharified starch solution, molasses, and saccharified solution from cellulose-containing biomass, each containing any of those saccharides.

The above-listed carbon sources may be used individually or in combination. However, it is especially preferred that the genetically modified microorganism be cultured in a culture medium containing glucose. In the addition of a carbon source, the concentration of the carbon source in a culture medium is not specifically limited and can be appropriately set depending on the type of the carbon source. The concentration is preferably from 5 g/L to 300 g/L in the case of glucose.

As the nitrogen source used for culturing the genetically modified microorganism, for example, ammonia gas, aqueous ammonia, ammonium salts, urea, nitric acid salts, other supportively used organic nitrogen sources, such as oil cakes, soybean hydrolysate, casein degradation products, other amino acids; vitamins, corn steep liquor, yeast or yeast extract, meat extract, peptides such as peptone, and bacterial cells and hydrolysate of various fermentative bacteria can be used. The concentration of the nitrogen source in the culture medium is not particularly limited, and is preferably from 0.1 g/L to 50 g/L.

As the inorganic salts used for culturing the genetically modified microorganism, for example, phosphoric acid salts, magnesium salts, calcium salts, iron salts, and manganese salts can be appropriately added to the culture medium and used.

The culture conditions for the genetically modified microorganism to produce 3-hydroxyadipic acid, α-hydromuconic acid, and/or adipic acid are set by appropriately adjusting or selecting, for example, the culture medium with the above composition, culture temperature, stirring speed, pH, aeration rate, and inoculation amount, depending on, for example, the species of the genetically modified microorganism and external conditions.

The pH range of the culture is not specifically limited, as long as the genetically modified microorganism can be grown in the pH range. However, the pH range is preferably from pH 5 to 8, more preferably from pH 5.5 to 6.8.

Although the range of aeration rates in the culture is not specifically limited, as long as 3-hydroxyadipic acid,

α-hydromuconic acid, and/or adipic acid can be produced under the aeration conditions, it is preferred that oxygen remain in the gaseous phase and/or liquid phase in a culture container for good growth of the mutant microorganism at least at the start of incubation.

In cases where foam is formed in a liquid culture, an antifoaming agent such as a mineral oil, silicone oil, or surfactant may be appropriately added to the culture medium.

After a recoverable amount of 3-hydroxyadipic acid, α-hydromuconic acid, and/or adipic acid is produced during culturing of the microorganism, the produced products can be recovered. The produced products can be recovered, for example isolated, according to a commonly used method, in which the culturing is stopped once a product of interest is accumulated to an appropriate level, and the fermentation product is collected from the culture. Specifically, the products can be isolated from the culture by separation of bacterial cells through, for example, centrifugation or filtration prior to, for example, column chromatography, ion exchange chromatography, activated charcoal treatment, crystallization, membrane separation, or distillation. More specifically, examples include, but are not limited to, a method in which an acidic component is added to salts of the products, and the resulting precipitate is collected; a method in which water is removed from the culture by concentration using, for example, a reverse osmosis membrane or an evaporator to increase the concentrations of the products and the products and/or salts of the products are then crystallized and precipitated by cooling or adiabatic crystallization to recover the crystals of the products and/or salts of the products by, for example, centrifugation or filtration; and a method in which an alcohol is added to the culture to produce esters of the products and the resulting esters of the products are subsequently collected by distillation and then hydrolyzed to recover the products. These recovery methods can be appropriately selected and optimized depending on, for example, physical properties of the products.

Examples

The present invention will be specifically described below with reference to examples.

Reference Example 1

Production of plasmids each expressing an enzyme catalyzing a reaction to generate 3-oxoadipyl-CoA and coenzyme A (the reaction A), an enzyme catalyzing a reaction to generate 3-hydroxyadipic acid from 3-hydroxyadipyl-CoA (the reaction E) and a reaction to generate α-hydromuconic acid from 2,3-dehydroadipyl-CoA (the reaction F), and a polypeptide represented by SEQ ID NO: 1, 2, 3, 4, 5, 6, or 7

The pBBR1MCS-2 vector, which is capable of autonomous replication in *E. coli* (ME Kovach, (1995), Gene 166: 175-176), was cleaved with *Xho*I to obtain pBBR1MCS-2/XhoI. To integrate a constitutive expression promoter into the vector, primers (SEQ ID NOs: 187 and 188) were designed to amplify an upstream 200-b region (SEQ ID NO: 186) of *gapA* (NCBI Gene ID: NC_000913.3) by PCR using the genomic DNA of *Escherichia coli* K-12 MG1655 as a template, and a PCR reaction was performed in accordance with routine procedures. The obtained fragment and pBBR1MCS-2/XhoI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant *E. coli* strain was confirmed in accordance with routine procedures, and the plasmid was designated as pBBR1MCS-2::Pgap. Then, the pBBR1MCS-2::Pgap was cleaved with *Sca*I to obtain pBBR1MCS-2::Pgap/ScaI. For amplification of a gene encoding an enzyme catalyzing the reaction A, primers (SEQ ID NOs: 190 and 191) were designed to amplify the full length of the acyl transferase gene *pcaF* (NCBI Gene ID: 1041755, SEQ ID NO: 189) by PCR using the genomic DNA of *Pseudomonas putida* strain KT2440 as a template, and a PCR reaction was performed in accordance with routine procedures. The obtained fragment and the pBBR1MCS-2::Pgap/ScaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::AT. Then, the pBBR1MCS-2::AT was cleaved with *Hpa*I to obtain pBBR1MCS-2::AT/HpaI. For amplification of a gene encoding an enzyme catalyzing the reactions D and E, primers (SEQ ID NOs: 194 and 195) were designed to amplify a continuous sequence including the full lengths of genes together encoding a CoA transferase, *pcaI* and *pcaJ* (NCBI Gene IDs: 1046613 and 1046612, SEQ ID NOs: 192 and 193) by PCR using the genomic DNA of *Pseudomonas putida* strain KT2440 as a template, and a PCR reaction was performed in accordance with routine procedures. The obtained fragment and the pBBR1MCS-2::AT/HpaI were ligated together using the In-Fusion HD Cloning Kit, and the resulting plasmid was introduced into *E. coli* strain DH5α. The nucleotide sequence on the plasmid isolated from the obtained recombinant strain was confirmed in accordance with routine procedures, and the plasmid was designated as pBBR1MCS-2::ATCT.

The pBBR1MCS-2::ATCT was cleaved with *Sca*I to obtain pBBR1MCS-2::ATCT/ScaI. For amplification of a nucleic acid encoding a polypeptide represented by SEQ ID NO: 1, primers (SEQ ID NOs: 196 and 197) were designed

to amplify the nucleic acid represented by SEQ ID NO: 87 through PCR using the genomic DNA of *Serratia marcescens* strain ATCC13880 as a template, and a PCR reaction was performed in accordance with routine procedures. For amplification of a nucleic acid encoding a polypeptide represented by SEQ ID NO: 2, primers (SEQ ID NOs: 198 and 199) were designed to amplify the nucleic acid represented by SEQ ID NO: 88 through PCR using the genomic DNA of *Serratia nematodiphila* strain DSM21420 as a template, and a PCR reaction was performed in accordance with routine procedures. For amplification of a nucleic acid encoding a polypeptide represented by SEQ ID NO: 3, primers (SEQ ID NOs: 200 and 201) were designed to amplify the nucleic acid represented by SEQ ID NO: 89 through PCR using the genomic DNA of *Serratia plymuthica* strain NBRC102599 as a template, and a PCR reaction was performed in accordance with routine procedures. For amplification of a nucleic acid encoding a polypeptide represented by SEQ ID NO: 4, primers (SEQ ID NOs: 202 and 203) were designed to amplify the nucleic acid represented by SEQ ID NO: 90 through PCR using the genomic DNA of *Serratia proteamaculans* strain 568 as a template, and a PCR reaction was performed in accordance with routine procedures. For amplification of a nucleic acid encoding a polypeptide represented by SEQ ID NO: 5, primers (SEQ ID NOs: 204 and 205) were designed to amplify the nucleic acid represented by SEQ ID NO: 91 through PCR using the genomic DNA of *Serratia ureilytica* strain Lr5/4 as a template, and a PCR reaction was performed in accordance with routine procedures. For amplification of a nucleic acid encoding a polypeptide represented by SEQ ID NO: 6, primers (SEQ ID NOs: 206 and 207) were designed to amplify the nucleic acid represented by SEQ ID NO: 92 through PCR using the genomic DNA of *Serratia* sp. strain BW106 as a template, and a PCR reaction was performed in accordance with routine procedures. For amplification of a nucleic acid encoding a polypeptide represented by SEQ ID NO: 7, primers (SEQ ID NOs: 208 and 209) were designed to amplify the nucleic acid represented by SEQ ID NO: 93 through PCR using the genomic DNA of *Serratia liquefaciens* strain FK01 as a template, and a PCR reaction was performed in accordance with routine procedures. Each of the obtained fragments and the pBBR1MCS-2::ATCT/ScaI were ligated together using the In-Fusion HD Cloning Kit (manufactured by Takara Bio Inc.), and each of the resulting plasmids was introduced into *E. coli* strain DH5$\alpha$. The nucleotide sequence on the plasmid isolated from each of the obtained recombinant strains was confirmed in accordance with routine procedures.

The plasmid for expression of the polypeptide represented by SEQ ID NO: 1 was designated as "pBBR1MCS-2::ATCTOR1"; the plasmid for expression of the polypeptide represented by SEQ ID NO: 2 was designated as "pBBR1MCS-2::ATCTOR2"; the plasmid for expression of the polypeptide represented by SEQ ID NO: 3 was designated as "pBBR1MCS-2::ATCTOR3"; the plasmid for expression of the polypeptide represented by SEQ ID NO: 4 was designated as "pBBR1MCS-2::ATCTOR4"; the plasmid for expression of the polypeptide represented by SEQ ID NO: 5 was designated as "pBBR1MCS-2::ATCTOR5"; the plasmid for expression of the polypeptide represented by SEQ ID NO: 6 was designated as "pBBR1MCS-2::ATCTOR6"; and the plasmid for expression of the polypeptide represented by SEQ ID NO: 7 was designated as "pBBR1MCS-2::ATCTOR7"; and these plasmids are listed in Table 4.

[Table 4]

| Plasmid | Originating organism | Gene ID | SEQ ID NO: |
|---|---|---|---|
| pBBR1MCS-2::ATCTOR1 | *Serratia marcescens* ATCC 13880 | JMPQ01000047.1 | 87 |
| pBBR1MCS-2::ATCTOR2 | *Serratia nematodiphila* DSM21420 | JPUX00000000.1 | 88 |
| pBBR1MCS-2::ATCTOR3 | *Serratia plymuthica* NBRC102599 | BCTU01000013.1 | 89 |
| pBBR1MCS-2::ATCTOR4 | *Serratia proteamaculans* 568 | CP000826.1 | 90 |
| pBBR1MCS-2::ATCTOR5 | *Serratia ureilytica* Lr5/4 | JSFB01000001 | 91 |
| pBBR1MCS-2::ATCTOR6 | *Serratia* sp. BW106 | MCGS01000002.1 | 92 |
| pBBR1MCS-2::ATCTOR7 | *Serratia liquefaciens* FK01 | CP006252.1 | 93 |

Reference Example 2

Production of a plasmid for expression of an enzyme catalyzing a reaction to generate 2,3-dehydroadipyl-CoA from 3-hydroxyadipyl-CoA (the reaction C)

The pMW119 expression vector (manufactured by Nippon Gene Co., Ltd.), which is capable of autonomous replication in *E. coli*, was cleaved with *Sac*I to obtain pMW119/SacI. To integrate a constitutive expression promoter into the vector, primers (SEQ ID NOs: 210 and 211) were designed to amplify the upstream 200-b region (SEQ ID NO: 186) of *gapA* (NCBI Gene ID: NC_000913.3) by PCR using the genomic DNA of *Escherichia coli* K-12 MG1655 as a template, and a PCR reaction was performed in accordance with routine procedures. The obtained fragment and the pMW119/SacI were ligated together using the In-Fusion HD Cloning Kit (manufactured by Takara Bio Inc.), and the resulting plasmid

was introduced into *E. coli* strain DH5α. The nucleotide sequence on the plasmid isolated from the obtained recombinant *E. coli* strain was confirmed in accordance with routine procedures, and the plasmid was designated as pMW119::Pgap. Then, the pMW119::Pgap was cleaved with *Sph*I to obtain pMW119::Pgap/SphI. For amplification of a gene encoding an enzyme catalyzing the reaction C, primers (SEQ ID NOs: 212 and 213) were designed to amplify the full length of the enoyl-CoA hydratase gene *paaF* (NCBI Gene ID: 1046932, SEQ ID NO: 176) by PCR using the genomic DNA of *Pseudomonas putida* strain KT2440 as a template, and a PCR reaction was performed in accordance with routine procedures. The obtained fragment and the pMW119::Ygap/SphI were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures. The obtained plasmid was designated as "pMW119::EH".

Reference Example 3

Production of plasmids each expressing an enzyme catalyzing a reaction to generate 3-oxoadipyl-CoA and coenzyme A from acetyl-CoA and succinyl-CoA (the reaction A), an enzyme catalyzing a reaction to generate adipic acid from adipyl-CoA (the reaction G), and a polypeptide represented by SEQ ID NO: 1, 2, 3, 4, 5, 6, or 7

For amplification of a gene encoding an enzyme catalyzing the reaction G, primers (SEQ ID NOs: 241 and 242) were designed to amplify a continuous sequence including the full lengths of genes together encoding a CoA transferase, *dcaI* and *dcaJ* (NCBI Gene ID: CR543861.1, SEQ ID NOs: 239 and 240) by PCR using the genomic DNA of *Acinetobacter baylyi* strain ADP1 as a template, and a PCR reaction was performed in accordance with routine procedures. The obtained fragment and each of the fragments obtained by cutting the pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, and pBBR1MCS-2::ATCTOR7 with *Hpa*I, which were produced in Reference Example 1, were ligated together using the In-Fusion HD Cloning Kit, and each of the resulting plasmids was introduced into *E. coli* strain DH5α. The nucleotide sequences on the plasmids isolated from the obtained recombinant strains were confirmed in accordance with routine procedures, and the plasmids were designated as pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, and pBBR1MCS-2::ATCT2OR7, respectively.

Reference Example 4

Production of a plasmid for expression of enzymes catalyzing a reaction to generate 2,3-dehydroadipyl-CoA from 3-hydroxyadipyl-CoA (the reaction C) and a reaction to generate adipyl-CoA from 2,3-dehydroadipyl-CoA (the reaction D)

The pMW119::EH was cleaved with *Hind*III to obtain pMW119::EH/HindIII. For amplification of a gene encoding an enzyme catalyzing the reaction D, primers (SEQ ID NOs: 215 and 216) were designed to amplify the full length of *dcaA* (NCBI-Protein ID: AAL09094.1, SEQ ID NO: 214) from *Acinetobacter baylyi* strain ADP1 by PCR, and a PCR reaction was performed in accordance with routine procedures. The obtained fragment and the pMW119::EH/HindIII were ligated together using the In-Fusion HD Cloning Kit (manufactured by Takara Bio Inc.), and the resulting plasmid was introduced into *E. coli* strain DH5α. The nucleotide sequence on the plasmid isolated from the obtained recombinant strain was confirmed in accordance with routine procedures, and the plasmid was designated as pMW119::EHER.

Reference Example 5

Production of plasmids each expressing a PEP carboxykinase, an enzyme catalyzing a reaction to generate 3-oxoadipyl-CoA and coenzyme A (the reaction A), an enzyme catalyzing a reaction to generate 3-hydroxyadipic acid from 3-hydroxyadipyl-CoA (the reaction E) and a reaction to generate α-hydromuconic acid from 2,3-dehydroadipyl-CoA (the reaction F), and a polypeptide represented by SEQ ID NO: 1, 2, 3, 4, 5, 6, or 7

To integrated a promoter for constitutive expression of a PEP carboxykinase, primers (SEQ ID NOs: 217 and 218) were designed to amplify the upstream 200-b region (SEQ ID NO: 186) of *gapA* (NCBI Gene ID: NC 000913.3) by PCR using the genomic DNA of *Escherichia coli* K-12 MG1655 as a template, and a PCR reaction was performed in accordance with routine procedures. The obtained fragment and each of the fragments obtained by cutting the pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, and pBBR1MCS-2::ATCTOR7 with *Sac*I, which were produced in Reference Example 1, were ligated together using the In-Fusion HD Cloning Kit, and each of the resulting plasmids was introduced into *E. coli* strain DH5α. The nucleotide sequences on the plasmids isolated from the obtained recombinant

strains were confirmed in accordance with routine procedures, and the plasmids were designated as pBBR1MCS-2::ATCTOR1Pgap, pBBR1MCS-2::ATCTOR2Pgap, pBBR1MCS-2::ATCTOR3Pgap, pBBR1MCS-2::ATCTOR4Pgap, pBBR1MCS-2::ATCTOR5Pgap, pBBR1MCS-2::ATCTOR6Pgap, and pBBR1MCS-2::ATCTOR7Pgap, respectively. Subsequently, to amplify a gene encoding a PEP carboxykinase, primers (SEQ ID NOs: 220 and 221) were designed to amplify a continuous sequence including the full length of a PEP carboxykinase gene (SEQ ID NO: 219) by PCR using the genomic DNA of *Seratia grimesii* strain NBRC 13537 as a template, and a PCR reaction was performed in accordance with routine procedures. The obtained fragment and each of the fragments obtained by cutting the pBBR1MCS-2::ATCTOR1Pgap, pBBR1MCS-2::ATCTOR2Pgap, pBBR1MCS-2::ATCTOR3Pgap, pBBR1MCS-2::ATCTOR4Pgap, pBBR1MCS-2::ATCTOR5Pgap, pBBR1MCS-2::ATCTOR6Pgap, and pBBR1MCS-2::ATCTOR7Pgap with *Sac*I were ligated together using the In-Fusion HD Cloning Kit, and each of the resulting plasmids was introduced into *E. coli* strain DH5α. The nucleotide sequences on the plasmids isolated from the obtained recombinant strains were confirmed in accordance with routine procedures, and the plasmids were designated as pBBR1MCS-2::ATCTOR1PCK, pBBR1MCS-2::ATCTOR2PCK, pBBR1MCS-2::ATCTOR3PCK, pBBR1MCS-2::ATCTOR4PCK, pBBR1MCS-2::ATCTOR5PCK, pBBR1MCS-2::ATCTOR6YCK, and pBBR1MCS-2::ATCTOR7PCK, respectively.

Reference Example 6

Production of plasmids each expressing a PEP carboxykinase, an enzyme catalyzing a reaction to generate 3-oxoadipyl-CoA and coenzyme A from acetyl-CoA and succinyl-CoA (the reaction A), an enzyme catalyzing a reaction to generate adipic acid from adipyl-CoA (the reaction G), and a polypeptide represented by SEQ ID NO: 1, 2, 3, 4, 5, 6, or 7

By using the same method and primers as in Reference Example 5, the upstream 200-b region of gapA (NCBI Gene ID: NC_000913.3) obtained using the genomic DNA of *Escherichia coli* K-12 MG1655 as a template and the PEP carboxykinase gene from *Serratia grimesii* strain NBRC13537 were inserted into each of the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, and pBBR1MCS-2::ATCT2OR7 produced in Reference Example 3. The obtained plasmids were designated as pBBR1MCS-2::ATCT2OR1PCK, pBBR1MCS-2::ATCT2OR2PCK, pBBR1MCS-2::ATCT2OR3PCK, pBBR1MCS-2::ATCT2OR4PCK, pBBR1MCS-2::ATCT2OR5PCK, pBBR1MCS-2::ATCT2OR6PCK, and pBBR1MCS-2::ATCT2OR7PCK, respectively.

Example 1

Generation of a mutant microorganism of the genus *Serratia* with impaired pyruvate kinase function

Genes encoding the pyruvate kinase of a microorganism of the genus *Serratia*, *pykF* and *pykA*, were disrupted to generate a mutant microorganism of the genus *Serratia* with impaired pyruvate kinase function.

The procedure for disrupting *pykF* and *pykA* followed the method described in Proc Natl Acad Sci USA., 2000 Jun 6, 97(12): 6640-6645.

Generation of a mutant microorganism of the genus *Serratia* deficient in *pykF*

A PCR reaction was performed using pKD4 as a template and oligo DNAs represented by SEQ ID NOs: 222 and 223 as primers to obtain a PCR fragment of 1.6 kb in length for disruption of *pykF*. A FRT recombinase expression plasmid, pKD46, was introduced into *Serratia grimesii* strain NBRC13537, and an ampicillin-resistant strain was obtained. The obtained strain was inoculated into 5 mL of LB medium containing 500 μg/mL ampicillin and was cultured at 30°C with shaking for 1 day. Subsequently, 0.5 mL of the culture fluid was inoculated into 50 mL of LB medium containing 500 μg/mL ampicillin and 50 mM arabinose and was cultured in rotation at 30°C for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 5 μL of the PCR fragment, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 2 hours. The total volume of the culture was applied to LB agar medium containing 25 μg/mL kanamycin and was incubated at 30°C for 1 day. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 226 were used.

Subsequently, one of the kanamycin-resistant strains was inoculated into 5 mL of LB medium and was cultured at 37°C and passaged twice to segregate away the pKD46 and to obtain an ampicillin-sensitive strain. The plasmid

pCP20 was introduced into the ampicillin-sensitive strain, and ampicillin-resistant strains were again obtained. After culturing the obtained strains at 40°C, colony direct PCR was performed on the resulting strains to confirm the deletion of the kanamycin resistance gene from the length of the amplified band. Oligo DNA primers represented by SEQ ID NOs: 225 and 226 were used. Subsequently, one of the kanamycin-sensitive strains was inoculated into 5 mL of LB medium and was cultured at 37°C and passaged twice to segregate away the pCP20. The obtained strain was designated as *Serratia grimesii* NBRC13537 *ΔpykF*.

Generation of a mutant microorganism of the genus *Serratia* deficient in *pykA*

A PCR reaction was performed using pKD4 as a template and oligo DNAs represented by SEQ ID NOs: 227 and 228 as primers to obtain a PCR fragment of 1.6 kb in length for disruption of *pykA*.

By the same method as used for the generation of the *pykF*-deficient strain, *pykA* was disrupted in the *Serratia grimesii* NBRC13537 *ΔpykF* strain. After the plasmid pKD46 was introduced into the above strain, the PCR fragment used for disruption of *pykA* was introduced to the resulting strain. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 230 were used.

Subsequently, an ampicillin-sensitive strain was obtained by segregating away the pKD46. The plasmid pCP20 was introduced into the ampicillin-sensitive strain, and ampicillin-resistant strains were again obtained. Colony direct PCR was performed on the obtained strains to confirm the deletion of the kanamycin resistance gene from the length of the amplified band. Oligo DNA primers represented by SEQ ID NOs: 229 and 230 were used. The pCP20 was segregated away from one of the kanamycin-sensitive strains. The obtained strain was designated as SgΔPP.

Example 2

Generation of mutant microorganisms of the genus *Serratia* with impaired pyruvate kinase function and carrying a plasmid expressing a PEP carboxykinase and enzymes catalyzing the reactions A, B, E, and F

Each of the plasmids produced in Reference Example 1 was introduced into the SgΔPP produced in Example 1 to generate mutant microorganisms of the genus *Serratia*.

The SgΔPP was inoculated into 5 mL of LB medium and cultured at 30°C with shaking for 1 day. Subsequently, 0.5 mL of the culture fluid was inoculated into 5 mL of LB medium and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2::ATCTOR1PCK, pBBR1MCS-2::ATCTOR2PCK, pBBR1MCS-2::ATCTOR3PCK, pBBR1MCS-2::ATCTOR4PCK, pBBR1MCS-2::ATCTOR5PCK, pBBR1MCS-2::ATCTOR6PCK, or pBBR1MCS-2::ATCTOR7PCK, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 25 μg/mL kanamycin and was incubated at 30°C for 1 day. The obtained strains were designated as SgΔPP/3HA1PCK, SgΔPP/3HA2PCK, SgΔPP/3HA3PCK, SgΔPP/3HA4PCK, SgΔPP/3HA5PCK, SgΔPP/3HA6PCK, and SgΔPP/3HA7PCK, respectively.

Reference Example 7

Generation of mutant microorganisms of the genus *Serratia* with intact pyruvate kinase function and carrying a plasmid expressing a PEP carboxykinase and enzymes catalyzing the reactions A, B, E, and F

By the same method as in Example 2, the pBBR1MCS-2::ATCTOR1PCK, pBBR1MCS-2::ATCTOR2PCK, pBBR1MCS-2::ATCTOR3PCK, pBBR1MCS-2::ATCTOR4PCK, pBBR1MCS-2::ATCTOR5PCK, pBBR1MCS-2::ATCTOR6PCK, or pBBR1MCS-2::ATCTOR7PCK was introduced into *Serratia grimesii* NBRC13537. Additionally, a control strain was generated by introducing the pBBR1MCS-2 empty vector into *Serratia grimesii* NBRC13537. The obtained strains were designated as Sg/3HA1PCK, Sg/3HA2PCK, Sg/3HA3PCK, Sg/3HA4PCK. Sg/3HA5PCK, Sg/3HA6PCK, Sg/3HA7PCK, and Sg/pBBR (negative control) , respectively.

Reference Example 8

Generation of mutant microorganisms of the genus *Serratia* with impaired pyruvate kinase function and carrying a plasmid expressing enzymes that catalyze the reactions A, B, E, and F

By the same method as in Example 2, the pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into the SgΔPP. Additionally, a control strain was generated by introducing the pBBR1MCS-2 empty vector into the SgΔPP. The obtained strains were designated as SgΔPP/3HA1, SgΔPP/3HA2, SgΔPP/3HA3, SgΔPP/3HA4, SgΔPP/3HA5, SgΔPP/3HA6, SgΔPP/3HA7, and SgΔPP/pBBR (negative control), respectively.

Example 3

Production test of 3-hydroxyadipic acid and α-hydromuconic acid using mutant microorganisms of the genus *Serratia* with impaired pyruvate kinase function and with enhanced PEP carboxykinase activity

The production test of 3-hydroxyadipic acid and α-hydromuconic acid was conducted using the mutant microorganisms of the genus *Serratia* produced in Example 2.

A loopful of each mutant produced in Example 2 was inoculated into 5 mL (in a glass test tube of 18-mm diameter with aluminum cap) of the culture medium I (10 g/L Bacto Tryptone (manufactured by Difco Laboratories), 5 g/L Bacto Yeast Extract (manufactured by Difco Laboratories), 5 g/L sodium chloride, 25 μg/mL kanamycin) adjusted to pH 7 and was cultured at 30°C with shaking at 120 $min^{-1}$ for 24 hours. Subsequently, 0.25 mL of the culture fluid was added to 5 mL (in a glass test tube of 18-mm diameter with aluminum cap) of the culture medium II (50g/L glucose, 1 g/L ammonium sulfate, 50 mM potassium phosphate, 0.025 g/L magnesium sulfate, 0.0625 mg/L iron sulfate, 2.7 mg/L manganese sulfate, 0.33 mg/L calcium chloride, 1.25 g/L sodium chloride, 2.5 g/L Bacto Tryptone, 1.25 g/L Bacto Yeast Extract, 25 μg/mL kanamycin) adjusted to pH 6.5 and was cultured at 30°C with shaking at 120 $min^{-1}$ for 24 hours.

Quantitative analysis of substrate and product

The supernatant separated from bacterial cells by centrifugation of each culture fluid was processed by membrane treatment using Millex-GV (0.22 μm; PVDF; manufactured by Merck KGaA), and the resulting filtrate was analyzed by the following methods to quantify the concentrations of 3-hydroxyadipic acid, α-hydromuconic acid, and other products accumulated in the culture supernatant and the concentration of sugars remaining unused in the culture medium. The yields of 3-hydroxyadipic acid and α-hydromuconic acid calculated using the formula (2) below from the measurement results are shown in Table 5.

A concentration of not more than 0.1 mg/L is considered to be below the detection limit in the quantitative LC-MS/MS analysis and is hereinafter denoted in each table as N.D.

$$\text{Yield (\%)} = \text{amount of a generated product (mol)} / \text{amount of consumed sugars (mol)} \times 100 \qquad \text{Formula (2)}$$

Quantitative analysis of 3-hydroxyadipic acid and α-hydromuconic acid by LC-MS/MS

• HPLC: 1290 Infinity (manufactured by Agilent Technologies, Inc.)
Column: Synergi hydro-RP (manufactured by Phenomenex Inc.), length: 100 mm, internal diameter: 3 mm, particle size: 2.5 μm
Mobile phase: 0.1% aqueous formic acid solution / methanol = 70/30
Flow rate: 0.3 mL/min
Column temperature: 40°C
LC detector: 1260DAD VL+ (210 nm)
• MS/MS: Triple-Quad LC/MS (manufactured by Agilent Technologies, Inc.)
Ionization method: ESI in negative mode.

Quantitative analysis of organic acids by HPLC

- HPLC:LC-10A (manufactured by Shimadzu Corporation)
  Column: Shim-pack SPR-H (manufactured by Shimadzu GLC Ltd.), length: 250 mm, internal diameter: 7.8 mm, particle size: 8 μm
  Shim-pack SCR-101H (manufactured by Shimadzu GLC Ltd.) length: 250 mm, internal diameter: 7.8 mm, particle size: 10 μm
  Mobile phase: 5 mM *p*-toluenesulfonic acid
  Reaction solution: 5 mM *p*-toluenesulfonic acid, 0.1 mM EDTA, 20 mM Bis-Tris
  Flow rate: 0.8 mL/min
  Column temperature: 45°C
  Detector: CDD-10Avp (manufactured by Shimadzu Corporation)

Quantitative analysis of sugars and alcohol by HPLC

- HPLC: Shimazu Prominence (manufactured by Shimadzu Corporation)
  Column: Shodex Sugar SH1011 (manufactured by Showa Denko K.K.), length: 300 mm, internal diameter: 8 mm, particle size: 6 μm
  Mobile phase: 0.05M aqueous sulfuric acid solution
  Flow rate: 0.6 mL/min
  Column temperature: 65°C
  Detector: RID-10A (manufactured by Shimadzu Corporation).

Comparative Example 1

Production test of 3-hydroxyadipic acid and α-hydromuconic acid using mutant microorganisms of the genus *Serratia* with intact pyruvate kinase function and with enhanced PEP carboxykinase activity

The mutants produced in Reference Example 7 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the formula (2) from the measurement results are shown in Table 5.

Comparative Example 2

Production test of 3-hydroxyadipic acid and α-hydromuconic acid using mutant microorganisms of the genus *Serratia* with impaired pyruvate kinase function and with unenhanced PEP carboxykinase activity

The mutants produced in Reference Example 8 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the formula (2) from the measurement results are shown in Table 5.

By comparing the results of Comparative Examples 1 and 2 with that of Example 3, it was found that the yields of 3-hydroxyadipic acid and α-hydromuconic acid were increased by impairing the function of pyruvate kinase and enhancing the PEP carboxykinase activity in the microorganism of the genus *Serratia.*

[Table 5]

| | Strain | Yield of 3HA (%) | Yield of HMA (%) |
|---|---|---|---|
| Example 3 | SgΔPP/3HA1PCK | 6.84 | 0.103 |
| | SgΔPP/3HA2 PCK | 7.91 | 0.132 |
| | SgΔPP/3HA3 PCK | 7.43 | 0.148 |
| | SgΔPP/3HA4 PCK | 7.12 | 0.103 |
| | SgΔPP/3HA5 PCK | 7.03 | 0.127 |
| | SgΔPP/3HA6 PCK | 8.13 | 0.149 |
| | SgΔPP/3HA7 PCK | 6.70 | 0.127 |
| Comparative Example 1 | Sg/pBBR | N.D. | N.D. |
| | Sg/3HA1 PCK | 0.568 | 0.0206 |
| | Sg/3HA2 PCK | 0.857 | 0.0336 |
| | Sg/3HA3 PCK | 0.684 | 0.0330 |
| | Sg/3HA4 PCK | 0.635 | 0.0298 |
| | Sg/3HA5 PCK | 0.733 | 0.0290 |
| | Sg/3HA6 PCK | 0.750 | 0.0260 |
| | Sg/3HA7 PCK | 0.685 | 0.0165 |
| Comparative Example 2 | SgΔPP/pBBR | 0.0362 | 0.0113 |
| | SgAPP/3HA1 | 3.47 | 0.0782 |
| | SgΔPP/3HA2 | 5.78 | 0.0960 |
| | SgΔPP/3HA3 | 5.24 | 0.0846 |
| | SgΔPP/3HA4 | 5.10 | 0.0909 |
| | SgΔPP/3HA5 | 6.21 | 0.107 |
| | SgΔPP/3HA6 | 6.28 | 0.103 |
| | SgΔPP/3HA7 | 4.96 | 0.0638 |

Example 4

Generation of an *E. coli* mutant with impaired pyruvate kinase function

Genes encoding the pyruvate kinase of *E. coli*, *pykF* and *pykA*, were disrupted to generate an *E. coli* mutant with impaired pyruvate kinase function. The procedure for disrupting *pykF* and *pykA* followed the method described in Proc Natl Acad Sci U S A., 2000 Jun 6, 97(12): 6640-6645.

Generation of an *E. coli* mutant deficient in *pykF*

A PCR reaction was performed using pKD4 as a template and oligo DNAs represented by SEQ ID NOs: 231 and 232 as primers to obtain a PCR fragment of 1.6 kb in length for disruption of *pykF.*

A FRT recombinase expression plasmid, pKD46, was introduced into *Escherichia coli* strain MG1655, and an ampicillin-resistant strain was obtained. The obtained strain was inoculated into 5 mL of LB medium containing 100 μg/mL ampicillin and cultured at 30°C with shaking for 1 day. Subsequently, 0.5 mL of the culture fluid was inoculated into 50 mL of LB medium containing 100 μg/mL ampicillin and 50 mM arabinose and was cultured in rotation at 30°C for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 5 μL of the PCR fragment, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μE), and 1 mL of SOC medium was added to the electroporation cuvette immediately after the electroporation, and the bacterial

cells in the cuvette were incubated at 30°C with shaking for 2 hours. The total volume of the culture was applied to LB agar medium containing 25 μg/mL kanamycin and was incubated at 30°C for 1 day. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 234 were used.

Subsequently, one of the kanamycin-resistant strains was inoculated into 5 mL of LB medium and was cultured at 37°C and passaged twice to segregate away the pKD46 and to obtain an ampicillin-sensitive strain. The plasmid pCP20 was introduced into the ampicillin-sensitive strain, and ampicillin-resistant strains were again obtained. After culturing the obtained strains at 40°C, colony direct PCR was performed on the resulting strains to confirm the deletion of the kanamycin resistance gene from the length of the amplified band. Oligo DNA primers represented by SEQ ID NOs: 233 and 234 were used. Subsequently, one of the kanamycin-sensitive strains was inoculated into 5 mL of LB medium and was cultured at 37°C and passaged twice to segregate away the pCP20. The obtained strain was designated as *Escherichia coli* MG1655 *ΔpykF.*

Generation of an *E. coli* mutant deficient in *pykA*

A PCR reaction was performed using pKD4 as a template and oligo DNAs represented by SEQ ID NOs: 235 and 236 as primers to obtain a PCR fragment of 1.6 kb in length for disruption of *pykA.*

By the same method as used for the generation of the *pykF*-deficient strain, *pykA* was disrupted in the *Escherichia coli* MG1655 *ΔpykF* strain. After the plasmid pKD46 was introduced into the above strain, the PCR fragment used for disruption of *pykA* was introduced to the resulting strain. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 238 were used.

Subsequently, an ampicillin-sensitive strain was obtained by segregating away the pKD46. The plasmid pCP20 was introduced into the ampicillin-sensitive strain, and ampicillin-resistant strains were again obtained. Colony direct PCR was performed on the obtained strains to confirm the deletion of the kanamycin resistance gene from the length of the amplified band. Oligo DNA primers represented by SEQ ID NOs: 237 and 238 were used. The pCP20 was segregated away from one of the kanamycin-sensitive strains. The obtained strain was designated as EcΔPP.

Example 5

Generation of *E. coli* mutants with impaired pyruvate kinase function and carrying a plasmid expressing a PEP carboxykinase and enzymes catalyzing the reactions A, B, E, and F

Each of the plasmids produced in Reference Example 1 was introduced into the EcΔPP produced in Example 4 to generate *E. coli* mutants.

The EcΔPP was inoculated into 5 mL of LB medium and cultured at 30°C with shaking for 1 day. Subsequently, 0.5 mL of the culture fluid was inoculated into 5 mL of LB medium and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pBBR1MCS-2::ATCTOR1PCK, pBBR1MCS-2::ATCTOR2PCK, pBBR1MCS-2::ATCTOR3PCK, pBBR1MCS-2::ATCTOR4PCK, pBBR1MCS-2::ATCTOR5PCK, pBBR1MCS-2::ATCTOR6PCK, or pBBR1MCS-2::ATCTOR7PCK, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 25 μg/mL kanamycin and was incubated at 30°C for 1 day. The obtained strains were designated as EcΔPP/3)HA1PCK, EcΔPP/3HA2PCK, EcΔPP/3HA3PCK, EcΔPP/3HA4PCK, EcΔPP/3HA5PCK, EcΔPP/3HA6PCK, and EcΔPP/3HA7PCK, respectively.

Reference Example 9

Generation of *E. coli* mutants with intact pyruvate kinase function and carrying a plasmid expressing a PEP carboxykinase and enzymes catalyzing the reactions A, B, E, and F

By the same method as in Example 5, the pBBR1MCS-2::ATCTOR1PCK, pBBR1MCS-2::ATCTOR2PCK, pBBR1MCS-2::ATCTOR3PCK, pBBR1MCS-2::ATCTOR4PCK, pBBR1MCS-2::ATCTOR5PCK, pBBR1MCS-2::ATCTOR6PCK, or pBBR1MCS-2::ATCTOR7PCK was introduced into *Escherichia coli* MG1655. Additionally, a control strain was generated by introducing the pBBR1MCS-2 empty vector into *Escherichia coli* MG1655. The obtained strains

were designated as Ec/3HA1PCK, Ec/3HA2PCK, Ec/3HA3PCK, Ec/3HA4PCK, Ec/3HA5PCK, Ec/3HA6PCK, Ec/3HA7PCK, and Ec/pBBR (negative control), respectively.

Reference Example 10

Generation of *E. coli* mutants with impaired pyruvate kinase function and carrying a plasmid expressing enzymes that catalyze the reactions A, B, E, and F

By the same method as in Example 5, the pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into EcΔPP. Additionally, a control strain was generated by introducing the pBBR1MCS-2 empty vector into the EcΔPP. The obtained strains were designated as EcΔPP/3HA1, EcΔPP/3HA2, EcΔPP/3HA3, EcΔPP/3HA4, EcΔPP/3HA5, EcΔPP/3HA6, EcΔPP/3HA7, and EcΔPP/pBBR (negative control), respectively.

Example 6

Production test of 3-hydroxyadipic acid and α-hydromuconic acid using *E. coli* mutants with impaired pyruvate kinase function and with enhanced PEP carboxykinase activity

The mutants produced in Example 5 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 6.

Comparative Example 3

Production test of 3-hydroxyadipic acid and α-hydromuconic acid using *E. coli* mutants with intact pyruvate kinase function and with enhanced PEP carboxykinase activity

The mutants produced in Reference Example 9 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 6.

Comparative Example 4

Production test of 3-hydroxyadipic acid and α-hydromuconic acid using *E. coli* mutants with impaired pyruvate kinase function and with unenhanced PEP carboxykinase activity

The mutants produced in Reference Example 10 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the formula (2) from the measurement results are shown in Table 6.

By comparing the results of Comparative Examples 3 and 4 with that of Example 6, it was found that the yields of 3-hydroxyadipic acid and α-hydromuconic acid were increased by impairing the function of pyruvate kinase and enhancing the PEP carboxykinase activity in *E. coli.*

[Table 6]

| | Strain | Yield of 3HA (%) | Yield of HMA (%) |
|---|---|---|---|
| Example 5 | EcΔPP/3HA1PCK | 4.76 | 0.0370 |
| | EcΔPP/3HA2 PCK | 5.31 | 0.0380 |
| | EcΔPP/3HA3 PCK | 4.35 | 0.0323 |
| | EcΔPP/3HA4 PCK | 4.60 | 0.0375 |
| | EcΔPP/3HA5 PCK | 5.14 | 0.0389 |
| | EcΔPP/3HA6 PCK | 5.54 | 0.0389 |
| | EcΔPP/3HA7 PCK | 4.65 | 0.0362 |
| Comparative Example 3 | Ec/pBBR | N.D. | N.D. |
| | Ec/311A1 PCK | 1.08 | 0.0124 |
| | Ec/3HA2 PCK | 1.91 | 0.0116 |
| | Ec/3HA3 PCK | 2.15 | 0.0124 |
| | Ec/3HA4 PCK | 1.44 | 0.0147 |
| | Ec/3HA5 PCK | 1.72 | 0.0109 |
| | Ec/3HA6 PCK | 1.86 | 0.0148 |
| | Ec/3HA7 PCK | 1.34 | 0.0117 |
| Comparative Example 4 | EcΔPP/pBBR | 0.0427 | 0.0132 |
| | EcΔPP/3HA1 | 2.54 | 0.0292 |
| | EcΔPP /3 HA2 | 3.97 | 0.0333 |
| | EcΔPP/3HA3 | 3.64 | 0.0273 |
| | EcΔPP/3HA4 | 2.86 | 0.0257 |
| | EcΔPP/3HA5 | 3.67 | 0.0269 |
| | EcΔPP/3HA6 | 3.57 | 0.0348 |
| | EcΔPP/3HA7 | 3.13 | 0.0274 |

Example 7

Generation of mutant microorganisms of the genus *Serratia* with impaired pyruvate kinase function and carrying plasmids expressing a PEP carboxykinase and enzymes catalyzing the reactions A, B, C, E, and F

The plasmid pMW119::EH produced in Reference Example 2 was introduced into each mutant microorganism of the genus *Serratia* produced in Example 2 to generate mutant microorganisms of the genus *Serratia*.

The SgΔPP/3HA1PCK, SgΔPP/3HA2PCK, SgΔPP/3HA3PCK, SgΔPP/3HA4PCK, SgΔPP/3HA5PCK, SgΔPP/3HA6PCK, or SgΔPP/3HA7PCK was inoculated into 5 mL of LB medium containing 25 μg/mL kanamycin and cultured at 30°C with shaking for 1 day. Subsequently, 0.5 mL of the culture fluid was inoculated into 5 mL of LB medium containing 25 μg/mL kanamycin and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 μL of the pMW119::EH, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 500 μg/mL ampicillin and 25 μg/mL kanamycin and was incubated at 30°C for 1 day. The obtained strains were designated as SgΔPP/HMA1PCK, SgΔPP/HMA2PCK, SgΔPP/HMA3PCK, SgΔPP/HMA4PCK, SgΔPP/HMA5PCK, SgΔPP/HMA6PCK, and SgΔPP/HMA7PCK, respectively.

Reference Example 11

Generation of mutant microorganisms of the genus *Serratia* with intact pyruvate kinase function and carrying plasmids expressing a PEP carboxykinase and enzymes catalyzing the reactions A, B, C, E, and F

By the same method as in Example 7, the pMW119::EH was introduced into the Sg/3HA1PCK, Sg/3HA2PCK, Sg/3HA3PCK, Sg/3HA4PCK, Sg/3HA5PCK, Sg/3HA6PCK, or Sg/3HA7PCK. Additionally, a control strain was generated by introducing the pMW119 empty vector into the Sg/pBBR. The obtained strains were designated as Sg/HMA1PCK, Sg/HMA2PCK, Sg/HMA3PCK, Sg/HMA4PCK, Sg/HMA5PCK, Sg/HMA6PCK, Sg/HMA7PCK, and Sg/pBBRpMW (negative control), respectively.

Reference Example 12

Generation of mutant microorganisms of the genus *Serratia* with impaired pyruvate kinase function and carrying plasmids expressing enzymes that catalyze the reactions A, B, C, E, and F

By the same method as in Example 7, the pMW119::EH was introduced into the SgΔPP/3HA1, SgΔPP/3HA2, SgΔPP/3HA3. SgΔPP/3HA4, SgΔPP/3HA5, SgΔPP/3HA6, or SgΔPP/3HA7. Additionally, a control strain was generated by introducing the pMW119 empty vector into the SgΔPP/pBBR. The obtained strains were designated as SgΔPP/HMA1, SgΔPP/HMA2, SgΔPP/HMA3, SgΔPP/HMA4, SgΔPP/HMA5, SgΔPP/HMA6, SgΔPP/HMA7, and SgΔPP/pBBRpMW (negative control), respectively.

Example 8

Production test of α-hydromuconic acid using mutant microorganisms of the genus *Serratia* with impaired pyruvate kinase function and with enhanced PEP carboxykinase activity

The mutants produced in Example 7 were cultured in the same manner as in Example 3, except that ampicillin was added to the culture medium to a final concentration of 500 μg/mL. The concentrations of α-hydromuconic acid and other products accumulated in the culture supernatant and the concentration of sugars remaining unused in the culture medium were quantified. The yield of a-hydromuconic acid calculated using the formula (2) from the measured values is shown in Table 7.

Comparative Example 5

Production test of α-hydromuconic acid using mutant microorganisms of the genus *Serratia* with intact pyruvate kinase function and with enhanced PEP carboxykinase activity

The mutants produced in Reference Example 11 were cultured in the same manner as in Example 8. The concentrations of α-hydromuconic acid and other products accumulated in the culture supernatant and the concentration of sugars remaining unused in the culture medium were quantified. The yield of a-hydromuconic acid calculated using the formula (2) from the measured values is shown in Table 7.

Comparative Example 6

Production test of α-hydromuconic acid using mutant microorganisms of the genus *Serratia* with impaired pyruvate kinase function and with unenhanced PEP carboxykinase activity

The mutants produced in Reference Example 12 were cultured in the same manner as in Example 8. The concentrations of α-hydromuconic acid and other products accumulated in the culture supernatant and the concentration of sugars remaining unused in the culture medium were quantified. The yield of α-hydromuconic acid calculated using the formula (2) from the measurement results is shown in Table 7.

By comparing the results of Comparative Examples 5 and 6 with that of Example 8, it was found that the yield of α-hydromuconic acid was increased by disrupting the pyruvate kinase gene and enhancing the PEP carboxykinase activity in the microorganism of the genus *Serratia.*

[Table 7]

| | Strain | Yield of HMA (%) |
|---|---|---|
| Example 8 | SgΔPP/HMA1PCK | 0.182 |
| | SgΔPP/HMA2 PCK | 0.205 |
| | SgΔPP/HMA3 PCK | 0.218 |
| | SgΔPP/HMA4 PCK | 0.221 |
| | SgΔPP/HMA5 PCK | 0.246 |
| | SgΔPP/HMA6 PCK | 0.294 |
| | SgΔPP/HMA7 PCK | 0.216 |
| Comparative Example 5 | Sg/pBBRpMW | N.D. |
| | Sg/HMA1 PCK | 0.0355 |
| | Sg/HMA2 PCK | 0.0420 |
| | Sg/HMA3 PCK | 0.0310 |
| | Sg/HMA4 PCK | 0.0376 |
| | Sg/HMA5 PCK | 0.0423 |
| | Sg/HMA6 PCK | 0.0445 |
| | Sg/HMA7 PCK | 0.0372 |
| Comparative Example 6 | SgΔPP/pBBRpMW | 0.0119 |
| | SgΔPP/HMA1 | 0.156 |
| | SgΔPP/HMA2 | 0.179 |
| | SgΔPP/HMA3 | 0.153 |
| | SgΔPP/HMA4 | 0.118 |
| | SgΔPP/HMA5 | 0.217 |
| | SgΔPP/HMA6 | 0.241 |
| | SgΔPP/HMA7 | 0.140 |

Example 9

Generation of *E. coli* mutants with impaired pyruvate kinase function and carrying plasmids expressing a PEP carboxykinase and enzymes catalyzing the reactions A, B, C, E, and F

The plasmid pMW119::EH produced in Reference Example 2 was introduced into each of the *E. coli* mutants produced in Example 5 to generate *E. coli* mutants.

The EcΔPP/3HA1PCK, EcΔPP/3HA2PCK, EcΔPP/3HA3PCK, EcΔPP/3HA4PCK, EcΔPP/3HA5PCK, EcΔPP/3HA6PCK, or EcΔPP/3HA7PCK was inoculated into 5 mL of LB medium containing 25 μg/mL kanamycin and cultured at 30°C with shaking for 1 day. Subsequently, 0.5 mL of the culture fluid was inoculated into 5 mL of LB medium containing 25 μg/mL kanamycin and was cultured at 30°C with shaking for 2 hours. The culture fluid was cooled on ice for 20 minutes, and the bacterial cells were then washed with 10% (w/w) glycerol three times. The washed pellet was suspended in 100 μL of 10% (w/w) glycerol and mixed with 1 uL of the pMW119::EH, and the mixture was then cooled in an electroporation cuvette on ice for 10 minutes. Electroporation was performed using a Gene Pulser electroporator (manufactured by Bio-Rad Laboratories, Inc.; 3 kV, 200 Ω, 25 μF), and 1 mL of SOC medium was added to the electroporation cuvette immediately after the electroporation, and the bacterial cells in the cuvette were incubated at 30°C with shaking for 1 hour. Fifty μL of the culture was applied to LB agar medium containing 100 μg/mL ampicillin and 25 μg/mL kanamycin and was incubated at 30°C for 1 day. The obtained strains were designated as EcΔPP/HMA1PCK, EcΔPP/HMA2PCK, EcΔPP/HMA3PCK, EcΔPP/HMA4PCK, EcΔPP/HMA5PCK, EcΔPP/HMA6PCK, and EcΔPP/HMA7PCK, respectively.

Reference Example 13

Generation of *E. coli* mutants with intact pyruvate kinase function and carrying plasmids expressing a PEP carboxykinase and enzymes catalyzing the reactions A, B, C, E, and F

By the same method as in Example 9, the pMW119::EH was introduced into the Ec/3HA1PCK, Ec/3HA2PCK, Ec/3HA3PCK, Ec/3HA4PCK, Ec/3HA5PCK, Ec/3HA6PCK, or Ec/3HA7PCK. Additionally, a control strain was generated by introducing the pMW119 empty vector into the Ec/pBBR. The obtained strains were designated as Ec/HMA1PCK, Ec/HMA2PCK, Ec/HMA3PCK, Ec/HMA4PCK, Ec/HMA5PCK, Ec/HMA6PCK, Ec/HMA7PCK, and Ec/pBBRpMW (negative control), respectively.

Reference Example 14

Generation of *E. coli* mutants with impaired pyruvate kinase function and carrying plasmids expressing enzymes that catalyze the reactions A, B, C, E, and F

By the same method as in Example 9, the pMW119::EH was introduced into the EcΔPP/3HA1, EcΔPP/3HA2, EcΔPP/3HA3, EcΔPP/3HA4, EcΔPP/3HA5, EcΔPP/3HA6, or EcΔPP/3HA7. Additionally, a control strain was generated by introducing the pMW119 empty vector into the EcΔPP/pBBR. The obtained strains were designated as EcΔPP/HMA1, EcΔPP/HMA2, EcΔPP/HMA3, EcΔPP/HMA4, EcΔPP/HMA5, EcΔPP/HMA6, EcΔPP/HMA7, and EcΔPP/pBBRpMW (negative control), respectively.

Example 10

Production test of α-hydromuconic acid using *E. coli* mutants with impaired pyruvate kinase function and with enhanced PEP carboxykinase activity

The mutants produced in Reference Example 9 were cultured in the same manner as in Example 6, except that ampicillin was added to the culture medium to a final concentration of 100 μg/mL. The concentrations of α-hydromuconic acid and other products accumulated in the culture supernatant and the concentration of sugars remaining unused in the culture medium were quantified. The yield of α-hydromuconic acid calculated using the formula (2) from the measured values is shown in Table 8.

Comparative Example 7

Production test of α-hydromuconic acid using *E. coli* mutants with intact pyruvate kinase function and with enhanced PEP carboxykinase activity

The mutants produced in Reference Example 13 were cultured in the same manner as in Example 10. The concentrations of α-hydromuconic acid and other products accumulated in the culture supernatant and the concentration of sugars remaining unused in the culture medium were quantified. The yield of α-hydromuconic acid calculated using the formula (2) from the measured values is shown in Table 8.

Comparative Example 8

Production test of α-hydromuconic acid using *E. coli* mutants with impaired pyruvate kinase function and with unenhanced PEP carboxykinase activity

The mutants produced in Reference Example 14 were cultured in the same manner as in Example 10. The concentrations of α-hydromuconic acid and other products accumulated in the culture supernatant and the concentration of sugars remaining unused in the culture medium were quantified. The yield of α-hydromuconic acid calculated using the formula (2) from the measurement results is shown in Table 8.

By comparing the results of Comparative Examples 7 and 8 with that of Example 10, it was found that the yield of α-hydromuconic acid was increased by impairing the function of pyruvate kinase and enhancing the PEP carboxykinase activity in *E. coli.*

[Table 8]

| | Strain | Yield of HMA (%) |
|---|---|---|
| Example 10 | EcΔPP/HMA1PCK | 0.154 |
| | EcΔPP/HMA2 PCK | 0.176 |
| | EcΔPP/HMA3 PCK | 0.174 |
| | EcΔPP/HMA4 PCK | 0.125 |
| | EcΔPP/HMA5 PCK | 0.213 |
| | EcΔPP/HMA6 PCK | 0.214 |
| | EcΔPP/HMA7 PCK | 0.191 |
| Comparative Example 7 | Ec/pBBRpMW | N.D. |
| | Ec/HMA1 PCK | 0.0257 |
| | Ec/HMA2 PCK | 0.0459 |
| | Ec/HMA3 PCK | 0.0409 |
| | Ec/HMA4 PCK | 0.0459 |
| | Ec/HMA5 PCK | 0.0448 |
| | Ec/HMA6 PCK | 0.0462 |
| | Ec/HMA7 PCK | 0.0352 |
| Comparative Example 8 | EcΔPP/pBBRpMW | 0.0167 |
| | EcΔPP/HMA1 | 0.0511 |
| | EcΔPP/HMA2 | 0.0818 |
| | EcΔPP/HMA3 | 0.0717 |
| | EcΔPP/HMA4 | 0.0688 |
| | EcΔPP /HMA5 | 0.0765 |
| | EcΔPP/HMA6 | 0.0761 |
| | EcΔPP/HMA 7 | 0.0599 |

Example 11

Generation of mutant microorganisms of the genus *Serratia* with impaired pyruvate kinase function and carrying plasmids expressing a PEP carboxykinase and enzymes catalyzing the reactions A, B, C, D, and G

By the same method as in Example 2, the pBBR1MCS-2::ATCT2OR1PCK, pBBR1MCS-2::ATCT2OR2PCK, pBBR1MCS-2::ATCT2OR3PCK, pBBR1MCS-2::ATCT2OR4PCK, pBBR1MCS-2::ATCT2OR5PCK, pBBR1MCS-2::ATCT2OR6PCK, or pBBR1MCS-2::ATCT2OR7PCK produced in Reference Example 6 was introduced into the SgΔPP. By the same method as in Example 7, the plasmid pMW119::EHER produced in Reference Example 4 was introduced into each of the obtained mutants to generate mutant microorganisms of the genus *Serratia.* The obtained strains were designated as SgΔPP/ADA1PCK, SgΔPP/ADA2PCK, SgΔPP/ADA3PCK, SgΔPP/ADA4PCK, SgΔPP/ADASPCK, SgΔPP/ADA6PCK, and SgΔPP/ADA7PCK, respectively.

Reference Example 15

Generation of mutant microorganisms of the genus *Serratia* with intact pyruvate kinase function and carrying plasmids expressing a PEP carboxykinase and enzymes catalyzing the reactions A, B, C, D, and G

By the same method as in Example 2, the pBBR1MCS-2::ATCT2OR1PCK, pBBR1MCS-2::ATCT2OR2PCK, pBBR1MCS-2::ATCT2OR3PCK, pBBR1MCS-2::ATCT2OR4PCK, pBBR1MCS-2::ATCT2OR5PCK, pBBR1MCS-2::ATCT2OR6PCK, or pBBR1MCS-2::ATCT2OR7PCK produced in Reference Example 6 was introduced into *Serratia*

*grimesii* NBRC13537. By the same method as in Example 7, the plasmid pMW119::EHER produced in Reference Example 4 was introduced into each of the obtained mutants to generate mutant microorganisms of the genus *Serratia*. The obtained strains were designated as Sg/ADA1PCK, Sg/ADA2PCK, Sg/ADA3PCK, Sg/ADA4PCK, Sg/ADA5PCK, Sg/ADA6PCK, and Sg/ADA7PCK, respectively.

Reference Example 16

Generation of mutant microorganisms of the genus *Serratia* with impaired pyruvate kinase function and carrying plasmids expressing enzymes that catalyze the reactions A, B, C, D, and G

By the same method as in Example 2, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into the SgΔPP. By the same method as in Example 7, the plasmid pMW1 19::EHER produced in Reference Example 4 was introduced into each of the obtained mutants to generate mutant microorganisms of the genus *Serratia*. The obtained strains were designated as SgΔPP/ADA1, SgΔPP/ADA2, SgΔPP/ADA3, SgΔPP/ADA4, SgΔPP/ADA5, SgΔPP/ADA6, and SgΔPP/ADA7, respectively.

Example 12

Production test of adipic acid using mutant microorganisms of the genus *Serratia* with impaired pyruvate kinase function and with enhanced PEP carboxykinase activity

The mutants produced in Example 11 were cultured in the same manner as in Example 3, except that ampicillin was added to the culture medium to a final concentration of 500 μg/mL. The concentrations of adipic acid and other products accumulated in the culture supernatant and the concentration of sugars remaining unused in the culture medium were quantified. The quantification of adipic acid was performed using LC-MS/MS under the same conditions for the quantification of 3-hydroxyadipic acid and α-hydromuconic acid. The yield of adipic acid calculated using the formula (2) from the measured values is shown in Table 9.

Comparative Example 9

Production test of adipic acid using mutant microorganisms of the genus *Serratia* with intact pyruvate kinase function and with enhanced PEP carboxykinase activity

The mutants produced in Reference Example 15 and the Sg/pBBRpMW were cultured in the same manner as in Example 12. The concentrations of adipic acid and other products accumulated in the culture supernatant and the concentration of sugars remaining unused in the culture medium were quantified. The yield of adipic acid calculated using the formula (2) from the measured values is shown in Table 9.

Comparative Example 10

Production test of adipic acid using mutant microorganisms of the genus *Serratia* with impaired pyruvate kinase function and with unenhanced PEP carboxykinase activity

The mutants produced in Reference Example 16 and the SgΔPP/pBBRpMW were cultured in the same manner as in Example 12. The concentrations of adipic acid and other products accumulated in the culture supernatant and the concentration of sugars remaining unused in the culture medium were quantified. The yield of adipic acid calculated using the formula (2) from the measurement results is shown in Table 9.

By comparing the results of Comparative Examples 9 and 10 with that of Example 12, it was found that the yield of adipic acid was increased by impairing the function of pyruvate kinase and enhancing the PEP carboxykinase activity in the microorganism of the genus *Serratia*.

[Table 9]

| | Strain | Yield of ADA (%) |
|---|---|---|
| Example 12 | SgΔPP/ADA1PCK | 0.170 |
| | SgΔPP/ ADA 2 PCK | 0.187 |
| | SgΔPP/ ADA 3 PCK | 0.176 |
| | SgΔPP/ ADA 4 PCK | 0.168 |
| | SgΔPP/ ADA 5 PCK | 0.172 |
| | SgΔPP/ ADA 6 PCK | 0.205 |
| | SgΔPP/ ADA 7 PCK | 0.159 |
| Comparative Example 9 | Sg/pBBRpMW | N.D. |
| | Sg/ ADA 1 PCK | 0.0170 |
| | Sg/ ADA 2 PCK | 0.0229 |
| | Sg/ ADA 3 PCK | 0.0177 |
| | Sg/ ADA 4 PCK | 0.0171 |
| | Sg/ ADA 5 PCK | 0.0221 |
| | Sg/ ADA 6 PCK | 0.0185 |
| | Sg/ ADA 7 PCK | 0.0203 |
| Comparative Example 10 | SgΔPP/pBBRpMW | N.D. |
| | SgΔPP/ ADA 1 | 0.0783 |
| | SgΔPP/ ADA 2 | 0.110 |
| | SgΔPP/ ADA 3 | 0.0861 |
| | SgΔPP/ ADA 4 | 0.116 |
| | SgΔPP/ ADA 5 | 0.108 |
| | SgΔPP/ ADA 6 | 0.136 |
| | SgΔPP/ ADA 7 | 0.0958 |

Example 13

Generation of *E. coli* mutants with impaired pyruvate kinase function and carrying plasmids expressing a PEP carboxykinase and enzymes catalyzing the reactions A, B, C, D, and G

By the same method as in Example 5, the pBBR1MCS-2::ATCT2OR1PCK, pBBR1MCS-2::ATCT2OR2PCK, pBBR1MCS-2::ATCT2OR3PCK, pBBR1MCS-2::ATCT2OR4PCK, pBBR1MCS-2::ATCT2OR5PCK, pBBR1MCS-2::ATCT2OR6PCK, or pBBR1MCS-2::ATCT2OR7PCK produced in Reference Example 6 was introduced into the EcΔPP. By the same method as in Example 9, the plasmid pMW119::EHER produced in Reference Example 4 was introduced into each of the obtained mutants to generate mutants. The obtained strains were designated as EcΔPP/ADA1PCK, EcΔPP/ADA2PCK, EcΔPP/ADA3PCK, EcΔPP/ADA4PCK, EcΔPP/ADA5PCK, EcΔPP/ADA6PCK, and EcΔPP/ADA7PCK, respectively.

Reference Example 17

Generation of *E. coli* mutants with intact pyruvate kinase function and carrying plasmids expressing a PEP carboxykinase and enzymes catalyzing the reactions A, B, C, D, and G

By the same method as in Example 5, the pBBR1MCS-2::ATCT2OR1PCK, pBBR1MCS-2::ATCT2OR2PCK, pBBR1MCS-2::ATCT2OR3PCK, pBBR1MCS-2::ATCT2OR4PCK, pBBR1MCS-2::ATCT2OR5PCK, pBBR1MCS-2::ATCT2OR6PCK, or pBBR1MCS-2::ATCT2OR7PCK produced in Reference Example 6 was introduced into *Es-*

*cherichia coli* MG1655. By the same method as in Example 9, the plasmid pMW119::EHER produced in Reference Example 4 was introduced into each of the obtained mutants to generate mutants. The obtained strains were designated as Ec/ADA1PCK, Ec/ADA2PCK, Ec/ADA3PCK, Ec/ADA4PCK, Ec/ADA5PCK, Ec/ADA6PCK, and Ec/ADA7PCK, respectively.

Reference Example 18

Generation of *E. coli* mutants with impaired pyruvate kinase function and carrying plasmids expressing enzymes that catalyze the reactions A, B, C, D, and G

By the same method as in Example 5, the PBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6 or pBBR1MCS-2::ATCT2OR7 produced in Reference Example 3 was introduced into the EcΔPP. By the same method as in Example 9, the plasmid pMW119::EHER produced in Reference Example 4 was introduced into each of the obtained mutants to generate mutants. The obtained strains were designated as EcΔPP/ΛDΛ1, EcΔPP/ADA2, EcΔPP/ADA3, EcΔPP/ADA4, EcΔPP/ADA5, EcΔPP/ADA6, and EcΔPP/ADA7, respectively.

Example 14

Production test of adipic acid using *E. coli* mutants with impaired pyruvate kinase function and with enhanced PEP carboxykinase activity

The mutants produced in Example 13 were cultured in the same manner as in Example 6, except that ampicillin was added to the culture medium to a final concentration of 500 μg/mL. The concentrations of adipic acid and other products accumulated in the culture supernatant and the concentration of sugars remaining unused in the culture medium were quantified. The quantification of adipic acid unused the culture medium were quantified. The quantification of adipic acid was performed using LC-MS/MS under the same conditions for the quantification of 3-hydroxyadipic acid and α-hydromuconic acid. The yield of adipic acid calculated using the formula (2) from the measured values is shown in Table 10.

Comparative Example 11

Production test of adipic acid using *E. coli* mutants with intact pyruvate kinase function and with enhanced PEP carboxykinase activity

The mutants produced in Reference Example 17 were cultured in the same manner as in Example 14. The concentrations of adipic acid and other products accumulated in the culture supernatant and the concentration of sugars remaining unused in the culture medium were quantified. The yield of adipic acid calculated using the formula (2) from the measured values is shown in Table 10.

Comparative Example 12

Production test of adipic acid using *E. coli* mutants with impaired pyruvate kinase function and with unenhanced PEP carboxykinase activity

The mutants produced in Reference Example 18 were cultured in the same manner as in Example 14. The concentrations of adipic acid and other products accumulated in the culture supernatant and the concentration of sugars remaining unused in the culture medium were quantified. The yield of adipic acid calculated using the formula (2) from the measurement results is shown in Table 10.

By comparing the results of Comparative Examples 11 and 12 with that of Example 14, it was found that the yield of adipic acid was increased by impairing the function of pyruvate kinase and enhancing the PEP carboxykinase activity in *E. coli.*

[Table 10]

| | Strain | Yield of ADA (%) |
|---|---|---|
| Example 14 | Ec∆PP/ADA1PCK | 0.0374 |
| | Ec∆PP/ ADA 2 PCK | 0.0479 |
| | Ec∆PP/ ADA 3 PCK | 0.0391 |
| | Ec∆PP/ ADA 4 PCK | 0.0372 |
| | Ec∆PP/ ADA 5 PCK | 0.0436 |
| | Ec∆PP/ ADA 6 PCK | 0.0500 |
| | Ec∆PP/ ADA 7 PCK | 0.0345 |
| Comparative Example 11 | Ec/pBBRpMW | N.D. |
| | Ec/ ADA 1 PCK | 0.0109 |
| | Ec/ ADA 2 PCK | 0.0115 |
| | Ec/ ADA 3 PCK | 0.00993 |
| | Ec/ ADA 4 PCK | 0.0131 |
| | Ec/ ADA 5 PCK | 0.00920 |
| | Ec/ ADA 6 PCK | 0.0104 |
| | Ec/ ADA 7 PCK | 0.0113 |
| Comparative Example 12 | Ec∆PP/pBBRpMW | N.D. |
| | Ec∆PP/ ADA 1 | 0.0213 |
| | Ec∆PP/ ADA 2 | 0.0338 |
| | Ec∆PP/ ADA 3 | 0.0293 |
| | Ec∆PP/ ADA 4 | 0.0315 |
| | Ec∆PP/ ADA 5 | 0.0382 |
| | Ec∆PP/ ADA 6 | 0.0407 |
| | Ec∆PP/ ADA 7 | 0.0235 |

Example 15

Production test 2 of 3-hydroxyadipic acid and α-hydromuconic acid using mutant microorganisms of the genus *Serratia* with impaired pyruvate kinase function and with enhanced PEP carboxykinase activity

The production test of 3-hydroxyadipic acid and α-hydromuconic acid was conducted using the mutant microorganisms of the genus *Serratia* produced in Example 2 under anaerobic conditions.

The mutant microorganisms of the genus *Serratia* produced in Example 2 were cultured in the same manner as in Example 3, except that the mutant microorganisms were cultured statically using the culture medium II. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 11.

Comparative Example 13

Production test 2 of 3-hydroxyadipic acid and α-hydromuconic acid using mutant microorganisms of the genus *Serratia* with intact pyruvate kinase function and with enhanced PEP carboxykinase activity

The mutants produced in Reference Example 7 were cultured in the same manner as in Example 15. The concentrations of 3-hydroxyadipic acid, α-hydromuconic acid, and other products accumulated in the culture supernatant and the concentration of sugars remaining unused in the culture medium were quantified. The yields of 3-hydroxyadipic

acid and α-hydromuconic acid calculated using the above formula (2) from the measured values are shown in Table 11.

Comparative Example 14

Production test 2 of 3-hydroxyadipic acid and α-hydromuconic acid using mutant microorganisms of the genus *Serratia* with impaired pyruvate kinase function and with unenhanced PEP carboxykinase activity

The mutants produced in Reference Example 8 were cultured in the same manner as in Example 15. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 11.

By comparing the results of Comparative Examples 13 and 14 with that of Example 15, it was found that the yields of 3-hydroxyadipic acid and α-hydromuconic acid were increased even under anaerobic conditions by impairing the function of pyruvate kinase and enhancing the PEP carboxykinase activity in the microorganism of the genus *Serratia.*

[Table 11]

| | Strain | Yield of 3HA (%) | Yield of HMA (%) |
|---|---|---|---|
| Example 15 | SgΔPP/3HA1PCK | 6.22 | 0.233 |
| | SgΔPP/3HA2 PCK | 7.34 | 0.239 |
| | SgΔPP/3HA3 PCK | 7.30 | 0.245 |
| | SgΔPP/3HA4 PCK | 7.50 | 0.267 |
| | SgΔPP/3HA5 PCK | 6.45 | 0.233 |
| | SgΔPP/3HA6 PCK | 6.70 | 0.256 |
| | SgΔPP/3HA7 PCK | 6.85 | 0.224 |
| Comparative Example 13 | Sg/pBBR | N.D. | N.D. |
| | Sg/3HA1 PCK | 1.14 | 0.0345 |
| | Sg/3HA2 PCK | 1.71 | 0.0415 |
| | Sg/3HA3 PCK | 1.33 | 0.0337 |
| | Sg/3HA4 PCK | 1.37 | 0.0378 |
| | Sg/3HA5 PCK | 1.59 | 0.3750 |
| | Sg/3HA6 PCK | 2.07 | 0.0452 |
| | Sg/3HA7 PCK | 1.12 | 0.0428 |
| Comparative Example 14 | SgΔPP/pBBR | 0.0485 | 0.0224 |
| | SgΔPP/3HA1 | 4.84 | 0.159 |
| | SgΔPP/3HA2 | 6.07 | 0.171 |
| | SgΔPP/3HA3 | 5.99 | 0.143 |
| | SgΔPP/3HA4 | 5.30 | 0.195 |
| | SgΔPP/3HA5 | 5.84 | 0.180 |
| | SgΔPP/3HA6 | 6.02 | 0.202 |
| | SgΔPP/3HA7 | 5.98 | 0.160 |

Example 16

Production test 2 of 3-hydroxyadipic acid and α-hydromuconic acid using *E. coli* mutants with impaired pyruvate kinase function

The production test of 3-hydroxyadipic acid and α-hydromuconic acid was conducted under anaerobic conditions

using the E. *coli* mutants produced in Example 5.

The *E. coli* mutants produced in Example 5 were cultured in the same manner as in Example 6, except that the mutants were cultured statically using the culture medium II. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 12.

Comparative Example 15

Production test 2 of 3-hydroxyadipic acid and α-hydromuconic acid using *E. coli* mutants with intact pyruvate kinase function and with enhanced PEP carboxykinase activity

The mutants produced in Reference Example 9 were cultured in the same manner as in Example 16. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 12.

Comparative Example 16

Production test 2 of 3-hydroxyadipic acid and α-hydromuconic acid using *E. coli* mutants with impaired pyruvate kinase function and with unenhanced PEP carboxykinase activity

The mutants produced in Reference Example 10 were cultured in the same manner as in Example 16. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 12.

By comparing the results of Comparative Examples 15 and 16 with that of Example 16, it was found that the yields of 3-hydroxyadipic acid and α-hydromuconic acid were increased even under anaerobic conditions by impairing the function of pyruvate kinase and enhancing the PEP carboxykinase activity in *E. coli.*

[Table 12]

| | Strain | Yield of 3HA (%) | Yield of HMA (%) |
|---|---|---|---|
| Example 16 | EcΔPP/3HA1PCK | 15.6 | 0.0289 |
| | EcΔPP/3HA2 PCK | 16.8 | 0.0334 |
| | EcΔPP/3HA3 PCK | 15.5 | 0.0355 |
| | EcΔPP/3HA4 PCK | 15.8 | 0.0295 |
| | EcΔPP/3HA5 PCK | 16.2 | 0.0326 |
| | EcΔPP/3HA6 PCK | 17.2 | 0.0279 |
| | EcΔPP/3HA7 PCK | 16.4 | 0.0270 |
| Comparative Example 15 | Ec/pBBR | N.D. | N.D. |
| | Ec/3HA1 PCK | 0.963 | 0.0116 |
| | Ec/3HA2 PCK | 1.46 | 0.0103 |
| | Ec/3HA3 PCK | 1.34 | 0.0106 |
| | Ec/3HA4 PCK | 1.09 | 0.0102 |
| | Ec/3HA5 PCK | 1.49 | 0.0120 |
| | Ec/3HA6 PCK | 1.20 | 0.1120 |
| | Ec/3HA7 PCK | 0.940 | 0.1220 |

(continued)

| | Strain | Yield of 3HA (%) | Yield of HMA (%) |
|---|---|---|---|
| Comparative Example 16 | EcΔPP/pBBR | 0.0669 | 0.0113 |
| | EcΔPP/3HA1 | 13.2 | 0.0213 |
| | EcΔPP/3HA2 | 14.9 | 0.0277 |
| | EcΔPP/3HA3 | 13.9 | 0.0268 |
| | EcΔPP/3HA4 | 14.1 | 0.0224 |
| | EcΔPP/3HA5 | 14.3 | 0.0259 |
| | EcΔPP/3HA6 | 14.7 | 0.0226 |
| | EcΔPP/3HA 7 | 13.2 | 0.0213 |

Example 17

Production test 2 of adipic acid using mutant microorganisms of the genus *Serratia* with impaired pyruvate kinase function and with enhanced PEP carboxykinase activity

The production test of adipic acid was conducted using the mutant microorganisms of the genus *Serratia* produced in Example 11 under anaerobic conditions.

The mutant microorganisms of the genus *Serratia* produced in Example 11 were cultured in the same manner as in Example 12, except that the mutant microorganisms were cultured statically using the culture medium II. The concentrations of adipic acid and other products accumulated in the culture supernatant and the concentration of sugars remaining unused in the culture medium were quantified. The yield of adipic acid calculated using the formula (2) from the measured values is shown in Table 13.

Comparative Example 17

Production test 2 of adipic acid using mutant microorganisms of the genus *Serratia* with intact pyruvate kinase function and with enhanced PEP carboxykinase activity

The mutants produced in Reference Example 15 were cultured in the same manner as in Example 17. The concentrations of adipic acid and other products accumulated in the culture supernatant and the concentration of sugars remaining unused in the culture medium were quantified. The yield of adipic acid calculated unused in the culture medium were quantified. The yield of adipic acid calculated using the formula (2) from the measured values is shown in Table 13.

Comparative Example 18

Production test 2 of adipic acid using mutant microorganisms of the genus *Serratia* with impaired pyruvate kinase function and with unenhanced PEP carboxykinase activity

The mutants produced in Reference Example 16 were cultured in the same manner as in Example 17. The concentrations of adipic acid and other products accumulated in the culture supernatant and the concentration of sugars remaining unused in the culture medium were quantified. The yield of adipic acid calculated using the formula (2) from the measured values is shown in Table 13.

By comparing the results of Comparative Examples 17 and 18 with that of Example 17, it was found that the yield of adipic acid was increased even under anaerobic conditions by impairing the function of pyruvate kinase and enhancing the PEP carboxykinase activity in the microorganism of the genus *Serratia.*

[Table 13]

| | Strain | Yield of ADA (%) |
|---|---|---|
| Example 17 | SgΔPP/ADA1PCK | 0.0699 |
| | SgΔPP/ ADA 2 PCK | 0.0720 |
| | SgΔPP/ ADA 3 PCK | 0.0591 |
| | SgΔPP/ ADA 4 PCK | 0.0613 |
| | SgΔPP/ ADA 5 PCK | 0.0628 |
| | SgΔPP/ ADA 6 PCK | 0.0637 |
| | SgΔPP/ ADA 7 PCK | 0.0518 |
| Comparative Example 17 | Sg/pBBRpMW | N.D. |
| | Sg/ ADA 1 PCK | 0.0102 |
| | Sg/ ADA 2 PCK | 0.0123 |
| | Sg/ ADA 3 PCK | 0.0129 |
| | Sg/ ADA 4 PCK | 0.0122 |
| | Sg/ ADA 5 PCK | 0.0107 |
| | Sg/ ADA 6 PCK | 0.0103 |
| | Sg/ ADA 7 PCK | 0.0790 |
| Comparative Example 18 | SgΔPP/pBBRpMW | N.D. |
| | SgΔPP/ ADA 1 | 0.0359 |
| | SgΔPP/ ADA 2 | 0.0480 |
| | SgΔPP/ ADA 3 | 0.0379 |
| | SgΔPP/ ADA 4 | 0.0395 |
| | SgΔPP/ ADA 5 | 0.0431 |
| | SgΔPP/ ADA 6 | 0.0490 |
| | SgΔPP/ ADA 7 | 0.0375 |

Example 18

Production test 2 of adipic acid using *E. coli* mutants with impaired pyruvate kinase function

The production test of adipic acid was conducted using the *E. coli* mutants produced in Example 13 under anaerobic conditions. The *E. coli* mutants produced in Example 13 were cultured in the same manner as in Example 14, except that the mutants were cultured statically using the culture medium II. The concentrations of adipic acid and other products accumulated in the culture supernatant and the concentration of sugars remaining unused in the culture medium were quantified. The yield of adipic acid calculated using the formula (2) from the measured values is shown in Table 14.

Comparative Example 19

Production test 2 of adipic acid using E. *coli* mutants with intact pyruvate kinase function and with enhanced PEP carboxykinase activity

The mutants produced in Reference Example 17 were cultured in the same manner as in Example 18. The concentrations of adipic acid and other products accumulated in the culture supernatant and the concentration of sugars remaining unused in the culture medium were quantified. The yield of adipic acid calculated using the formula (2) from the measured values is shown in Table 14.

Comparative Example 20

Production test 2 of adipic acid using *E. coli* mutants with impaired pyruvate kinase function and with unenhanced PEP carboxykinase activity

The mutants produced in Reference Example 18 were cultured in the same manner as in Example 18. The concentrations of adipic acid and other products accumulated in the culture supernatant and the concentration of sugars remaining unused in the culture medium were quantified. The yield of adipic acid calculated using the formula (2) from the measured values is shown in Table 14.

By comparing the results of Comparative Examples 19 and 20 with that of Example 18, it was found that the yield of adipic acid was increased even under anaerobic conditions by impairing the function of pyruvate kinase and enhancing the PEP carboxykinase activity in *E. coli.*

[Table 14]

| | Strain | Yield of ADA (%) |
|---|---|---|
| Example 18 | EcΔPP/ADA1PCK | 0.0311 |
| | EcΔPP/ ADA 2 PCK | 0.0384 |
| | EcΔPP/ ADA 3 PCK | 0.0304 |
| | EcΔPP/ ADA 4 PCK | 0.0274 |
| | EcΔPP/ ADA 5 PCK | 0.0254 |
| | EcΔPP/ ADA 6 PCK | 0.0345 |
| | EcΔPP/ ADA 7 PCK | 0.0324 |
| Comparative Example 19 | Ec/pBBRpMW | N.D. |
| | Ec/ ADA 1 PCK | 0.0112 |
| | Ec/ ADA 2 PCK | 0.0123 |
| | Ec/ ADA 3 PCK | 0.0103 |
| | Ec/ ADA 4 PCK | 0.00990 |
| | Ec/ ADA 5 PCK | 0.0109 |
| | Ec/ ADA 6 PCK | 0.0151 |
| | Ec/ ADA 7 PCK | 0.00910 |
| Comparative Example 20 | EcΔPP/pBBRpMW | N.D. |
| | EcΔPP/ ADA 1 | 0.0255 |
| | EcΔPP/ ADA 2 | 0.0254 |
| | EcΔPP/ ADA 3 | 0.0269 |
| | EcΔPP/ ADA 4 | 0.0248 |
| | EcΔPP/ ADA 5 | 0.0212 |
| | EcΔPP/ ADA 6 | 0.0278 |
| | EcΔPP/ ADA 7 | 0.0212 |

Reference Example 19

Generation of mutant microorganisms of the genus *Serratia* with intact pyruvate kinase function and carrying a plasmid expressing enzymes that catalyze the reactions A, B, E, and F

By the same method as in Example 2, the pBBR1MCS-2 (control), pBBR1MCS-2::ATCTOR1 pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into *Serratia grimesii* NBRC13537. The obtained strains

were designated as Sg/pBBR (negative control), Sg/3HA1, Sg/3HA2, Sg/3HA3, Sg/3HA4, Sg/3HA5, Sg/3HA6, and Sg/3HA7, respectively.

Comparative Example 21

Production test of 3-hydroxyadipic acid and α-hydromuconic acid using mutant microorganisms of the genus *Serratia* with intact pyruvate kinase function

The mutant microorganisms of the genus *Serratia* produced in Reference Example 19 were cultured in the same manner as in Example 3. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measurement results are shown in Table 15.

By comparing the results of Comparative Example 21 and Comparative Example 1, it was found that the yields of 3-hydroxyadipic acid and α-hydromuconic acid were decreased in the microorganisms of the genus *Serratia* with intact pyruvate kinase function and with enhanced PEP carboxykinase activity, as compared to those with unenhanced PEP carboxykinase activity.

[Table 15]

| | Strain | Yield of 3HA (%) | Yield of HMA (%) |
|---|---|---|---|
| Comparative Example 21 | Sg/pBBR | N.D. | N.D. |
| | Sg/3HA1 | 0.784 | 0.0293 |
| | Sg/3HA2 | 1.15 | 0.0470 |
| | Sg/3HA3 | 0.942 | 0.0461 |
| | Sg/3HA4 | 0.875 | 0.0418 |
| | Sg/3HA5 | 1.01 | 0.0529 |
| | Sg/3HA6 | 1.03 | 0.0366 |
| | Sg/3HA7 | 0.943 | 0.0237 |

Reference Example 20

Generation of *E. coli* mutants with intact pyruvate kinase function and carrying a plasmid expressing enzymes that catalyze the reactions A, B, E, and F

By the same method as in Example 5, the pBBR1MCS-2 (control), pBBR1MCS-2::ATCTOR1, pBBR1MCS-2::ATCTOR2, pBBR1MCS-2::ATCTOR3, pBBR1MCS-2::ATCTOR4, pBBR1MCS-2::ATCTOR5, pBBR1MCS-2::ATCTOR6, or pBBR1MCS-2::ATCTOR7 was introduced into *Escherichia coli* MG1655. The obtained strains were designated as Ec/pBBR (negative control), Ec/3HA1, Ec/3HA2, Ec/3HA3, Ec/3HA4, Ec/3HA5, Ec/3HA6, and Ec/3HA7, respectively.

Comparative Example 22

Production test of 3-hydroxyadipic acid and α-hydromuconic acid using *E. coli* mutants with intact pyruvate kinase function

The mutants produced in Reference Example 20 were cultured in the same manner as in Example 6. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 16.

By comparing the results of Comparative Example 22 and Comparative Example 3, it was found that the yields of 3-hydroxyadipic acid and α-hydromuconic acid were decreased in the *E. coli* strains with intact pyruvate kinase function and with enhanced PEP carboxykinase activity, as compared to those with unenhanced PEP carboxykinase activity.

[Table 16]

| | Strain | Yield of 3HA (%) | Yield of HMA (%) |
|---|---|---|---|
| Comparative Example 22 | Ec/pBBR | N.D. | N.D. |
| | Ec/3HA1 | 1.48 | 0.0172 |
| | Ec/3HA2 | 2.59 | 0.0160 |
| | Ec/3HA3 | 2.64 | 0.0167 |
| | Ec/3HA4 | 1.82 | 0.0186 |
| | Ec/3HA5 | 2.47 | 0.0166 |
| | Ec/3HA6 | 2.66 | 0.0228 |
| | Ec/3HA7 | 1.78 | 0.0172 |

Reference Example 21

Generation of mutant microorganisms of the genus *Serratia* with intact pyruvate kinase function and carrying plasmids expressing enzymes that catalyze the reactions A, B, C, E, and F

By the same method as in Example 7, the pMW119 (control) or pMW119::EH was introduced into Sg/pBBR, Sg/3HA1, Sg/3HA2, Sg/3HA3, Sg/3HA4, Sg/3HA5, Sg/3HA6, and Sg/3HA7. The obtained strains were designated as Sg/pBBRpMW (negative control), Sg/HMA1, Sg/HMA2, Sg/HMA3, Sg/HMA4, Sg/HMA5, Sg/HMA6, and Sg/HMA7, respectively.

Comparative Example 23

Production test of α-hydromuconic acid using mutant microorganisms of the genus *Serratia* with intact pyruvate kinase function

The mutants produced in Reference Example 21 were cultured in the same manner as in Example 8. The concentrations of α-hydromuconic acid and other products accumulated in the culture supernatant and the concentration of sugars remaining unused in the culture medium were quantified. The yield of α-hydromuconic acid calculated using the above formula (2) from the measured values is shown in Table 17.

By comparing the results of Comparative Example 23 and Comparative Example 5, it was found that the yield of α-hydromuconic acid was decreased in the microorganisms of the genus *Serratia* with intact pyruvate kinase function and with enhanced PEP carboxykinase activity, as compared to those with unenhanced PEP carboxykinase activity.

[Table 17]

| | Strain | Yield of HMA (%) |
|---|---|---|
| Comparative Example 23 | Sg/pBBRpMW | N.D. |
| | Sg/HMA1 | 0.0495 |
| | Sg/HMA2 | 0.0584 |
| | Sg/HMA3 | 0.0434 |
| | Sg/HMA4 | 0.0524 |
| | Sg/HMA5 | 0.0587 |
| | Sg/HMA6 | 0.0618 |
| | Sg/HMA7 | 0.0519 |

Reference Example 22

Generation of *E. coli* mutants with intact pyruvate kinase function and carrying plasmids expressing enzymes that catalyze the reactions A, B, C, E, and F

By the same method as in Example 9, the pMW119 (control) or pMW119::EH was introduced into the Ec/pBBR, Ec/3HA1, Ec/3HA2, Ec/3HA3, Ec/3HA4, Ec/3HA5, Ec/3HA6, and Ec/3HA7. The obtained strains were designated as Ec/pBBRpMW (negative control), Ec/HMA1, Ec/HMA2, Ec/HMA3, Ec/HMA4, Ec/HMA5, Ec/HMA6, and Ec/HMA7, respectively.

Comparative Example 24

Production test of $\alpha$-hydromuconic acid using *E. coli* mutants with intact pyruvate kinase function

The mutants produced in Reference Example 22 were cultured in the same manner as in Example 10. The concentrations of $\alpha$-hydromuconic acid and other products accumulated in the culture supernatant and the concentration of sugars remaining unused in the culture medium were quantified. The yield of $\alpha$-hydromuconic acid calculated using the above formula (2) from the measured values is shown in Table 18.

By comparing the results of Comparative Example 24 and Comparative Example 7, it was found that the yield of $\alpha$-hydromuconic acid was decreased in the *E. coli* strains with intact pyruvate kinase function and with enhanced PEP carboxykinase activity, as compared to those with unenhanced PEP carboxykinase activity.

[Table 18]

| | Strain | Yield of HMA (%) |
|---|---|---|
| Comparative Example 24 | Ec/pBBRpMW | N.D. |
| | Ec/HMA1 | 0.0362 |
| | Ec/HMA2 | 0.0636 |
| | Ec/HMA3 | 0.0569 |
| | Ec/HMA4 | 0.0624 |
| | Ec/HMA5 | 0.0621 |
| | Ec/HMA6 | 0.0640 |
| | Ec/HMA7 | 0.0491 |

Reference Example 23

Generation of mutant microorganisms of the genus *Serratia* with intact pyruvate kinase function and carrying plasmids expressing enzymes that catalyze the reactions A, B, C, D, and G

By the same method as in Example 11, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into *Serratia grimesii* NBRC13537. By the same method as in Example 7, the plasmid pMW119::EHER produced in Reference Example 4 was introduced into each of the obtained mutants to generate mutant microorganisms of the genus *Serratia.* The obtained strains were designated as Sg/ADA1, Sg/ADA2, Sg/ADA3, Sg/ADA4, Sg/ADA5, Sg/ADA6, and Sg/ADA7, respectively.

Comparative Example 25

Production test of adipic acid using mutant microorganisms of the genus *Serratia* with intact pyruvate kinase function

The mutants produced in Reference Example 23 and the Sg/pBBRpMW were cultured in the same manner as in Example 8. The concentrations of adipic acid and other products accumulated in the culture supernatant and the concentration of sugars remaining unused in the culture medium were quantified. The yield of adipic acid calculated using the above formula (2) from the measured values is shown in Table 19.

By comparing the results of Comparative Example 25 and Comparative Example 9, it was found that the yield of adipic acid was decreased in the microorganisms of the genus *Serratia* with intact pyruvate kinase function and with enhanced PEP carboxykinase activity, as compared to those with unenhanced PEP carboxykinase activity.

[Table 19]

| | Strain | Yield of ADA (%) |
|---|---|---|
| Comparative Example 25 | Sg/pBBRpMW | N.D. |
| | Sg/ADA1 | 0.0244 |
| | Sg/ADA2 | 0.0325 |
| | Sg/ADA3 | 0.0254 |
| | Sg/ADA4 | 0.0246 |
| | Sg/ADA5 | 0.0314 |
| | Sg/ADA6 | 0.0264 |
| | Sg/ADA7 | 0.0289 |

Reference Example 24

Generation of *E. coli* mutants with intact pyruvate kinase function and carrying plasmids expressing enzymes that catalyze the reactions A, B, C, D, and G

By the same method as in Example 13, the pBBR1MCS-2::ATCT2OR1, pBBR1MCS-2::ATCT2OR2, pBBR1MCS-2::ATCT2OR3, pBBR1MCS-2::ATCT2OR4, pBBR1MCS-2::ATCT2OR5, pBBR1MCS-2::ATCT2OR6, or pBBR1MCS-2::ATCT2OR7 produced in Reference Example 3 was introduced into *Escherichia coli* MG1655. By the same method as in Example 9, the plasmid pMW119::EHER produced in Reference Example 4 was introduced into each of the obtained mutants to generate *E. coli* mutants. The obtained strains were designated as Ec/ADA1 , Ec/ADA2, Ec/ADA3, Ec/ADA4, Ec/ADA5, Ec/ADA6, and Ec/ADA7, respectively.

Comparative Example 26

Production test of adipic acid using *E. coli* mutants with intact pyruvate kinase function

The mutants produced in Reference Example 24 and the Ec/pBBRpMW were cultured in the same manner as in Example 10. The concentrations of adipic acid and other products accumulated in the culture supernatant and the concentration of sugars remaining unused in the culture medium were quantified. The yield of adipic acid calculated using the above formula (2) from the measured values is shown in Table 20.

By comparing the results of Comparative Example 26 and Comparative Example 11, it was found that the yield of adipic acid was decreased in the *E. coli* strains with intact pyruvate kinase function and with enhanced PEP carboxykinase activity, as compared to those with unenhanced PEP carboxykinase activity.

[Table 20]

| | Strain | Yield of ADA (%) |
|---|---|---|
| Comparative Example 26 | Ec/pBBRpMW | N.D. |
| | Ec/ADA1 | 0.0148 |
| | Ec/ADA2 | 0.0153 |
| | Ec/ADA3 | 0.0107 |
| | Ec/ADA4 | 0.0192 |
| | Ec/ADA5 | 0.0139 |
| | Ec/ADA6 | 0.0147 |
| | Ec/ADA7 | 0.0167 |

Comparative Example 27

Production test 2 of 3-hydroxyadipic acid and α-hydromuconic acid using mutant microorganisms of the genus *Serratia* with intact pyruvate kinase function

The mutants produced in Reference Example 19 were cultured in the same manner as in Example 15. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 21.

By comparing the results of Comparative Example 27 and Comparative Example 13, it was found that the yields of 3-hydroxyadipic acid and α-hydromuconic acid were decreased even under anaerobic conditions in the microorganisms of the genus *Serratia* with intact pyruvate kinase function and with enhanced PEP carboxykinase activity, as compared to those with unenhanced PEP carboxykinase activity.

[Table 21]

| | Strain | Yield of 3HA (%) | Yield of HMA (%) |
|---|---|---|---|
| Comparative Example 27 | Sg/pBBR | N.D. | N.D. |
| | Sg/3HA1 | 1.68 | 0.0482 |
| | Sg/3HA2 | 2.46 | 0.0577 |
| | Sg/3HA3 | 1.94 | 0.0471 |
| | Sg/3HA4 | 1.99 | 0.0527 |
| | Sg/3HA5 | 2.29 | 0.0523 |
| | Sg/3HA6 | 2.95 | 0.0627 |
| | Sg/3HA7 | 1.66 | 0.0595 |

Comparative Example 28

Production test 2 of 3-hydroxyadipic acid and α-hydromuconic acid using *E. coli* mutants with intact pyruvate kinase function

The mutants produced in Reference Example 20 were cultured in the same manner as in Example 16. The concentrations of 3-hydroxyadipic acid, α-hydromuconic acid, and other products accumulated in the culture supernatant and the concentration of sugars remaining unused in the culture medium were quantified. The yields of 3-hydroxyadipic acid and α-hydromuconic acid calculated using the above formula (2) from the measured values are shown in Table 22.

By comparing the results of Comparative Example 28 and Comparative Example 15, it was found that the yields of 3-hydroxyadipic acid and α-hydromuconic acid were decreased even under anaerobic conditions in the *E. coli* strains with intact pyruvate kinase function and with enhanced PEP carboxykinase activity, as compared to those with unenhanced PEP carboxykinase activity.

[Table 22]

| | Strain | Yield of 3HA (%) | Yield of HMA (%) |
|---|---|---|---|
| Comparative Example 28 | Ec/pBBR | N.D. | N.D. |
| | Ec/3HA1 | 1.32 | 0.0171 |
| | Ec/3HA2 | 2.00 | 0.0154 |
| | Ec/3HA3 | 1.82 | 0.0130 |
| | Ec/3HA4 | 1.47 | 0.0136 |
| | Ec/3HA5 | 2.17 | 0.0138 |
| | Ec/3HA6 | 1.77 | 0.0166 |
| | Ec/3HA7 | 1.14 | 0.0179 |

Comparative Example 29

Production test 2 of adipic acid using mutant microorganisms of the genus *Serratia* with intact pyruvate kinase function

The mutants produced in Reference Example 23 were cultured in the same manner as in Example 17. The concentrations of adipic acid and other products accumulated in the culture supernatant and the concentration of sugars remaining unused in the culture medium were quantified. The yield of adipic acid calculated using the above formula (2) from the measured values is shown in Table 23.

By comparing the results of Comparative Example 29 and Comparative Example 17, it was found that the yield of adipic acid was decreased even under anaerobic conditions in the microorganisms of the genus *Serratia* with intact pyruvate kinase function and with enhanced PEP carboxykinase activity, as compared to those with unenhanced PEP carboxykinase activity.

[Table 23]

| | Strain | Yield of ADA (%) |
|---|---|---|
| Comparative Example 29 | Sg/pBBRpMW | N.D. |
| | Sg/ADA1 | 0.0152 |
| | Sg/ADA2 | 0.0181 |
| | Sg/ADA3 | 0.0188 |
| | Sg/ADA4 | 0.0179 |
| | Sg/ADA5 | 0.0135 |
| | Sg/ADA6 | 0.0130 |
| | Sg/ADA7 | 0.00930 |

Comparative Example 30

Production test 2 of adipic acid using *E. coli* mutants with intact pyruvate kinase function

The mutants produced in Reference Example 24 were cultured in the same manner as in Example 18. The concentrations of adipic acid and other products accumulated in the culture supernatant and the concentration of sugars remaining unused in the culture medium were quantified. The yield of adipic acid calculated using the above formula (2) from the measured values is shown in Table 24.

By comparing the results of Comparative Example 30 and Comparative Example 19, it was found that the yield of adipic acid was decreased even under anaerobic conditions in the *E. coli* strains with intact pyruvate kinase function and with enhanced PEP carboxykinase activity, as compared to those with unenhanced PEP carboxykinase activity.

[Table 24]

| | Strain | Yield of ADA (%) |
|---|---|---|
| Comparative Example 30 | Ec/pBBRpMW | N.D. |
| | Ec/ADA1 | 0.0166 |
| | Ec/ADA2 | 0.0180 |
| | Ec/ADA3 | 0.0140 |
| | Ec/ADA4 | 0.0161 |
| | Ec/ADA5 | 0.0148 |
| | Ec/ADA6 | 0.0219 |
| | Ec/ADA7 | 0.0123 |

Example 19

Generation of a mutant microorganism of the genus *Serratia* with defects in genes encoding pyruvate kinase and a phosphotransferase system enzyme

A mutant microorganism of the genus *Serratia* with impaired function of both pyruvate kinase and a phosphotransferase system enzyme was generated by disrupting a gene encoding a phosphotransferase, *ptsG,* in the SgΔPP strain produced in Example 1.

A PCR reaction was performed using pKD4 as a template and oligo DNAs represented by SEQ ID NOs: 244 and 245 as primers to obtain a PCR fragment of 1.6 kb in length for disruption *of ptsG.* The introduction of pKD46 into the above strain was followed by the introduction of the PCR fragment for disruption *of ptsG* into the resulting strain. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 247 were used.

Subsequently, an ampicillin-sensitive strain was obtained by segregating away the pKD46. The plasmid pCP20 was introduced into the ampicillin-sensitive strain, and ampicillin-resistant strains were again obtained. Colony direct PCR was performed on the obtained strains to confirm the deletion of the kanamycin resistance gene from the length of the amplified band. Oligo DNA primers represented by SEQ ID NOs: 246 and 247 were used. The pCP20 was segregated away from one of the kanamycin-sensitive strains. The obtained strain is hereinafter referred to as SgΔPPG.

Example 20

Generation of a mutant microorganism of the genus *Serratia* with defects in genes encoding pyruvate kinase and a phosphotransferase system enzyme and carrying a plasmid expressing a PEP carboxykinase and enzymes catalyzing the reactions A, B, E, and F

By the same method as in Example 2, a plasmid produced in Reference Example 5, pBBR1MCS-2::ATCTOR1PCK, was introduced into the SgΔPPG strain produced in Example 19, and the obtained mutant microorganism of the genus *Serratia* was designated as SgΔPPG/3HA1PCK.

Example 21

Production test of 3-hydroxyadipic acid and α-hydromuconic acid using a mutant microorganism of the genus *Serratia* with impaired function of both pyruvate kinase and a phosphotransferase system enzyme, and carrying a plasmid expressing a PEP carboxykinase and enzymes catalyzing the reactions A, B, E, and F

By the same method as in Example 15, the production test of 3-hydroxyadipic acid and α-hydromuconic acid was conducted using the mutant microorganism of the genus *Serratia* produced in Example 20.

Comparative Example 31

Production test of 3-hydroxyadipic acid and α-hydromuconic acid using a mutant microorganism of the genus *Serratia* with intact pyruvate kinase function and intact phosphotransferase system enzyme function and carrying a plasmid expressing a PEP carboxykinase and enzymes catalyzing the reactions A, B, E, and F

By the same method as in Comparative Example 13, the production test of 3-hydroxyadipic acid and α-hydromuconic acid was conducted using the Sg/3HA1PCK strain produced in Reference Example 7.

By comparing the results of Example 21 and Example 15, it was found that the yields of 3-hydroxyadipic acid and α-hydromuconic acid were further increased in the mutant microorganism of the genus *Serratia* with defects in the genes encoding pyruvate kinase and the phosphotransferase system enzyme and with enhanced activities of PEP carboxykinase and of an enzyme that catalyzes the reaction of reducing 3-oxoadipyl-CoA to 3-hydroxyadipyl-CoA. Additionally, by comparing the results of Example 21 and Comparative Example 31, it was found that the yields of acetic acid and ethanol, both of which were generated by conversion of acetyl-CoA, were also increased in the mutant with defects in the genes encoding pyruvate kinase and the phosphotransferase system enzyme.

[Table 25]

| | Strain | Yield of 3HA (%) | Yield of HMA (%) | Yield of succinic acid (%) | Yield of acetic acid (%) | Yield of ethanol (%) |
|---|---|---|---|---|---|---|
| Example 21 | SgΔPPG/ 3HA1PCK | 8.21 | 0.467 | 45.6 | 43.2 | 50.2 |
| Comparative Example 31 | Sg/3HA1 PCK | 1.14 | 0.0345 | 12.65 | 28.5 | 34.9 |

Example 22

Generation of an *E. coli* mutant with defects in genes encoding pyruvate kinase and a phosphotransferase system enzyme

An *E. coli* mutant with impaired function of both pyruvate kinase and a phosphotransferase system enzyme was generated by disrupting a gene encoding a phosphotransferase, *ptsG,* in the EcΔPP produced in Example 4.

A PCR reaction was performed using pKD4 as a template and oligo DNAs represented by SEQ ID NOs: 248 and 249 as primers to obtain a PCR fragment of 1.6 kb in length for disruption of *ptsG.* The introduction of pKD46 into the above strain was followed by the introduction of the PCR fragment for disruption of *ptsG* into the resulting strain. Colony direct PCR was performed on the resulting kanamycin-resistant strains to confirm the deletion of the gene of interest and the insertion of a kanamycin resistance gene from the length of the amplified band. Oligo DNA primers represented by SEQ ID NOs: 224 and 251 were used.

Subsequently, an ampicillin-sensitive strain was obtained by segregating away the pKD46. The plasmid pCP20 was introduced into the ampicillin-sensitive strain, and ampicillin-resistant strains were again obtained. Colony direct PCR was performed on the obtained strains to confirm the deletion of the kanamycin resistance gene from the length of the amplified band. Oligo DNA primers represented by SEQ ID NOs: 250 and 251 were used. The pCP20 was segregated away from one of the kanamycin-sensitive strains. The obtained strain is hereinafter referred to as EcΔPPG.

Example 23

Generation of an *E. coli* mutant with defects in genes encoding pyruvate kinase and a phosphotransferase system enzyme and carrying a plasmid expressing a PEP carboxykinase and enzymes catalyzing the reactions A, B, E, and F

By the same method as in Example 5, the pBBR1MCS-2::ATCTOR1PCK produced in Reference Example 5 was introduced into the EcΔPPG strain produced in Example 22, and the obtained *E. coli* mutant was designated as EcΔPPG/3HA1PCK.

Example 24

Production test of 3-hydroxyadipic acid and α-hydromuconic acid using an *E. coli* mutant with impaired function of both pyruvate kinase and a phosphotransferase system enzyme, and carrying a plasmid expressing a PEP carboxykinase and enzymes catalyzing the reactions A, B, E, and F

By the same method as in Example 16, the production test of 3-hydroxyadipic acid and α-hydromuconic acid was conducted using the *E. coli* mutant produced in Example 23.

Comparative Example 32

Production test of 3-hydroxyadipic acid and α-hydromuconic acid using an *E. coli* mutant with intact pyruvate kinase function and intact phosphotransferase system enzyme function and carrying a plasmid expressing a PEP carboxykinase and enzymes catalyzing the reactions A, B, E, and F

By the same method as in Comparative Example 15, the production test of 3-hydroxyadipic acid and α-hydromuconic acid was conducted using the Ec/3HA1 produced in Reference Example 9.

By comparing the results of Example 24 and Example 16, it was found that the yields of 3-hydroxyadipic acid and α-hydromuconic acid were further increased in the *E. coli* mutant with defects in the genes encoding pyruvate kinase and the phosphotransferase system enzyme and with enhanced activity of an enzyme that catalyzes the reaction of

reducing 3-oxoadipyl-CoA to 3-hydroxyadipyl-CoA. Additionally, by comparing the results of Example 24 and Comparative Example 32, it was found that the yields of acetic acid and ethanol, both of which were generated by conversion of acetyl-CoA, were also increased in the mutant with defects in the genes encoding pyruvate kinase and the phosphotransferase system enzyme.

[Table 26]

| | Strain | Yield of 3HA (%) | Yield of HMA (%) | Yield of succinic acid (%) | Yield of acetic acid (%) | Yield of ethanol (%) |
|---|---|---|---|---|---|---|
| Example 24 | EcΔPPG/ 3HA1PCK | 18.0 | 0.0596 | 57.4 | 53.9 | 42.4 |
| Comparative Example 32 | Ec/3HA1PCK | 0.963 | 0.0116 | 20.0 | 33.4 | 38.5 |

## Claims

**1.** A genetically modified microorganism with an ability to produce 3-hydroxyadipic acid, α-hydromuconic acid, and/or adipic acid, in which the function of pyruvate kinase is impaired and the activities of phosphoenolpyruvate carboxykinase and of an enzyme that catalyzes a reaction to reduce 3-oxoadipyl-CoA to 3-hydroxyadipyl-CoA are enhanced.

**2.** The genetically modified microorganism according to claim 1, wherein the function of a phosphotransferase system enzyme is further impaired.

**3.** The genetically modified microorganism according to claim 1 or 2, wherein the enzyme that catalyzes a reaction to reduce 3-oxoadipyl-CoA to 3-hydroxyadipyl-CoA is any one of the following polypeptides (a) to (c):

(a) a polypeptide composed of an amino acid sequence represented by any one of SEQ ID NOs: 1 to 7;
(b) a polypeptide composed of the same amino acid sequence as that represented by any one of SEQ ID NOs: 1 to 7, except that one or several amino acids are substituted, deleted, inserted, and/or added, and having an enzymatic activity that catalyzes a reaction to reduce 3-oxoadipyl-CoA to 3-hydroxyadipyl-CoA;
(c) a polypeptide composed of an amino acid sequence with a sequence identity of not less than 70% to the sequence represented by any one of SEQ ID NOs: 1 to 7 and having an enzymatic activity that catalyzes a reaction to reduce 3-oxoadipyl-CoA to 3-hydroxyadipyl-CoA.

**4.** A method of producing 3-hydroxyadipic acid, α-hydromuconic acid, and/or adipic acid, comprising the step of culturing the genetically modified microorganism according to any one of claims 1 to 3.

| INTERNATIONAL SEARCH REPORT | International application No. PCT/JP2020/018665 |
|---|---|

A. CLASSIFICATION OF SUBJECT MATTER

C12P 7/42(2006.01)i; C12N 5/10(2006.01)i; C12N 1/15(2006.01)i; C12N 1/19(2006.01)i; C12N 1/21(2006.01)i; C12N 9/02(2006.01)i
FI: C12N1/21 ZNA; C12N1/19; C12P7/42; C12N9/02; C12N1/15; C12N5/10

According to International Patent Classification (IPC) or to both national classification and IPC

B. FIELDS SEARCHED

Minimum documentation searched (classification system followed by classification symbols)
C12P7/42; C12N5/10; C12N1/15; C12N1/19; C12N1/21; C12N9/02

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); MEDLINE (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2009/151728 A2 (GENOMATICA, INC.) 17.12.2009 (2009-12-17) claims 1-112, page 34, lines 4-24, page 35, line 29 to page 36, line 12, examples 1-2, 4 | 1-2,4<br>1-4 |
| Y | WO 2017/209103 A1 (TORAY INDUSTRIES, INC.) 07.12.2017 (2017-12-07) claims 1-7 | 1-4 |
| Y | WO 2017/209102 A1 (TORAY INDUSTRIES, INC.) 07.12.2017 (2017-12-07) claims 1-7 | 1-4 |
| Y | WO 2016/199858 A1 (TORAY INDUSTRIES, INC.) 15.12.2016 (2016-12-15) claims 1-13 | 1-4 |
| Y | WO 2016/199856 A1 (TORAY INDUSTRIES, INC.) 15.12.2016 (2016-12-15) claims 1-19 | 1-4 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 July 2020 (27.07.2020) | 04 August 2020 (04.08.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No.
PCT/JP2020/018665

C (Continuation). DOCUMENTS CONSIDERED TO BE RELEVANT

| Category* | Citation of document, with indication, where appropriate, of the relevant passages | Relevant to claim No. |
|---|---|---|
| Y | "3-hydroxybutyryl-CoA dehydrogenase [Serratia liquwfaciens].", Database GenBank [online], 11 February 2016, Accession No. AMG98270.1, [retrieval date: 06 February 2019], column "ORIGIN" | 3-4 |
| Y | "3-hydroxybutyryl-CoA dehydrogenase [Serratia marcescens].", Database GenBank [online], 20 February 2017, Accession No. ALL37524.1, [retrieval date: 06 February 2019], column "ORIGIN" | 3-4 |
| Y | "3-hydroxybutyryl-CoA dehydrogenase [Serratia nematodiphila DZ0503SBS1].", Database GenBank [online], 07 August 2014, Accession No. KFF90258.1, [retrieval date: 06 February 2019], column "ORIGIN" | 3-4 |
| Y | "3-hydroxybutyryl-CoA dehydrogenase [Serratia plymuthica S13].", Database GenBank [online], 30 January 2014, Accession No. AGP43956.1, [retrieval date: 06 February 2019], column "ORIGIN" | 3-4 |
| Y | "3-hydroxybutyryl-CoA dehydrogenase [Serratia proteamaculans 568].", Database GenBank [online], 28 January 2014, Accession No. ABV40935.1, [retrieval date: 06 February 2019], column "ORIGIN" | 3-4 |
| Y | "3-hydroxybutyryl-CoA dehydrogenase [Serratia marcescens].", Database GenBank [online], 18 July 2017, Accession No. ASM11476.1, [retrieval date: 06 February 2019], column "ORIGIN" | 3-4 |
| Y | "3-hydroxybutyryl-CoA dehydrogenase [Serratia proteamaculans].", Database GenBank [online], 14 April 2017, Accession No. SMB31239.1, [retrieval date: 06 February 2019], column "ORIGIN" | 3-4 |
| A | WO 2016/106367 A1 (GENOMATICA, INC.) 30.06.2016 (2016-06-30) claims 1-217 | 1-4 |

Form PCT/ISA/210 (continuation of second sheet) (January 2015)

**INTERNATIONAL SEARCH REPORT**
Information on patent family members

International application No.
PCT/JP2020/018665

| Patent Documents referred in the Report | Publication Date | Patent Family | Publication Date |
|---|---|---|---|
| WO 2009/151728 A2 | 17 Dec. 2009 | JP 2011-515111 A<br>claims 1-112, examples 1-2, 4<br>US 2009/0305364 A1<br>EP 2265709 A2<br>CN 102066551 A | |
| WO 2017/209103 A1 | 07 Dec. 2017 | EP 3467113 A1<br>claims 1-7<br>CA 3025886 A<br>CN 109196107 A | |
| WO 2017/209102 A1 | 07 Dec. 2017 | US 2019/0276860 A1<br>claims 1-7<br>EP 3498852 A1<br>CA 3025885 A<br>CN 109154008 A | |
| WO 2016/199858 A1 | 15 Dec. 2016 | US 2018/0223318 A1<br>claims 1-13<br>EP 3309259 A1<br>CA 2988566 A<br>CN 107614692 A | |
| WO 2016/199856 A1 | 15 Dec. 2016 | US 2018/0142271 A1<br>claims 1-19<br>EP 3309258 A1<br>CA 2988696 A<br>CN 107636157 A | |
| WO 2016/106367 A1 | 30 Jun. 2016 | JP 2018-500911 A<br>claims 1-217<br>US 2017/0369913 A1<br>EP 3237629 A1<br>CN 107709568 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013535203 A
- US 20110124911 A1
- JP 2015146810 A
- JP 2011515111 A
- WO 2017209102 A
- WO 2017209103 A
- JP 2008527991 A
- WO 2017099209 A
- WO 2016199858 A1
- WO 2016199856 A1
- JP 2015504688 A


### Non-patent literature cited in the description


- *Proc Natl Acad Sci U.S.A.,* 06 June 2000, vol. 97 (12), 6640-6645
- *Biosci Biotechnol Biochem.,* December 2007, vol. 71 (12), 2905-11
- *Journal of Molecular Biology,* 1970, vol. 53, 159
- **NM CALVIN ; PC HANAWALT.** *J. Bacteriol,* 1988, vol. 170, 2796-2801
- *Biochimie.,* February 2012, vol. 94 (2), 471-8
- *J Appl Microbiol,* October 2015, vol. 119 (4), 1057-63
- **ME KOVACH.** *Gene,* 1995, vol. 166, 175-176
- *Proc Natl Acad Sci USA.,* 06 June 2000, vol. 97 (12), 6640-6645
- *Proc Natl Acad Sci U S A.,* 06 June 2000, vol. 97 (12), 6640-6645